# EUROPEAN PATENT SPECIFICATION

(11) **EP 0 442 497 B1**
(45) Date of publication and mention of the grant of the patent: **14.08.1996**
(21) Application number: 91102101.2
(22) Date of filing: 14.02.1991
(51) Int. Cl.: C07D 477/00, A61K 31/40

(54) **Novel beta-lactam compounds and their production**
Beta-Lactam-Verbindungen und ihre Herstellung
Dérivés de bêta-lactame et leur préparation

(30) Priority: 14.02.1990 JP 34952/90
(43) Date of publication of application: 21.08.1991
(73) Proprietor: SUMITOMO PHARMACEUTICALS COMPANY, LIMITED, Osaka 541 (JP)
(72) Inventor: Sunagawa, Makoto, Itami, Hyogo (JP); Sasaki, Akira, Nishinomiya, Hyogo (JP); Yamaga, Hiroshi, Ibaraki, Osaka (JP); Fukasawa, Masatomo, Takarazuka, Hyogo (JP); Nouda, Hiroshi, Ibaraki, Osaka (JP)
(74) Representative: VOSSIUS & PARTNER

(56) References cited:
- EP-A- 0 126 587
- EP-A- 0 182 213
- EP-A- 0 242 134
- EP-A- 0 243 686

## Description

The present invention relates to β-lactam compounds and their production. More particularly, it relates to novel 3-pyrrolidinylthio-1-azabicyclo[3.2.0]hept-2-en-7-one-2-carboxylic acid compounds bearing a quaternary ammonium group on the pyrrolidine ring and their production.

There are known some β-lactam compounds having a carbapenem skeleton, which possess an excellent antimicrobial spectrum against a wide range of Gram-positive and Gram-negative bacteria. Among them, imipenem is already available on the market. Since, however, imipenem is sensitive to renal dehydropeptidase-I (DHP-I) in a living body and apt to be inactivated, it is normally used in combination with cylastatin for preventing the inactivation with DHP-I. Needless to say, it is clinically favorable that an antimicrobial agent exerts its antimicrobial activity without any auxiliary agent, and a great demand is present towards the development of a β-lactam compound which exerts its antimicrobial activity with resistance to DHP-I, i.e. saving the use of any auxiliary agent.

As the result of an extensive study, it has now been found that some 3-pyrrolidinylthio-1-azabicyclo[3.2.0]hept-2-en-7-one-2-carboxylic acid compounds having a quaternary ammonium group on the pyrrolidine ring exerts a strong antimicrobial activity with sufficient resistance to DHP-I. The present invention is based on the above finding.

Accordingly, a basic object of the present invention is to provide a novel β-lactam compound of the formula: 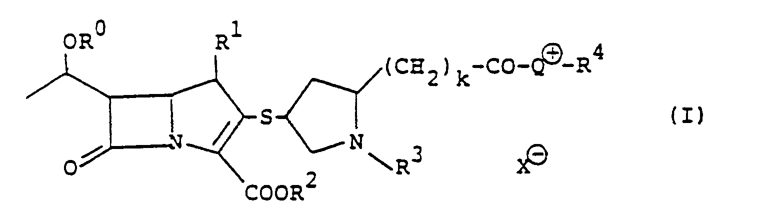 wherein R⁰ is a hydrogen atom or a protective group for hydroxyl, R¹ is a C₁₋₅ alkyl group, R² is a protective group for carboxyl or a negative charge, R³ is a hydrogen atom or a protective group for amino, R⁴ is a C₁₋₅ alkyl group or a C₁-₇ alkyl group substituted with carboxyl, C₂₋₅ alkanoyl, carbamoyl, C₁₋₅ alkylaminocarbonyl, di(C₁₋₅ alkyl)aminocarbonyl, cyano, C₁₋₅ alkoxy, hydroxy or phenyl, k is an integer of 0 to 4, X is an inorganic acid residue, benzenesulfonyloxy, p-toluenesulfonyloxy, methanesulfonyloxy or trifluoromethanesulfonyloxy or is nothing when R² is the negative charge and Q^{⊕} is a quaternary nitrogen atom-containing group represented by either one of the formulas (1) to (4): 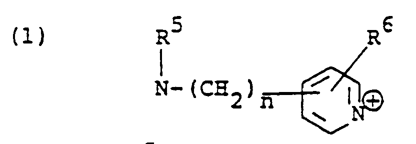 wherein R⁵ is a hydrogen atom, a C₁₋₅ alkyl group or a 2-hydroxyethyl group, R⁶ is a hydrogen atom or a C₁₋₅ alkyl group and n is an integer of 0 to 4; 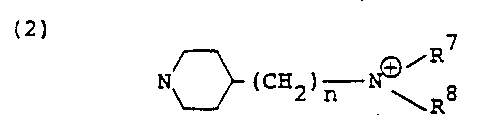 wherein R⁷ and R⁸ are each a C₁₋₅ alkyl group or may be combined together to form a lower alkylene group, or R⁸ represents a C₁-₇ alkyl group substituted with carboxyl, C₂₋₅ alkanoyl, carbamoyl, C₁₋₅ alkylaminocarbonyl, di(C₁₋₅ alkyl)aminocarbonyl, cyano, C₁₋₅ alkoxy, hydroxy or phenyl, and n is as defined above; 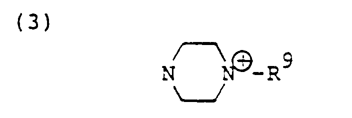 wherein R⁹ is a C₁₋₅ alkyl group or a C₁-₇ alkyl group substituted with carboxyl, C₂₋₅ alkanoyl, carbamoyl, C₁₋₅ alkylaminocarbonyl, di(C₁₋₅ alkyl)aminocarbonyl, cyano, C₁₋₅ alkoxy, hydroxy or phenyl; or 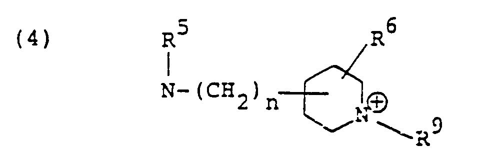 wherein R⁵, R⁶, R⁹ and n are each as defined above.

When R² is a negative charge and X is nothing, the β-lactam compound (I) forms an intramolecular quaternary salt, which is represented by the formula: 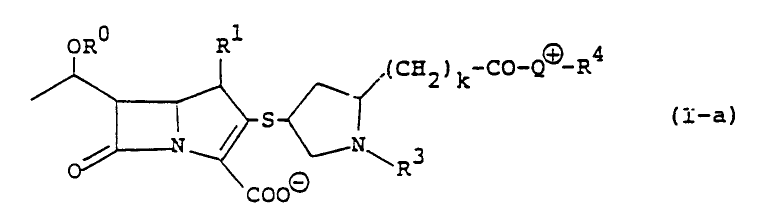 wherein R⁰, R¹, R³, R⁴, k and Q^{⊕} are each as defined above.

Among various β-lactam compounds which fall within the formula (I), the most preferred are those of the formula: 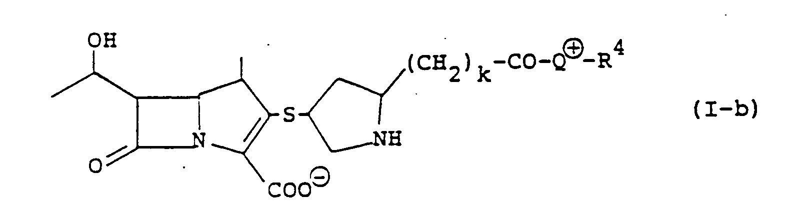 wherein R⁴, k and Q^{⊕} are each as defined above.

According to the present invention, the β-lactam compound (I) can be produced by reacting a β-lactam compound of the formula: 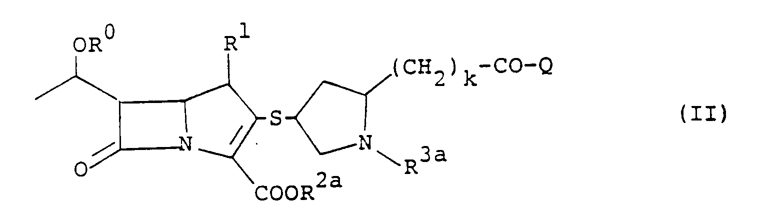 wherein R⁰, R¹ and k are each as defined above, R^{2a} is a protective group for carboxyl, R^{3a} is a protective group for amino and Q is a tertiary nitrogen atom-containing group resulting from elimination of a positive charge from either one of the groups (1) to (4) represented by Q^{⊕} with a compound of the formula:

R⁴-X^{a} (III)

wherein R⁴ is as defined above and X^{a} is an inorganic acid residue, benzenesulfonyloxy, p-toluenesulfonyloxy, methanesulfonyloxy or trifluoromethanesulfonyloxy to give a β-lactam compound of the formula: 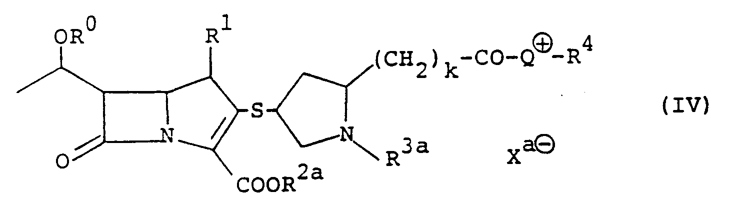 wherein R⁰, R¹, R^{2a}, R^{3a}, R⁴, k, Q^{⊕} and X^{a} are each as defined above, optionally followed by subjecting the β-lactam compound (IV) to elimination of the hydroxyl-protecting group represented by R⁰, elimination of the carboxyl-protecting group represented by R^{2a} and/or elimination of the amino-protecting group represented by R^{3a}, thereby giving the β-lactam compound (I) wherein R⁰ and R³ are each a hydrogen atom and R² is a negative charge.

The protective group for hydroxyl (i.e. hydroxyl-protecting group) represented by R⁰ and the protective group for amino (i.e. amino-protecting group) represented by R³ or R^{3a} may be any group is conventionally used in the related art field. Preferred examples are C₁-C₅ alkoxycarbonyl (e.g. t-butyloxycarbonyl), halo(C₁-C₅)alkoxycarbonyl (e.g. 2-iodoethyloxycarbonyl, 2,2,2-trichloroethyloxycarbonyl), C₃-C₇ alkenyloxycarbonyl (e.g. allyloxycarbonyl), ar(C₁-C₃)alkyloxycarbonyl such as phenyl(C₁-C₃)alkyloxycarbonyl (e.g. benzyloxycarbonyl) or substituted phenyl(C₁-C₃)alkyloxycarbonyl (e.g. p-methoxybenzyloxycarbonyl, o-nitrobenzyloxycarbonyl, p-nitrobenzyloxycarbonyl), and tri(C₁-C₅)alkylsilyl (e.g. trimethylsilyl, t-butyldimethylsilyl), groups.

The protective group for carboxyl (i.e. carboxyl-protective group) represented by R² or R^{2a} may be also any group as conventionally used. Preferred examples are straight or branched C₁-C₅ alkyl (e.g. methyl, ethyl, isopropyl, t-butyl), halo(C₁-C₅)alkyl (e.g. 2-iodoethyl, 2,2,2-trichloroethyl), C₁-C₅ alkoxymethyl (e.g. methoxymethyl, ethoxymethyl, isobutoxymethyl), C₁-C₅ aliphatic acyloxymethyl (e.g. acetoxymethyl, propionyloxymethyl, butyryloxymethyl, pivaloyloxymethyl), 1-(C₁-C₅)alkoxycarbonyloxyethyl (e.g. 1-ethoxycarbonyloxyethyl), ar(C₁-C₃)alkyl such as phenyl(C₁-C₃)alkyl (e.g. benzyl) or substituted phenyl(C₁-C₃)alkyl (e.g. p-methoxybenzyl, o-nitrobenzyl, p-nitrobenzyl), C₃-C₇ alkenyl (e.g. allyl, 2-methylallyl, 3-methylallyl), benzhydryl and phthalidyl groups.

Examples of the C₁₋₅ alkyl group represented by R¹, R⁴, R⁵, R⁶, R⁷, R⁸ or R⁹ are e.g. methyl, ethyl, n-propyl, isopropyl, n-butyl and n-pentyl groups. In case of the substituted C₁₋₇ alkyl group represented by R⁴, R⁸ or R⁹, the substituent on the C₁₋₇ alkyl group may be carboxyl, C₂₋₅ alkanoyl (e.g. acetyl, propionyl), carbamoyl, C₁₋₅ alkylaminocarbonyl (e.g. methylaminocarbonyl), di(C₁₋₅ alkyl)aminocarbonyl (e.g. dimethylaminocarbonyl), cyano, C₁₋₅ alkoxy (e.g. methoxy, ethoxy), hydroxyl, and phenyl groups. Thus, examples of the substituted C₁₋₇ alkyl group are C₁-C₇ alkyl substituted with one or more substitutents as exemplified above, specifically carboxymethyl, acetylmethyl, propionylmethyl, carbamoylmethyl, N-methylaminocarbonylmethyl, N,N-dimethylaminocarbonylmethyl, 2-cyanoethyl, 2-methoxyethyl, 2-ethoxyethyl, 2-carboxyethyl, 2-hydroxyethyl, 2-carbamoylethyl, 2-N-methylaminocarbonylethyl, 2-N,N-dimethylaminocarbonylethyl, 3-carboxypropyl, 4-hydroxybutyl, 5-hydroxypentyl and benzyl groups.

When R⁷ and R⁸ are combined together to make a lower alkylene group, they form a 3- to 7-membered ring together with the nitrogen atom to which they are attached, and examples of the 3- to 7-membered ring are aziridine, azetidine, pyrrolidine and piperidine groups.

Examples of the group represented by X or X^{a} are an inorganic acid residue (e.g. chlorine, bromine, fluorine, iodine) or benzenesulfonyloxy, p-toluenesulfonyloxy, methanesulfonyloxy, trifluoromethanesulfonyloxy.

The β-lactam compound (I) may be either in a free form or in a salt (preferably non-toxic salt) form. Examples of the salt are inorganic base salts (e.g. sodium, potassium, calcium, magnesium, ammonium), organic base salts (e.g. triethylammonium, pyridinium, diisopropylammonium), inorganic acid addition salts (e.g. hydrochloride, sulfate, phosphate), and organic acid addition salts (e.g. formate, acetate, methanesulfonate, benzenesulfonate).

Production of the β-lactam compound (I) will be hereinafter explained in details.

The quaternization or the β-lactam compound (II) may be performed by a per se conventional procedure, for instance, by reacting the β-lactam compound (II) with the compound (III) in an inert solvent chosen from water, ketones (e.g. acetone, methylethylketone), ethers (e.g. tetrahydrofuran, dioxane), acetonitrile and halogenated hydrocarbons (e.g. dichloromethane, dichloroethane, chloroform), or their mixtures. There is no limitation on the reaction temperature, but the reaction is normally effected at a temperature of -40 to 60°C. Upon termination of the reaction, the objective product is isolated from the reaction mixture by a per se conventional procedure.

The thus obtained product, i.e. the β-lactam compound (IV), is optionally subjected to elimination of the hydroxyl-protecting group represented by R⁰, elimination of the carboxyl-protecting group represented by R^{2a} and/or elimination of the amino-protecting group represented by R^{3a} to give the β-lactam compound (I) wherein at least one of R⁰ and R³ is a hydrogen atom and R² is a negative charge.

The elimination may be effected independently or concurrently by a per se conventional procedure such as treatment with an acid, a base or a reducing agent (T.W.Greene: Protective Groups in Organic Synthesis, J. Wiley & Sons Inc., 1981). As the acid, there are exemplified trifluoroacetic acid, formic acid, boron trifluoride and aluminium chloride. As the base, there are exemplified alkali metal carbonate (e.g. sodium carbonate, potassium carbonate), alkali metal sulfate (e.g. sodium sulfate, potassium sulfate) and tetrafluorobutylammonium. When the elimination is conducted through reduction, there may be adopted any procedure using zinc and acetic acid, hydrogen and palladium-carbon or platinum. The elimination with tetrakistriphenylphosphine palladium is also available. Any particular limitation is not present on the solvent to be used, and it may be chosen from water, alcohols (e.g. methanol, ethanol), ethers (e.g. tetrahydrofuran, dioxane) and aliphatic acids (e.g. acetic acid). The reaction temperature may be appropiately decided so as to control or accelerate the proceeding of the reaction, and a preferred temperature is normally from -30 to 40°C. The reaction product may be separated from the reaction mixture by a per se conventional procedure. For instance, the reaction mixture is neutralized and chromatographed on an adsorptive resin, followed by elution and lyophilization.

The β-lactam compound (II) as the starting compound is obtainable by reacting a β-lactam compound of the formula: 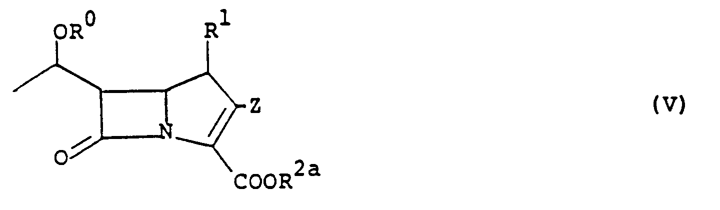 wherein R⁰, R¹ and R^{2a} are each as defined above and Z is a reactive ester on hydroxyl with a mercaptan compound of the formula: 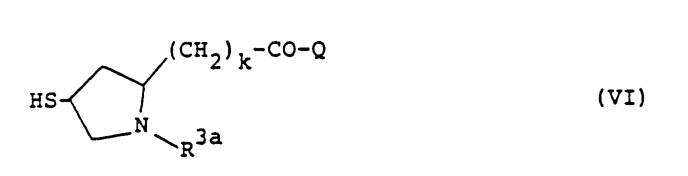 wherein R^{3a}, k and Q are each as defined above in an inert solvent in the presence of a base.

The β-lactam compound (V) is known (cf. Heterocycles, Vol. 21, p. 29-40, 1984), and its reactive ester on hydroxyl represented by Z may be chosen, for instance, from arylsulfonates such as benzenesulfonate and substituted benzenesulfonates (e.g. p-toluenesulfonate, p-nitrobenzenesulfonate, p-bromobenzenesulfonate), C₁-C₅ alkanesulfonates (e.g. methanesulfonate, ethanesulfonate), halo(C₁-C₅)alkanesulfonates (e.g. trifluoromechanesulfonate), diarylphosphates (e.g. diphenylphosphate) and halides (e.g. chloride, bromide, iodide). Of these, p-toluenesulfonate, methanesulfonate and diphenylphosphate are preferred.

The mercaptan compound (VI), which may be produced from trans-4-hydroxy-L-proline or cis-4-hydroxy-D-proline by a known method (cf. U.S. Patent Nos. 4,943,569 and 4,962,103), is usually employed in an excessive amount, particularly in a 1 to 2 equivalent amount to the β-lactam compound (V) so that the reaction with the β-lactam compound (V) proceeds sufficiently.

Examples of the inert solvent are dioxane, tetrahydrofuran, dimethylsulfoxide, acetonitrile and hexamethylphosphoramide. As the base, there may be used an inorganic base (e.g. sodium carbonate, potassium carbonate, sodium hydride, potassium hydride, potassium t-butoxide), an organic base (e.g. pyridine, dimethylaminopyridine, triethylamine, diisopropylethylamine and 1,8-diazabicyclo[5.4.0]-undec-7-ene (DBU), among which preferred are diisopropylethylamine and DBU. The base is used in such an amount as can assure the smooth proceeding of the reaction, normally in a 1 to 3 equimolar amount to the mercaptan compound (VI).

The reaction is normally carried out at a temperature of from -78 to 60°C, preferably from -40 to 40°C.

Upon termination of the reaction, the reaction mixture may be subjected to post-treatment in a per se conventional procedure so as to obtain the objective β-lactam compound (II), if necessary, followed by purification.

The β-lactam compound (I) of the invention includes asymmetric carbon atoms at the 4-, 5-, 6- and 8-positions in the carbapenem skeleton as shown in the following formula and has optical and steric isomers due to those asymmetric carbon atoms: 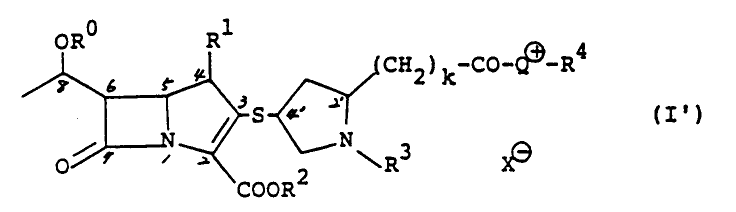 wherein R⁰, R¹, R², R³, R⁴, k and Q^{⊕} and X^{⊖} are each as defined above. While all these optical and steric isomers and their mixtures fall within the scope of the invention, preferred are those having an S-configuration at the 5-position, i.e. (5S,6S) or (5S,6R), those having an R-configuration at the 8-position and those having an R-configuration at the 4-position. More preferred are those having a (4R,5S,6S,8R) configuration as represented by the formula (I'-a) or a (4R,5S,6R,8R) configuration as represented by the formula (I'-b): 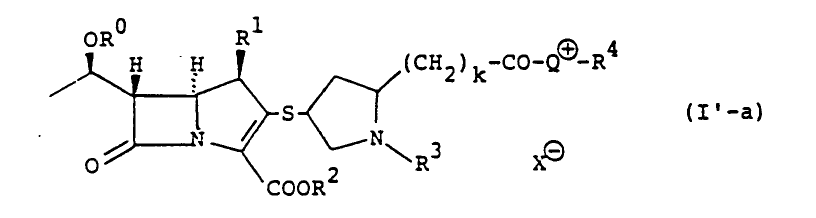 and 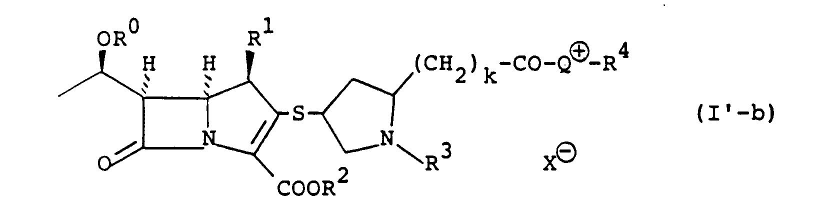
wherein R⁰, R¹, R², R³, R⁴, k, Q^{⊕} and X^{⊖} are each as defined above. The most preferred are those of the formula (I'-c): 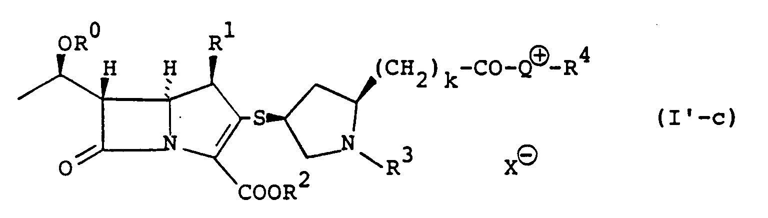
wherein R⁰, R¹, R², R³, R⁴, k, Q^{⊕} and X^{⊖} are each as defined above.

Production of the specific isomers as above stated can be achieved by the use of the corresponding isomers of the β-lactam compound (V) and the mercaptan compound (VI).

Typical examples of the β-lactam compound (I) wherein R⁰ and R³ are each a hydrogen atom, R¹ is a methyl group and R² is a negative charge are shown in Table 1, in which Me and Ph indicate respectively methyl and phenyl.

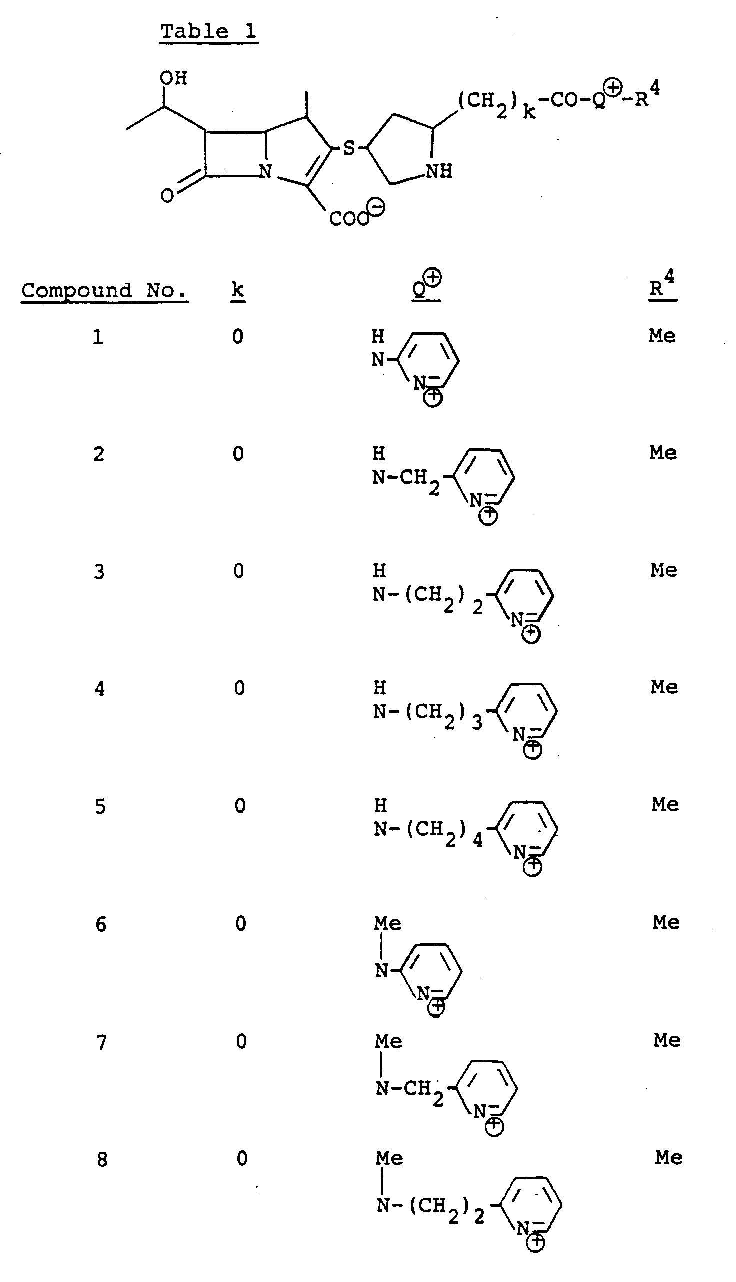

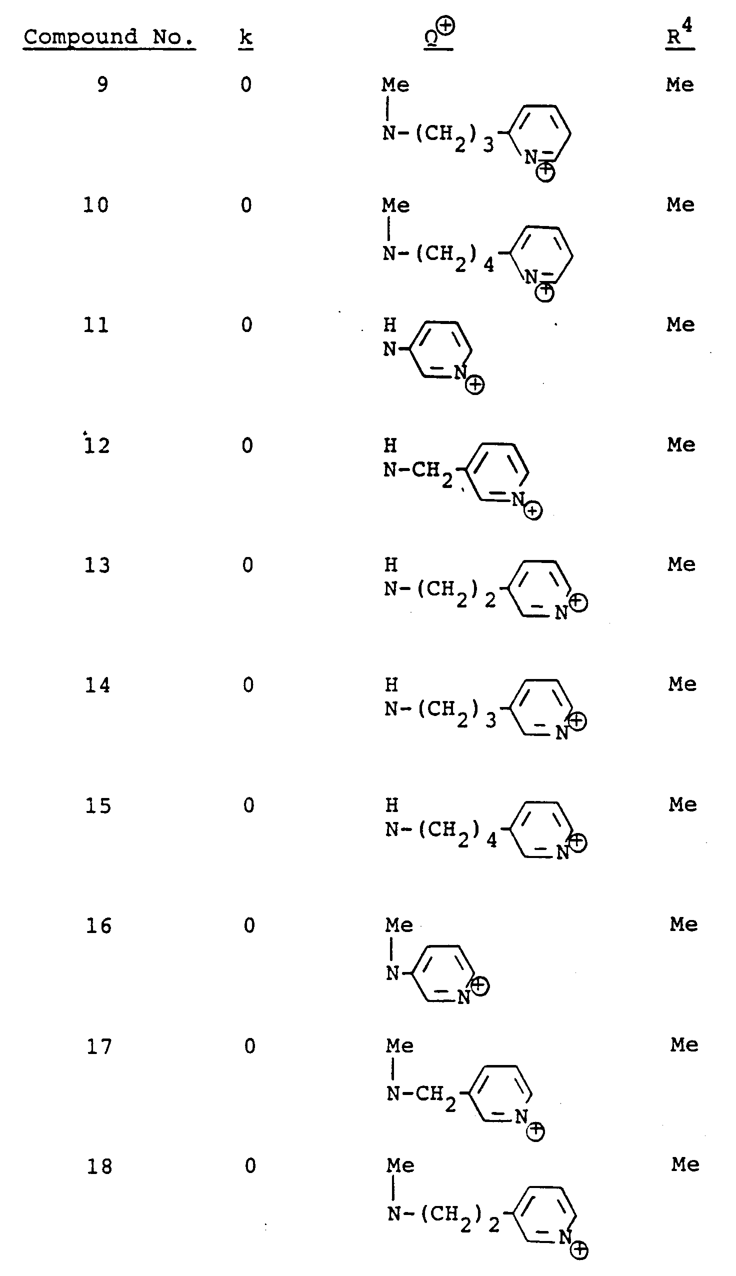

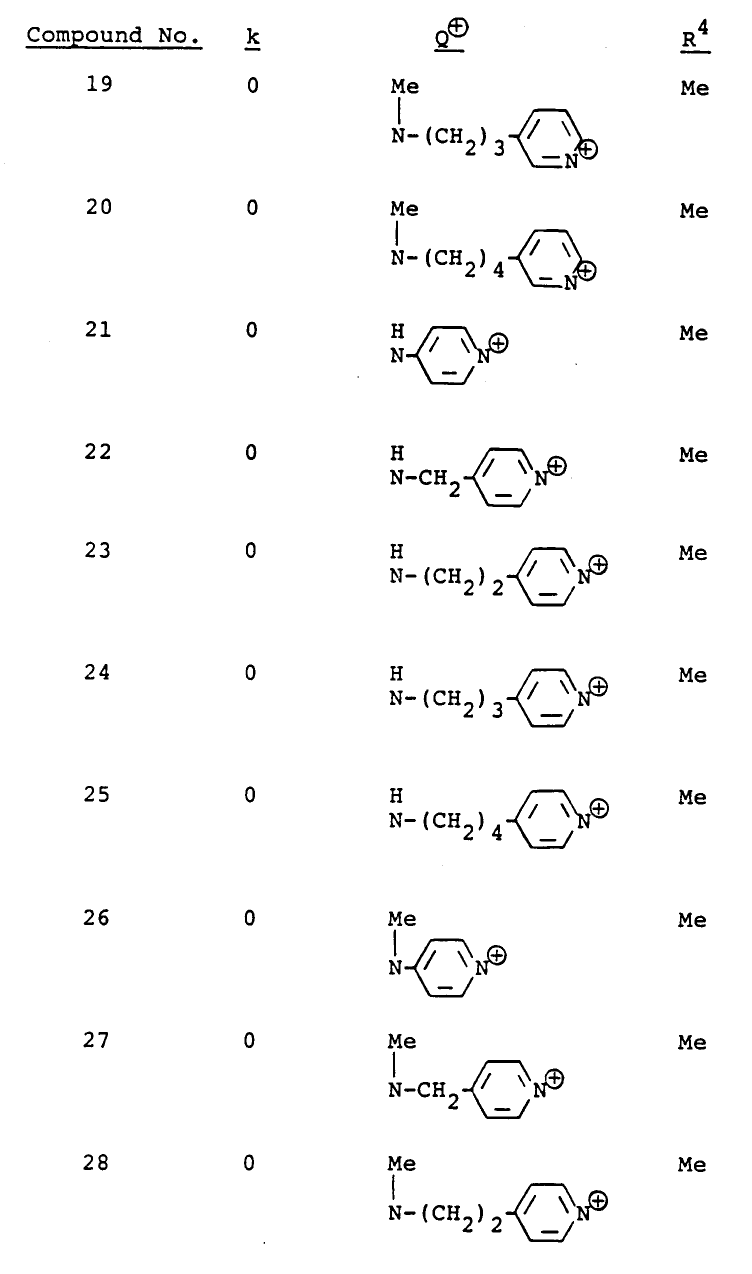

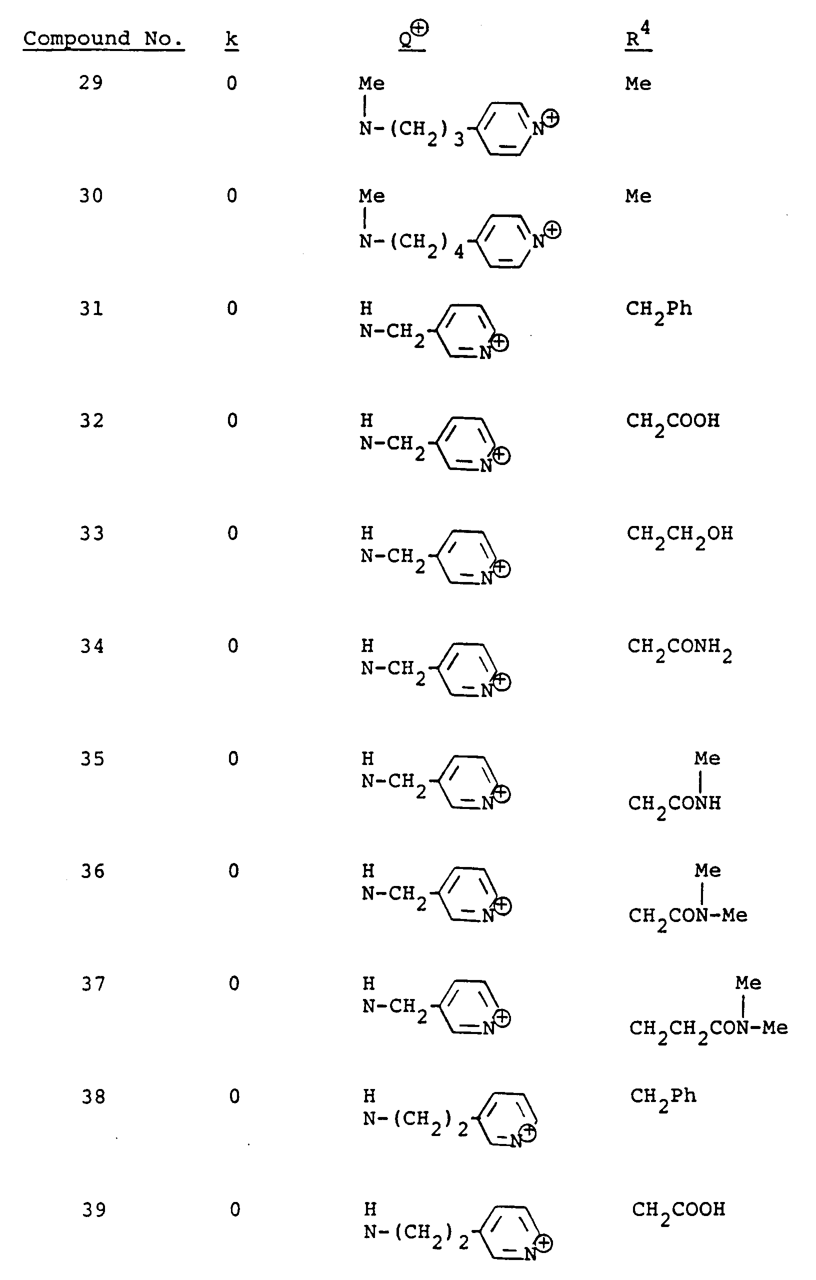

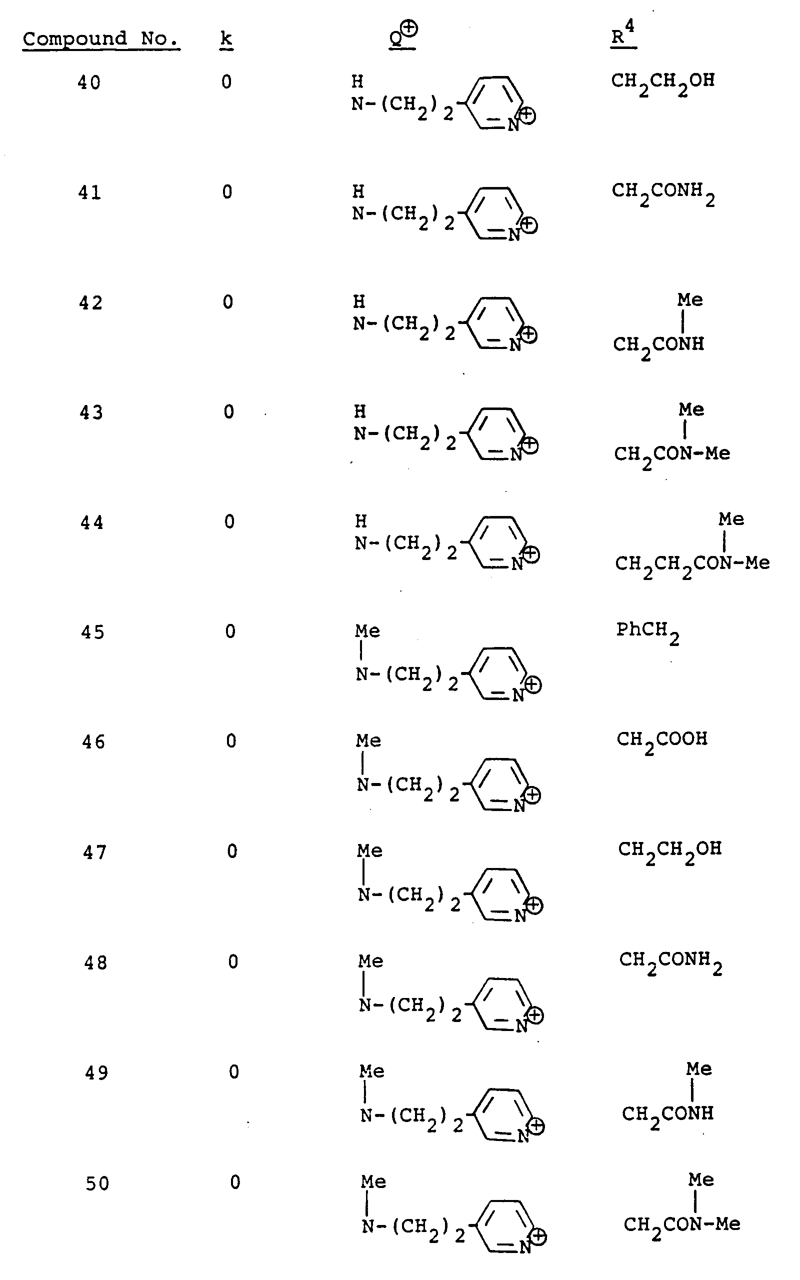

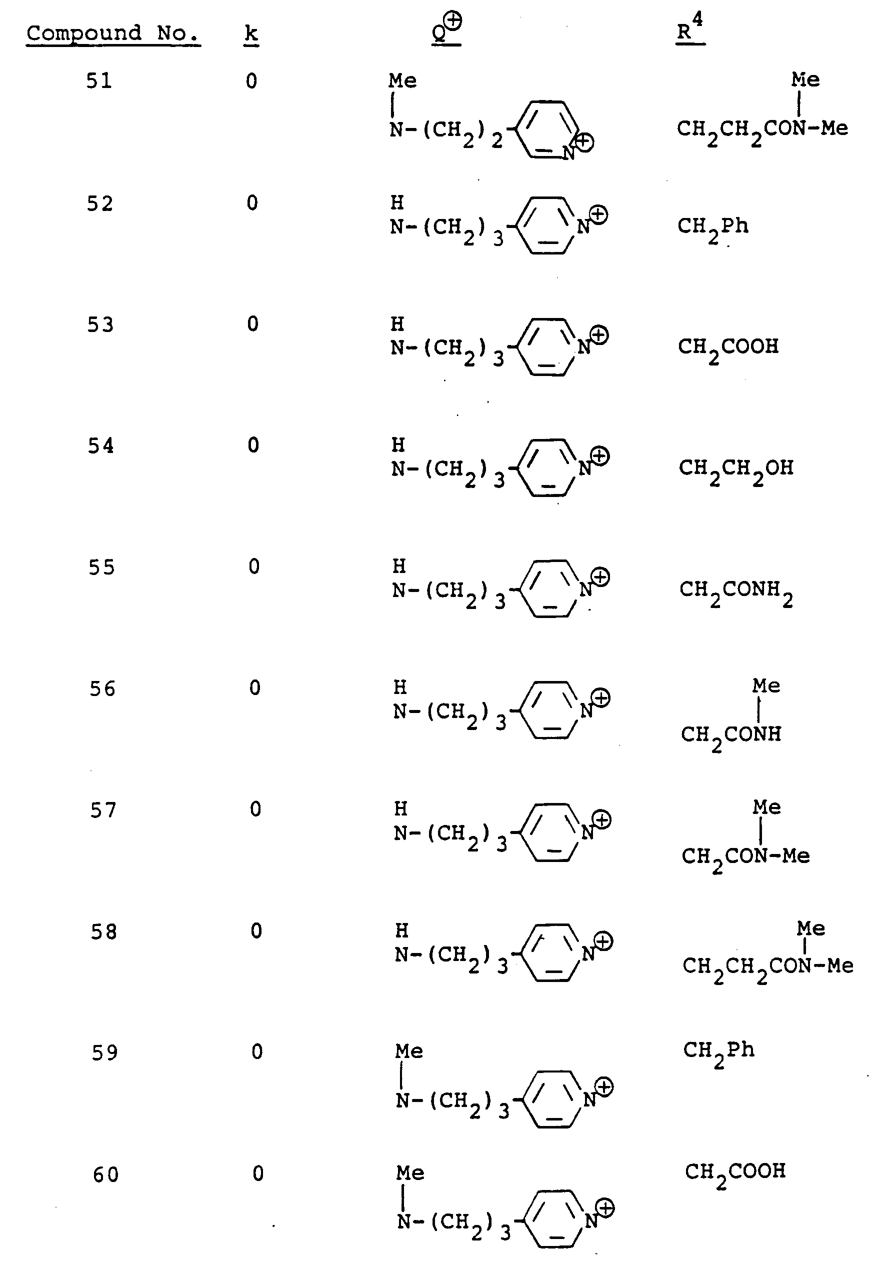

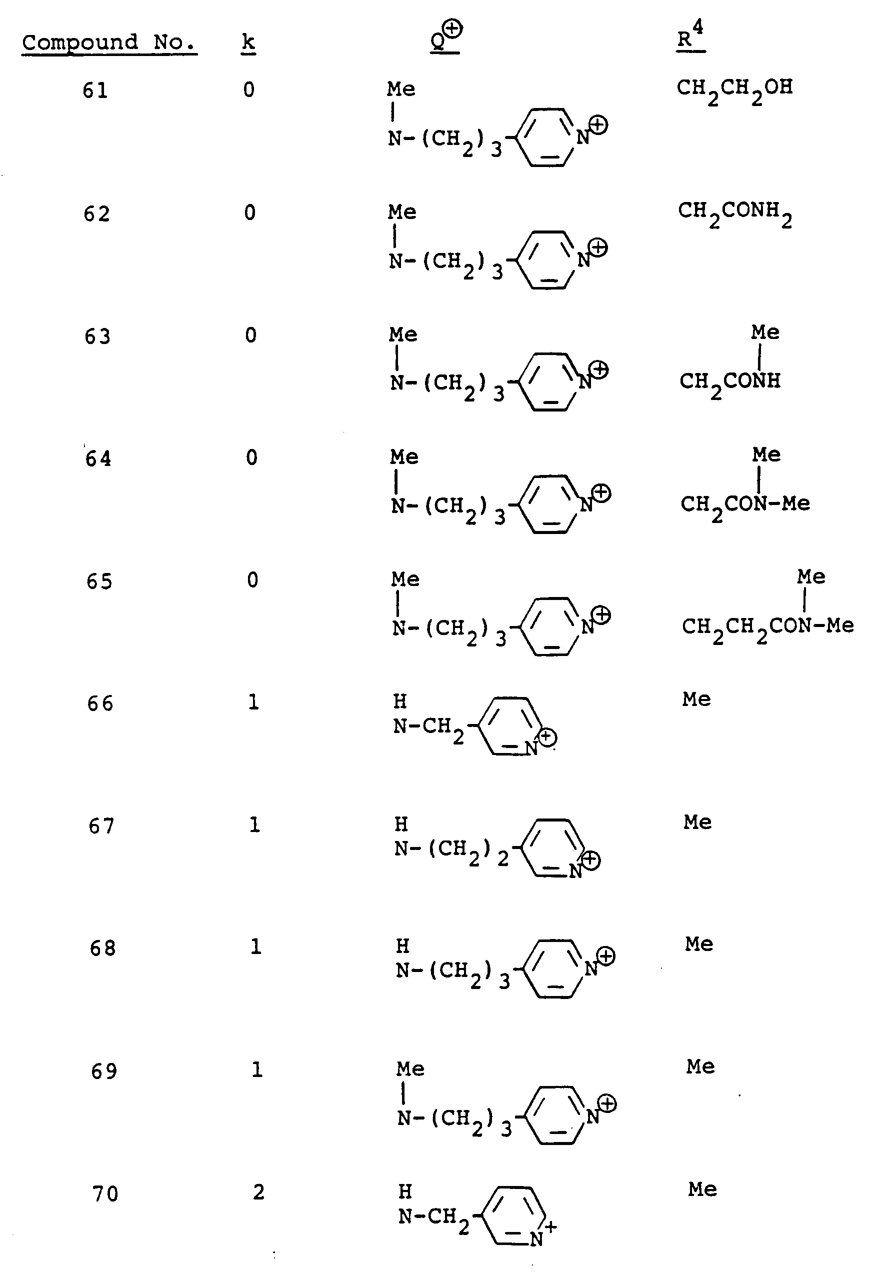

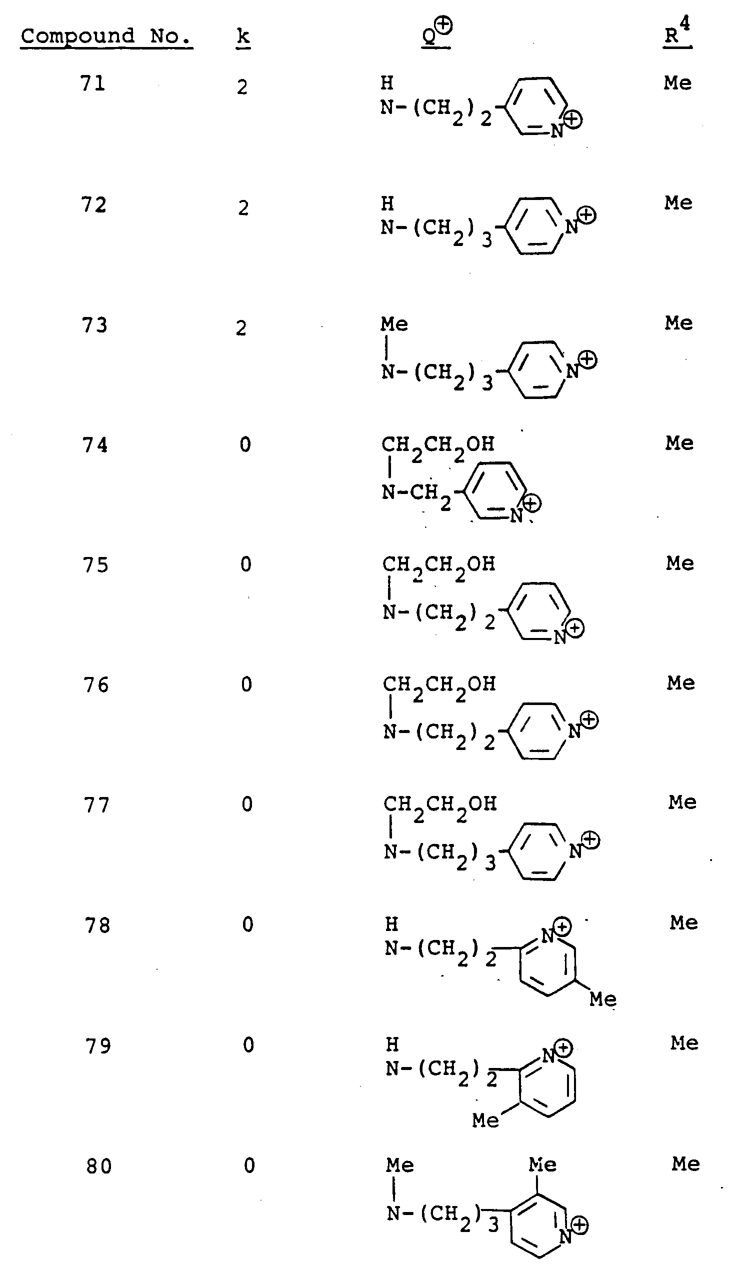

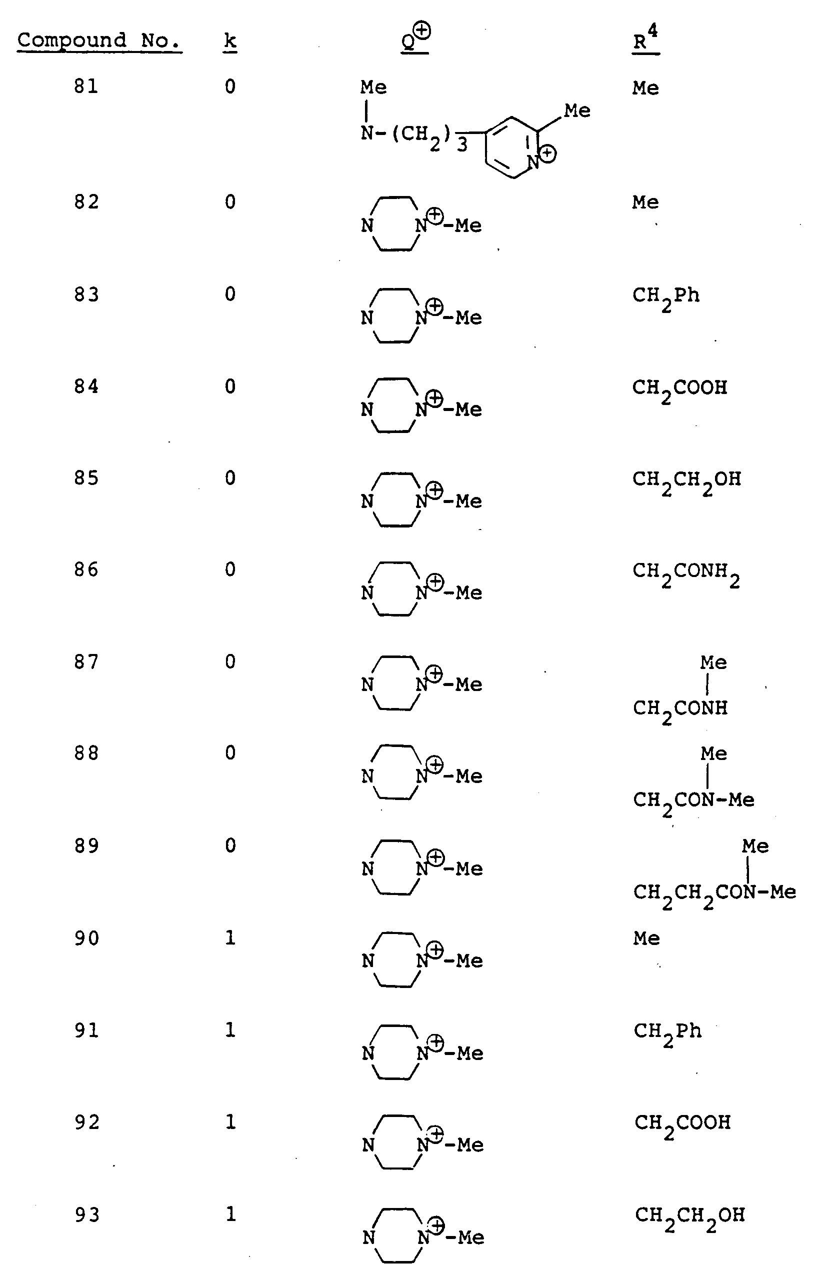

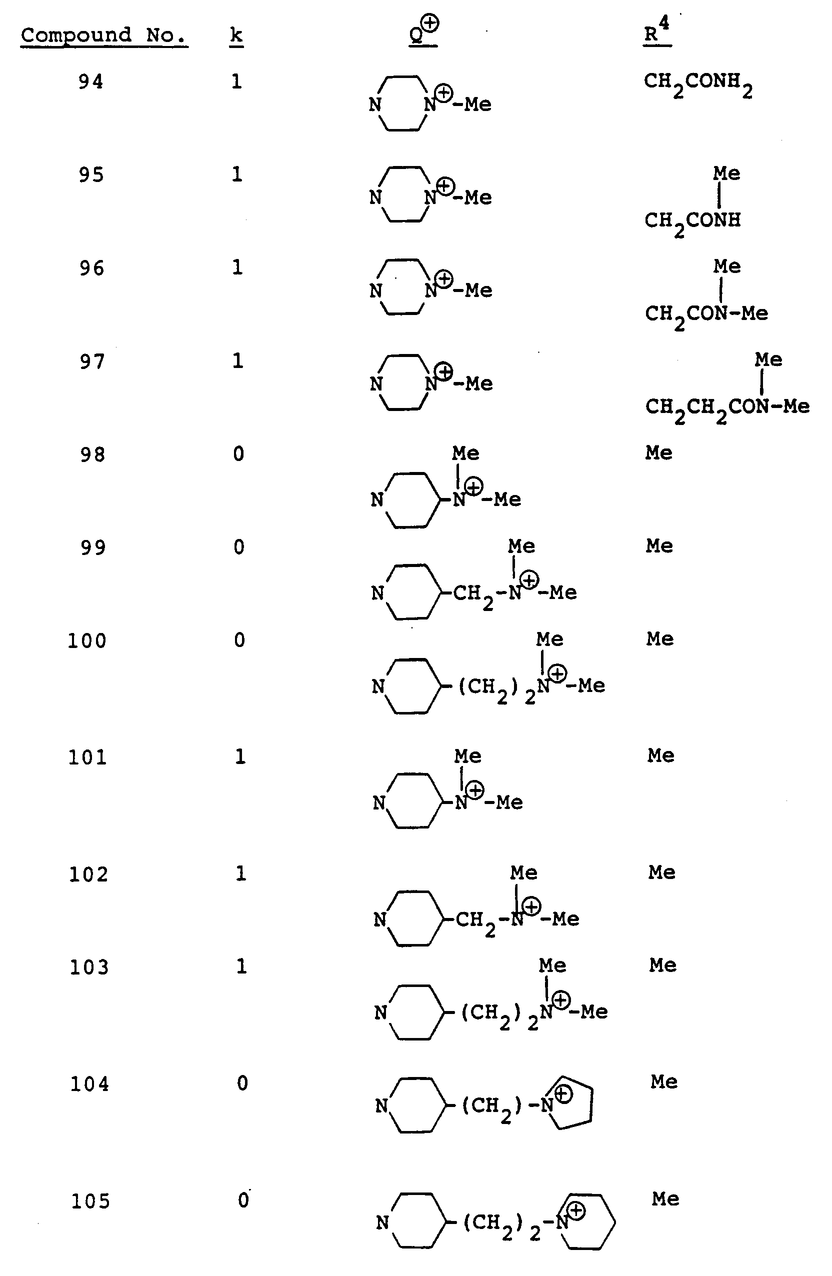

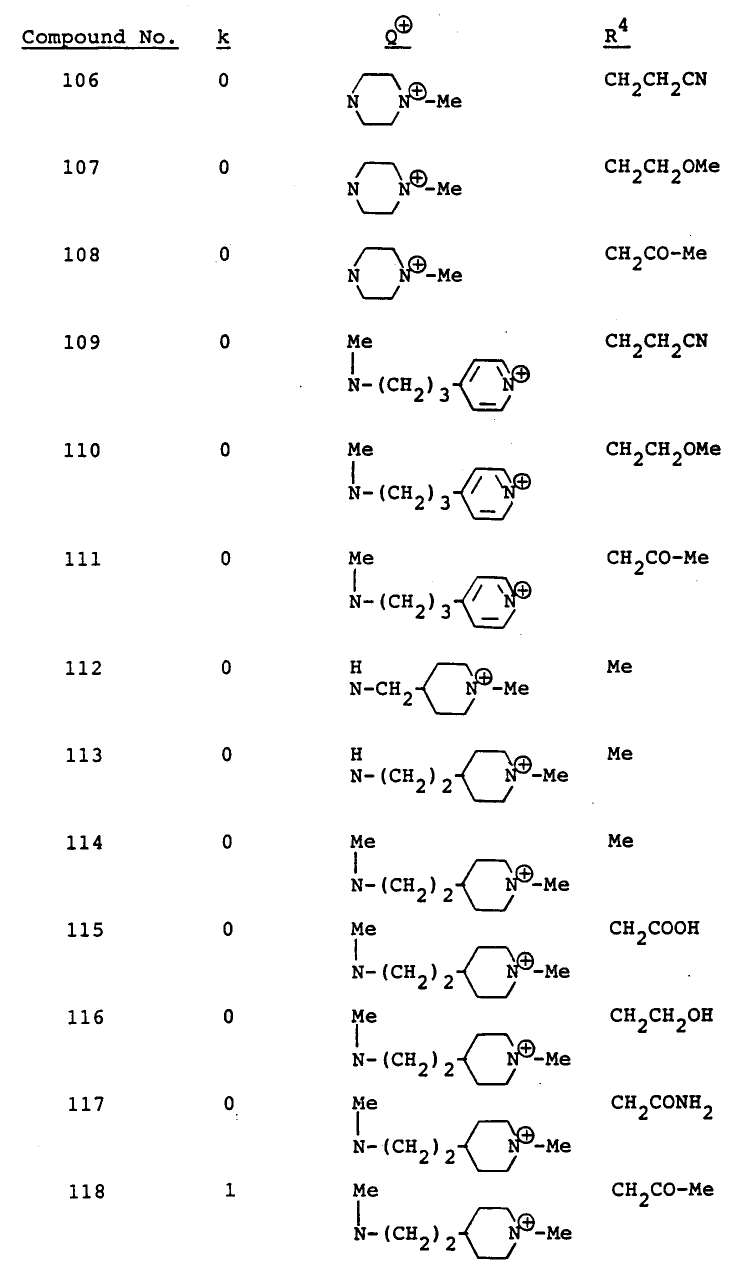

The β-lactam compounds as exemplified in Table 1 have their optical and steric isomers, and all of them are included within the scope of the present invention.

The β-lactam compounds (I) according to the invention are characteristic in having a 2-substituted pyrrolidin-4-ylthio group introduced with a quaternary ammonium group at the 3-position and a lower alkyl group at the 4-position in the carbapenem skeleton. Due to such characteristic structure, the β-lactam compounds (I) exert an excellent antimicrobial activity against Gram-positive and Gram-negative bacteria including Staphylococcus aureus, Streptococcus pyogenes, Escherichia coli, Serratia marcescens and Pseudomonas aeruginosa. It is notable that while conventional carbapenem compounds such as imipenem are generally unstable in a living body, especially sensitive to renal DHP-I, the β-lactam compounds (I), particularly those wherein R¹ is a methyl group in the R-configuration, are in general significantly resistant to renal DHP-I. It is also notable that the half life time (T½) of the β-lactam compounds (I) in a living body is generally longer than that of conventional carbapenem compounds such as imipenem. The β-lactam compounds (I) are thus useful as antimicrobial drugs or intermediates in the synthesis of such antimicrobial drugs.

For the practical usage of the β-lactam compounds (I) as antimicrobial drugs, they may be formulated into conventional preparation forms together with excipients or additives such as carriers, diluents, binders and stabilizers and administered in various modes, of which examples are oral administration in the form of tablets, capsules, dispersants and syrups, non-oral administration in the form of injection through vein, muscle or rectum. When they are applied in injection forms, the preparations may additionally include buffering agents, solubilizing agents and isotonic agents. The daily dosage may vary depending upon the state of disease, the age and body weight of patients, the administration mode and time, and the normal daily dosage to a human adult is between about 100 to 3000 mg, optionally divided in one to several times per day. If necessary, the dosage may be increased or decreased appropriately.

Practical and presently preferred embodiments of the invention are illustratively shown in the following Examples, which are not intended to limit the scope of the invention thereto. Further, the abbreviations used therein show the following meanings: PNZ, p-nitrobenzyloxycarbonyl; PNB, p-nitrobenzyl; Ph, phenyl; Ac, acetyl; TBDMS, t-butyldimethylsilyl; Me, methyl.

### Example 1

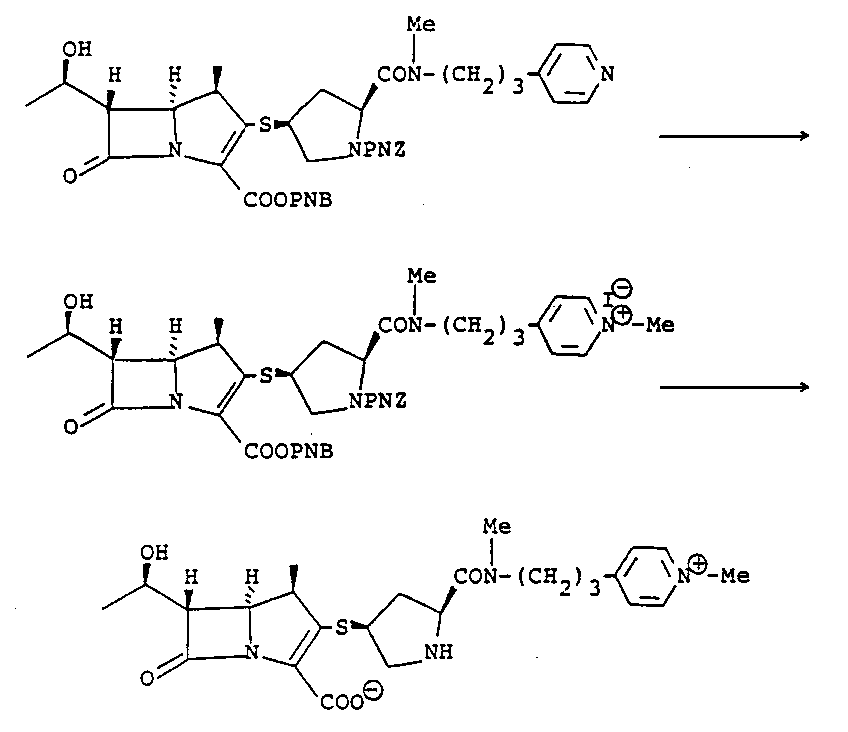

To a solution of (4R,5S,6S,8R,2'S,4'S)-p-nitrobenzyl-3-[1-p-nitrobenzyloxycarbonyl-2-((3-(4-pyridyl)propyl)methylaminocarbonyl)pyrrolidin-4-ylthio]-4-methyl-6-(1-hydroxyethyl)-1-azabicyclo[3.2.0]hept-2-en-7-one-2-carboxylate (100 mg) in acetone (2.0 ml), methyl iodide (1.14 g) was added, and the resultant mixture was stirred at room temperature for 20 hours, followed by removal of the solvent under reduced pressure. The residue was dissolved in tetrahydrofuran (5.0 ml) and 0.1M phosphate buffer (pH, 7.0; 5.0 ml), and 10 % palladium-carbon (150 mg) was added thereto. Catalytic reduction was performed at room temperature for 1.5 hours under atmospheric pressure of hydrogen. The catalyst was removed by filtration, and the filtrate was washed with dichloromethane three times. After removal of the solvent from the washed filtrate under reduced pressure, the residue was purified by polymer chromatography (CHP-20P) using 2 % aqueous tetrahydrofuran as an eluent. The eluted fractions were collected and freeze-dried to give (4R,5S,6S,8R,2'S,4'S)-3-[2-((3-(1-methylpyridinium-4-yl)propyl)methylaminocarbonyl)pyrrolidin-4-ylthio]-4-methyl-6-(1-hydroxyethyl)-1-azabicyclo[3.2.0]hept-2-en-7-one-2-carboxylate.
- UVₘₐₓ nm (H₂O):: 257, 263 (sh), 298;
- IRₘₐₓ cm⁻¹ (KBr):: 3400, 1737, 1682, 1367;
- NMR δ (D₂O):: 1.18 (3H, d, J = 7.3 Hz), 1.26 (3H, d, J = 6.6 Hz), 3.04 (3H, s), 4.20 (3H, s), 7.87 (2H, d, J = 6.6 Hz), 8.60 (2H, d, J = 6.6 Hz).

### Example 2

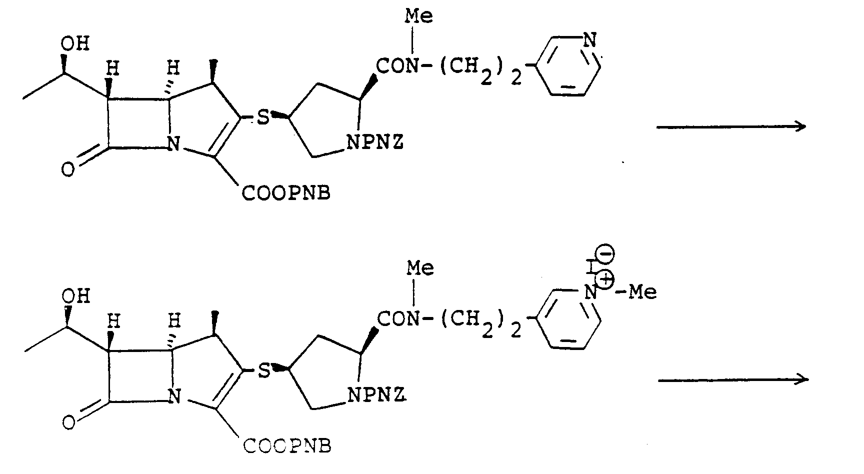

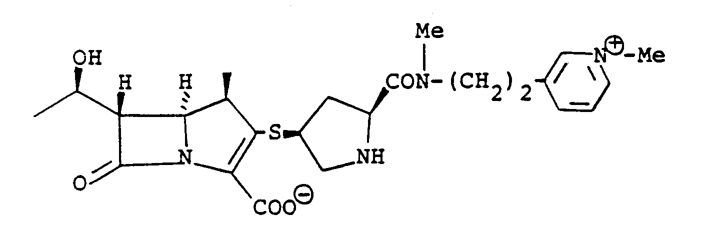

To a solution of (4R,5S,6S,8R,2'S,4'S)-p-nitrobenzyl-3-[1-p-nitrobenzyloxycarbonyl-2-((2-(3-pyridyl)ethyl)methylaminocarbonyl)pyrrolidin-4-ylthio]-4-methyl-6-(1-hydroxyethyl)-1-azabicyclo[3.2.0]hept-2-en-7-one-2-carboxylate (270 mg) in acetone (25 ml), methyl iodide (3.42 g) was added, and the resultant mixture was stirred at room temperature for 3 hours, followed by removal of the solvent under reduced pressure. The residue was dissolved in tetrahydrofuran (15 ml) and 0.1M phosphate buffer (pH, 7.0; 15.0 ml), and 10 % palladium-carbon (500 mg) was added thereto. Catalytic reduction was performed at room temperature for 1 hour under atmospheric pressure of hydrogen. The reaction mixture was subjected to post-treatment in the same manner as in Example 1. The filtrate was purified by polymer chromatography (CHP-20P) using 2 % aqueous tetrahydrofuran as an eluent to give (4R,5S,6S,8R,2'S,4'S)-3-[2-((2-methylpyridinium-3-yl)ethyl)methylaminocarbonyl)pyrrolidin-4-ylthio]-4-methyl-6-(1-hydroxyethyl)-1-azabicyclo[3.2.0]hept-2-en-7-one-2-carboxylate.
- UVₘₐₓ nm (H₂O):: 268, 273, 298;
- IRₘₐₓ cm⁻¹ (KBr):: 3320, 1748, 1637, 1585, 1378;
- NMR δ (D₂O):: 1.20 (3H, d, J = 7.3 Hz), 1.27 (3H, d, J = 6.3 Hz), 2.81 (1H, m), 3.00 - 3.30 (5H, m), 3.09 (3H, s), 3.45 (3H, m), 3.79 (1H, m), 4.20 (4H, m), 4.38 (3H, s), 7.98 (1H, dd, J = 6.3 and 8.3 Hz), 8.44 (1H, d, J = 8.3 Hz), 8.69 (1H, d, J = 6.3 Hz), 8.79 (1H, s).

### Example 3

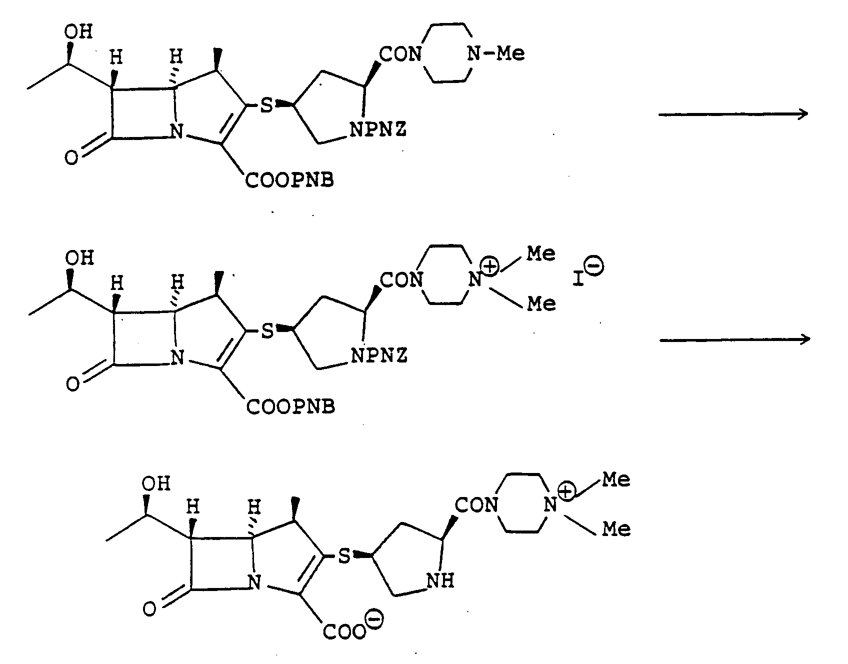

To a solution of (4R,5S,6S,8R,2'S,4'S)-p-nitrobenzyl-3-[1-p-nitrobenzyloxycarbonyl-2-(4-methylpiperazin-1-ylcarbonyl)pyrrolidin-4-ylthio]-4-methyl-6-(1-hydroxyethyl)-1-azabicyclo[3.2.0]hept-2-en-7-one-2-carboxylate (200 mg) in acetone (2.0 ml), methyl iodide (1.14 g) was added, and the resultant mixture was stirred at room temperature for 20 hours, followed by removal of the solvent under reduced pressure. The residue was dissolved in tetrahydrofuran (10.0 ml) and 0.1M phosphate buffer (pH, 7.0; 10.0 ml), and 10 % palladium-carbon (241 mg) was added thereto. Catalytic reduction was performed at room temperature for 1.5 hours under atmospheric pressure of hydrogen. The reaction mixture was subjected to post-treatment in the same manner as in Example 1. The filtrate was purified by polymer chromatography (CHP-20P) using 1 % aqueous tetrahydrofuran as an eluent to give (4R,5S,6S,8R,2'S,4'S)-3-[2-(4,4-dimethylpiperazinium-1-yl-carbonyl)pyrrolidin-4-ylthio]-4-methyl-6-(1-hydroxyethyl)-1-azabicyclo[3.2.0]hept-2-en-7-one-2-carboxylate.
- UVₘₐₓ nm (H₂O):: 299;
- IRₘₐₓ cm⁻¹ (KBr):: 3440, 1745, 1640, 1587, 1464, 1387, 1260;
- NMR δ (D₂O):: 1.21 (3H, d, J = 7.3 Hz), 1.29 (3H, d, J = 6.6 Hz), 1.72 (1H, m), 2.78 (1H, m) , 3.11 (1H, dd, J = 4.0 & 12.5 Hz), 3.27 (6H, s), 3.20 - 3.60 (9H, m), 3.80 - 4.20 (5H, m), 4.23 (3H, m).

### Examples 4 to 22

In the same manner as above, the compounds as shown in Table 2 were obtained. The physical properties of the compounds as obtained follow the Table.

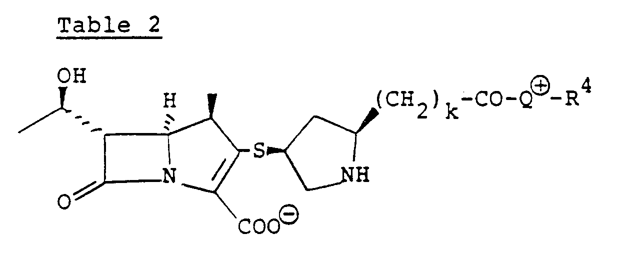

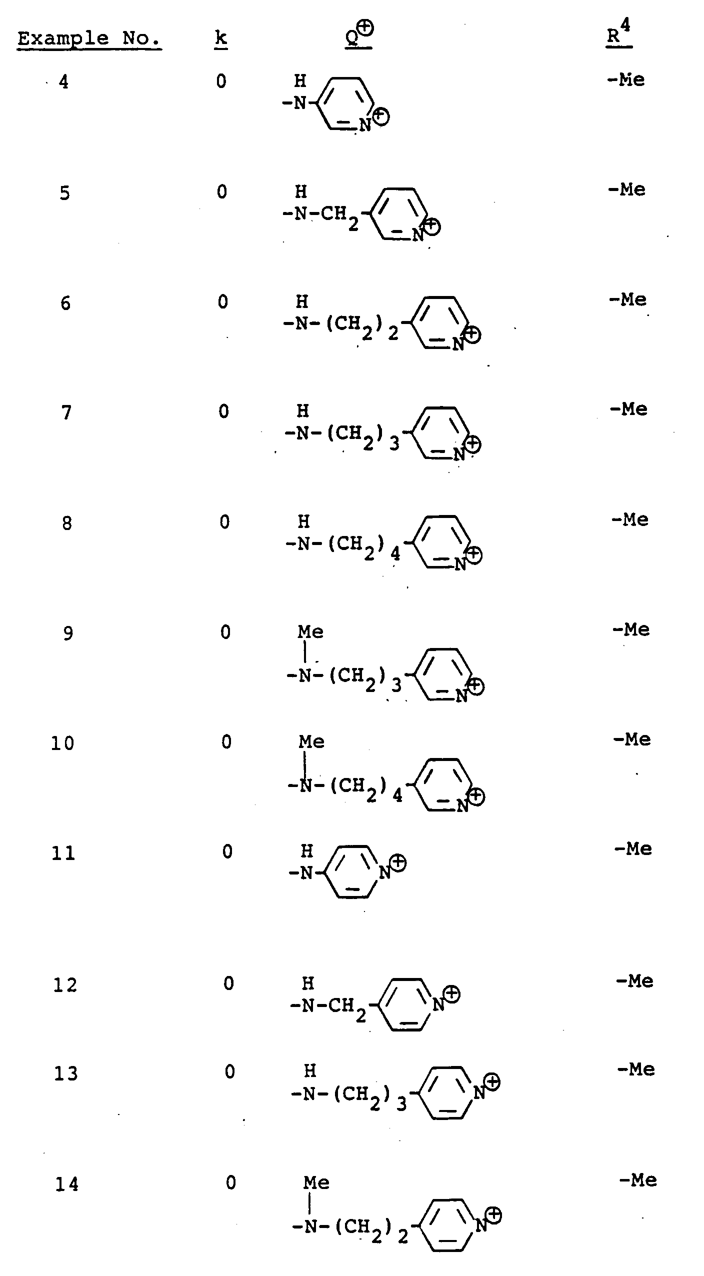

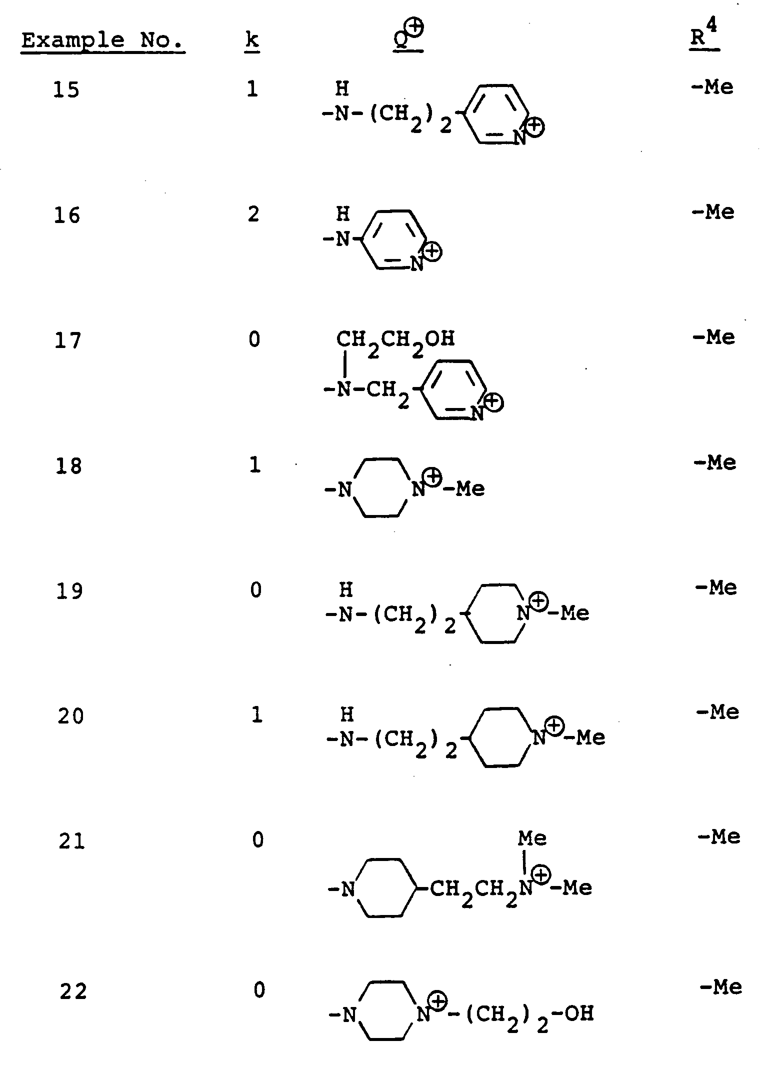

### Physical properties

### Example 4

- UVₘₐₓ nm (H₂O):: 252, 291;
- IRₘₐₓ cm⁻¹ (KBr):: 3380, 1737, 1580, 1502, 1364;
- NMR δ (D₂O) :: 1.19 (3H, d, J = 7.3 Hz), 1.29 (3H, d, J = 6.3 Hz), 1.95 (1H, m), 2.73 (1H, m), 3.00 (1H, dd, J = 4.6 & 11.9 Hz), 3.40 (3H, m), 3.79 (1H, m), 4.09 (1H, dd, J = 5.6 & 9.6 Hz), 4.25 (2H, m), 4.40 (3H, s), 7.99 (1H, dd, J = 6.3 & 8.6 Hz), 8.47 (1H, d, J = 8.6 Hz), 8.53 (1H, d, J = 6.3 Hz), 9.27 (1H, s).

### Example 5

- UVₘₐₓ nm (H₂O):: 265, 273 (sh), 296;
- IRₘₐₓ cm⁻¹ (KBr):: 3360, 1730, 1670, 1590, 1385;
- NMR δ (D₂O):: 1.19 (3H, d, J = 7.3 Hz), 1.25 (1H, m), 1.27 (3H, d, J = 6.3 Hz), 2.17 (1H, m), 3.02 (1H, m), 3.30 - 3.85 (4H, m), 4.08 (1H, m), 4.22 (2H, m), 4.37 (3H, s), 4.65 (3H, m) 8.02 (1H, t, J = 7.9 Hz), 8.46 (1H, d, J = 8.3 Hz), 8.71 (1H, d, J = 6.0 Hz), 8.77 (1H, s).

### Example 6

- UVₘₐₓ nm (H₂O):: 266, 272, 299;
- IRₘₐₓ cm⁻¹ (KBr):: 3300, 1750, 1652, 1588, 1380, 1366;
- NMR δ (D₂O):: 1.19 (3H, d, J = 7.3 Hz), 1.29 (3H, d, J = 6.3 Hz), 1.49 (1H, m), 2.69 (1H, m), 2.91 (1H, dd, J = 4.5 & 11.9 Hz), 3.10 (2H, t, J = 6.6 Hz), 3.30 - 3.50 (3H, m), 3.63 (2H, t, J = 5.9 Hz), 3.75 (1H, m), 3.93 (1H, dd, J = 6.3 & 9.6 Hz), 4.20 - 4.30 (2H, m), 4.37 (3H, s), 7.99 (1H, dd, J = 6.2 & 8.0 Hz), 8.44 (1H, d, J = 8.0 Hz), 8.67 (1H, d, J = 6.2 Hz), 8.77 (1H, s).

### Example 7

- UVₘₐₓ nm (H₂O):: 266, 273 (sh), 299;
- IRₘₐₓ cm⁻¹ (KBr):: 3410, 1749, 1640, 1588, 1380, 1278, 1255, 1178, 1140;
- NMR δ (D₂O):: 1.19 (3H, d, J = 7.3 Hz), 1.29 (3H, d, J = 6.3 Hz), 1.75 (1H, m), 1.96 (2H, m), 2.69 (1H, m), 2.89 (2H, m), 2.95 (1H, dd, J = 4.3 & 11.9 Hz), 3.20 - 3.50 (5H, m), 3.75 (1H, m), 3.87 (1H, dd, J = 6.3 & 9.5 Hz), 4.20 (2H, m), 4.35 (3H, s), 7.94 (1H, t, J = 7.0 Hz), 8.38 (1H, d, J = 7.9 Hz), 8.61 (1H, d, J = 6.0 Hz), 8.68 (1H, s).

### Example 8

- UVₘₐₓ nm (H₂O):: 266, 273, 299;
- NMR δ (D₂O):: 1.19 (3H, d, J = 7.0 Hz), 1.30 (3H, d, J = 6.6 Hz), 1.61 (2H, m), 1.73 (3H, m), 2.72 (1H, m), 2.89 (2H, m), 2.99 (1H, dd, J = 4.6 & 11.9 Hz), 3.29 (2H, m), 3.43 (3H, m), 3.79 (1H, m), 3.92 (1H, dd, J = 6.3 & 9.2 Hz), 4.23 (2H, m), 4.34 (3H, s), 7.93 (1H, dd, J = 6.3 & 8.3 Hz), 8.39 (1H, d, J = 8.3 Hz), 8.59 (1H, d, J = 6.3 Hz), 8.68 (1H, s).

### Example 9

- UVₘₐₓ nm (H₂O):: 268, 273, 297;
- IRₘₐₓ cm⁻¹ (KBr):: 3425, 1744, 1632, 1588, 1380, 1281;
- NMR δ (D₂O):: 1.21 (3H, d, J = 7.3 Hz), 1.30 (3H, d, J = 6.3 Hz), 1.82 (1H, m), 1.98 (2H, m), 2.86 (3H, m), 3.09 (3H, s), 3.20 - 3.70 (6H, m), 3.95 (1H, m), 4.24 (2H, m), 4.36 (3H, s), 4.48 (1H, dd, J = 6.9 & 9.6 Hz), 7.97 (1H, t, J = 7.0 Hz), 8.42 (1H, d, J = 7.6 Hz), 8.62 (1H, d, J = 6.3 Hz), 8.70 (1H, s).

### Example 10

- UVₘₐₓ nm (H₂O):: 268, 276, 296;
- IRₘₐₓ cm⁻¹ (KBr):: 3425, 1746, 1636, 1592, 1378, 1282, 1246;
- NMR δ (D₂O):: 1.18 (3H, d, J = 7.3 Hz), 1.28 (3H, d, J = 6.3 Hz), 1.66 (4H, m), 1.86 (1H, m), 2.93 (3H, m), 3.03 (3H, s), 3.20 - 3.80 (7H, m), 4.01 (1H, m), 4.23 (2H, m), 4.32 (3H, s), 7.92 (1H, t, J = 7.0 Hz), 8.39 (1H, d, J = 7.5 Hz), 8.56 (1H, d, J = 6.2 Hz), 8.65 (1H, s).

### Example 11

- UVₘₐₓ nm (H₂O):: 273, 300;
- IRₘₐₓ cm⁻¹ (KBr):: 3400, 1777, 1730, 1618;
- NMR δ (D₂O):: 1.20 (3H, d, J = 7.3 Hz), 1.28 (3H, d, J = 6.6 Hz), 2.14 (1H, m), 2.68 (1H, m), 2.94 (1H, m), 3.25 - 3.50 (3H, m), 3.63 (1H, dd, J = 6.3 & 11.9 Hz), 3.73 (1H, m), 4.02 (1H, m), 4.23 (3H, s), 4.47 (1H, m), 8.10 (2H, d, J = 7.3 Hz), 8.57 (2H, d, J = 7.3 Hz).

### Example 12

- UVₘₐₓ nm (H₂O):: 258, 263 (sh), 297;
- IRₘₐₓ cm⁻¹ (KBr):: 3420, 1743, 1638, 1582, 1381;
- NMR δ (D₂O):: 1.19 (3H, d, J = 7.3 Hz), 1.28 (3H, d, J = 6.6 Hz), 1.91 (1H, m), 2.76 (1H, m), 3.06 (1H, dd, J = 4.0 & 11.9 Hz), 3.43 (3H, m), 3.80 (1H, m), 4.10 (1H, dd, J = 5.9 & 9.7 Hz), 4.21 (2H, m), 4.34 (3H, s), 4.68 (1H, d, J = 18.2 Hz), 4.70 (1H, d, J = 18.2 Hz), 7.90 (2H, d, J = 6.3 Hz), 8.69 (2H, d, J = 6.3 Hz).

### Example 13

- UVₘₐₓ nm (H₂O):: 256, 263, 299;
- IRₘₐₓ cm⁻¹ (KBr):: 3410, 1743, 1639, 1584, 1376;
- NMR δ (D₂O):: 1.20 (3H, d, J = 7.3 Hz), 1.31 (3H, d, J = 6.3 Hz), 1.79 (1H, m), 2.01 (2H, m), 2.72 (1H, m) , 2.99 (3H, m), 3.20 - 3.50 (5H, m), 3.79 (1H, m), 3.92 (1H, m), 4.30 (2H, m), 4.33 (3H, s), 7.89 (2H, d, J = 6.6 Hz), 8.62 (2H, d, J = 6.6 Hz).

### Example 14

- UVₘₐₓ nm (H₂O):: 257, 263 (sh), 300;
- NMR δ (D₂O):: 1.18 (3H, d, J = 7.3 Hz), 1.26 (3H, d, J = 6.3 Hz), 1.80 (1H, m), 2.77 (1H, m), 3.05 (3H, s), 4.28 (3H, s), 7.93 (2H, d, J = 6.6 Hz), 8.64 (2H, d, J = 6.6 Hz).

### Example 15

- UVₘₐₓ nm (H₂O):: 269, 273, 296;
- IRₘₐₓ cm⁻¹ (KBr):: 3380, 1742, 1652, 1580, 1370, 1239, 1008;
- NMR δ (D₂O):: 1.18 (3H, d, J = 6.9 Hz), 1.26 (3H, d, J = 6.3 Hz), 1.92 (1H, m), 2.42 (1H, m), 2.56 (2H, m), 3.04 (3H, m), 3.25 - 3.70 (5H, m), 3.80 (1H, m), 3.96(1H, m), 4.26 (2H, m), 4.35 (3H, s), 7.95 (1H, t, J = 7.6 Hz), 8.40 (1H, d, J = 8.6 Hz), 8.65 (1H, d, J = 6.0 Hz), 8.72 (1H, s).

### Example 16

- UVₘₐₓ nm (H₂O):: 249, 294;
- NMR δ (D₂O):: 1.21 (3H, d, J = 6.9 Hz), 1.29 (3H, d, J = 6.3 Hz), 1.70 (1H, m), 2.15 (2H, m), 2.73 (2H, m), 3.20 - 3.50 (3H, m), 3.50 - 3.85 (3H, m), 4.10 (1H, m), 4.18 (2H, m), 4.38 (3H, s), 7.92 (1H, m), 8.37 (1H, d, J = 7.9 Hz), 8.51 (1H, d, J = 5.9 Hz), 9.27 (1H, s).

### Example 17

- UVₘₐₓ nm (H₂O):: 267, 272, 300;
- IRₘₐₓ cm⁻¹ (KBr):: 3410, 1746, 1638, 1586, 1383;
- NMR δ (D₂O):: 1.22 (3H, d, J = 7.3 Hz), 1.30 (3H, d, J = 6.3 Hz), 1.70 (1H, m), 2.85 (1H, m), 3.12 (1H, dd, J = 3.6 & 12.2 Hz), 3.25 (1H, dd, J= 5.3 & 12.2 Hz), 3.43 (2H, m), 3.70 - 3.90 (5H, m), 4.24 (2H, m), 4.39 (1H, m), 4.40 (3H, s), 4.80 (1H, d, J = 16.5 Hz), 4.92 (1H, d, J = 16.5 Hz), 8.03 (1H, dd, J = 6.5 & 7.9 Hz), 8.43 (1H, d, J = 7.9 Hz), 8.71 (2H, m).

### Example 18

- UVₘₐₓ nm (H₂O):: 297;
- IRₘₐₓ cm⁻¹ (KBr):: 3400, 1748, 1639, 1586, 1453, 1372, 1257, 1088;
- NMR δ (D₂O):: 1.17 (3H, d, J = 7.3 Hz), 1.24 (3H, d, J = 6.3 Hz), 1.49 (1H, m), 2.61 (1H, m), 2.90 (2H, d, J = 6.9 Hz), 3.08 (1H, dd, J = 3.3 & 12.5 Hz), 3.21 (6H, s), 3.30 - 3.60 (7H, m), 3.73 (1H, t, J = 7.3 Hz), 3.90 (5H, m), 4.20 (2H, m).

### Example 19

- UVₘₐₓ nm (H₂O):: 300;
- IRₘₐₓ cm⁻¹ (KBr):: 3410, 1744, 1650, 1588, 1485, 1381, 1250, 1206, 1092;
- NMR δ (D₂O):: 1.22 (3H, d, J = 7.0 Hz), 1.31 (3H, d, J = 6.3 Hz), 1.70 (6H, m), 2.00 (2H, m), 2.74 (1H, m), 3.04 (1H, dd, J = 5.0 & 12.2 Hz), 3.08 (3H, s), 3.15 (3H, s), 3.25 - 3.60 (9H, m), 3.81 (1H, m), 3.97 (1H, dd, J = 6.4 & 9.5 Hz), 4.24 (2H, m).

### Example 20

- UVₘₐₓ nm (H₂O):: 297;
- IRₘₐₓ cm⁻¹ (KBr):: 3400, 1742, 1635, 1582, 1479, 1363, 1241, 1201, 1082;
- NMR δ (D₂O):: 1.28 (3H, d, J = 7.3 Hz), 1.30 (3H, d, J = 6.3 Hz), 1.70 (6H, m), 1.97 (2H, m), 2.72 (1H, m), 2.79 (1H, m), 3.08 (3H, s), 3.15 (3H, s), 3.23 - 3.70 (10H, m), 4.02 (2H, m), 4.31 (2H, m).

### Example 21

- UVₘₐₓ nm (H₂O):: 295;
- IRₘₐₓ cm⁻¹ (KBr):: 3440, 1758, 1644, 1597, 1492, 1379, 1258;
- NMR δ (D₂O):: 1.21 (3H, d, J = 7.0 Hz), 1.29 (3H, d, J = 6.6 Hz), 1.80 (7H, m), 2.86 (1H, m), 3.02 (1H, m), 3.11 (9H, s), 3.18 (2H, m), 3.39 (5H, m), 3.60 (1H, m), 3.80 (1H, m), 3.99 (1H, m), 4.24 (2H, m), 4.34 (1H, m), 4.64 (1H, m).

### Example 22

- UVₘₐₓ nm (H₂O):: 297;
- IRₘₐₓ cm⁻¹ (KBr):: 3400, 1743, 1637, 1588, 1380;
- NMR δ (D₂O):: 1.22 (3H, d, J = 6.9 Hz), 1.30 (3H, d, J = 6.3 Hz), 1.68 (1H, m), 3.10 (1H, dd, J = 4.0 & 12.5 Hz), 2.76 (1H, m), 3.24 (1H, dd, J = 5.4 & 12.5 Hz), 3.31 (3H, s), 3.43 (2H, m), 3.68 (8H, m) 3.86 (1H, m), 3.97 (2H, m), 4.12 (2H, m), 4.25 (3H, m).

### Example 23

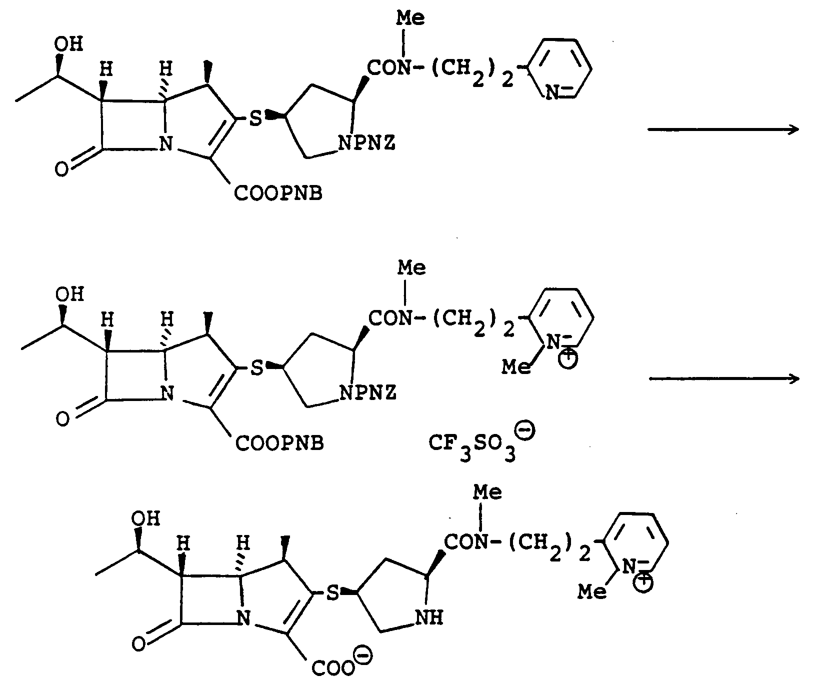

A solution of (4R,5S,6S,8R,2'S,4'S)-p-nitrobenzyl-3-[1-(p-nitrobenzyloxycarbonyl-2-((2-(2-pyridyl)ethyl)methylaminocarbonyl)pyrrolidin-4-ylthio]-4-methyl-6-(1-hydroxyethyl)-1-azabicyclo[3.2.0]hept-2-en-7-one-2-carboxylate (208 mg) in dry dichloromethane (3.0 ml) was stirred under ice-cooling, and methyl trifluoromethanesulfonate (64 mg) was dropwise added thereto, followed by stirring at the same temperature for 1 hour. The reaction mixture was combined with tetrahydrofuran (10.0 ml), 0.1M phosphate buffer (pH, 7.0; 10.0 ml) and 10 % palladium-carbon (350 mg), and catalytic reduction was performed at room temperature for 1 hour under atmospheric pressure of hydrogen. The reaction mixture was subjected to post-treatment in the same manner as in Example 1. The filtrate was purified by polymer chromatography (CHP-20P) using 2 % aqueous tetrahydrofuran as an eluent to give (4R,5S,6S,8R,2'S,4'S)-3-[2-((2-(1-methylpyridinium-2-yl)ethyl)methylaminocarbonyl)pyrrolidin-4-ylthio]-4-methyl-6-(1-hydroxyethyl)-1-azabicyclo[3.2.0]hept-2-en-7-one-2-carboxylate.
- UVₘₐₓ nm (H₂O):: 269, 274, 298;
- IRₘₐₓ cm⁻¹ (KBr):: 3450, 1737, 1625, 1580, 1372, 1251, 1153;
- NMR δ (D₂O):: 1.20 (3H, d, J = 7.2 Hz), 1.28 (3H, d, J = 6.3 Hz), 1.60 (1H, m), 3.00 (1H, m), 3.16 (3H, s), 3.26 (1H, dd, J = 3.3 & 12.2 Hz), 3.30 - 3.60 (5H, m), 3.65 (1H, m), 3.94 (1H, m), 4.13 (1H, m), 4.23 (2H, m), 4.40 (3H, s), 4.52 (1H, dd, J = 7.2 & 9.9 Hz), 7.90 (1H, d, J = 7.9 Hz), 7.92 (1H, t, J = 6.0 Hz), 8.45 (1H, t, J = 7.9 Hz), 8.76 (1H, d, J = 6.0 Hz).

### Examples 24 to 31

In the same manner as in Example 23, the compounds as shown in Table 3 were obtained. The physical properties of the compounds as obtained follow the Table.

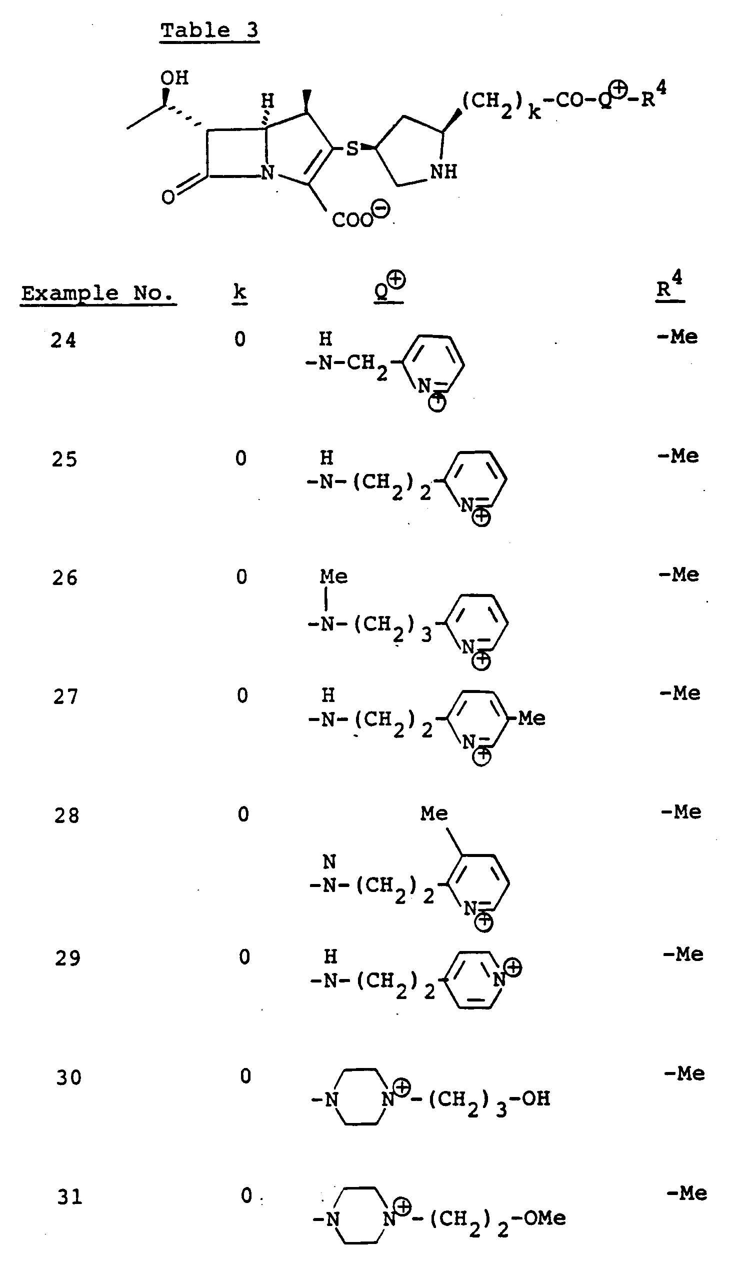

### Physical properties

### Example 24

- UVₘₐₓ nm (H₂O):: 267, 274 (sh), 298;
- IRₘₐₓ cm⁻¹ (KBr):: 3430, 1743, 1679, 1577, 1380, 1260, 1158;
- NMR δ (D₂O):: 1.21 (3H, d, J = 7.3 Hz), 1.28 (3H, d, J = 6.3 Hz), 2.20 (1H, m), 3.03 (1H, m), 3.03 - 3.60 (3H, m), 3.76 (1H, dd, J = 6.3 & 12.2 Hz), 4.07 (1H, m), 4.23 (2H, m), 4.34 (3H, s), 4.64 (1H, dd, J = 6.6 & 8.9 Hz), 4.89 (1H, d, J = 18.2 Hz), 4.90 (1H, d, J = 18.2 Hz), 7.95 (1H, t, J = 7.0 Hz) 7.96 (1H, d, J = 8.0 Hz), 8.52 (1H, t, J = 8.0 Hz), 8.79 (1H, d, J = 6.3 Hz).

### Example 25

- UVₘₐₓ nm (H₂O):: 268, 273 (sh), 297;
- IRₘₐₓ cm⁻¹ (KBr):: 3440, 1750, 1672, 1630, 1580, 1379, 1270;
- NMR δ (D₂O):: 1.19 (3H, d, J = 7.3 Hz), 1.28 (3H, d, J = 6.3 Hz), 1.88 (1H, m), 2.89 (1H, m), 3.30 - 3.50 (5H, m), 3.70 (2H, m), 3.85 (1H, dd, J = 6.9 & 14.2 Hz), 4.00 (1H, m), 4.24 (2H, m), 4.36 (3H, s), 4.41 (1H, dd, J = 6.6 & 9.6 Hz), 7.90 (2H, m), 8.46 (1H, t, J = 6.6 Hz), 8.76 (1H, d, J = 6.1 Hz).

### Example 26

- UVₘₐₓ nm (H₂O):: 267, 273 (sh), 296;
- IRₘₐₓ cm⁻¹ (KBr):: 3450, 1745, 1640, 1584, 1380, 1255, 1159;
- NMR δ (D₂O):: 1.21 (3H, d, J = 6.9 Hz), 1.30 (3H, d, J = 6.3 Hz), 2.03 (1H, m), 2.14 (2H, m), 3.20 - 3.30 (3H, m), 3.19 (3H, s), 3.30 - 3.70 (4H, m), 3.80 (2H, m), 4.10 (1H, m), 4.28 (2H, m), 4.30 (3H, s), 7.86 (1H, t, J = 6.6 Hz), 7.98 (1H, d, J = 8.3 Hz), 8.45 (1H, t, J = 7.6 Hz), 8.72 (1H, d, J = 5.6 Hz).

### Example 27

- UVₘₐₓ nm (H₂O):: 296, 270 (sh), 265;
- IRₘₐₓ cm⁻¹ (KBr):: 3430, 1740, 1669, 1578, 1441, 1378, 1267, 1246, 1157;
- NMR δ (D₂O):: 1.21 (3H, d, J = 7.2 Hz), 1.30 (3H, d, J = 6.6 Hz), 1.78 (1H, m), 2.75 (3H, s), 2.90 (1H, m), 3.09 (2H, m), 3.30 - 3.60 (5H, m), 3.68 (1H, dd, J = 6.0 & 12.2 Hz), 3.79 (1H, m), 4.01 (1H, m), 4.21 (3H, s), 4.23 (2H, m), 4.42 (1H, dd, J = 6.0 & 9.6 Hz), 7.87 (1H, d, J = 7.9 Hz), 8.30 (1H, dd, J = 1.3 & 7.9 Hz), 8.68 (1H, d, J = 1.3 Hz).

### Example 28

- UVₘₐₓ nm (H₂O):: 278, 296;
- IRₘₐₓ cm⁻¹ (KBr):: 3430, 1753, 1677, 1592, 1450, 1382, 1278, 1254, 1224, 1156;
- NMR δ (D₂O):: 1.22 (3H, d, J = 7.3 Hz), 1.30 (3H, (D₂O) d, J = 6.6 Hz), 1.81 (1H, m), 2.83 (3H, s), 2.89 (1H, m), 3.17 (2H, m), 3.38 (2H, m), 3.50 (2H, m), 3.72 (2H, m), 4.00 (1H, m), 4.24 (2H, m), 4.28 (3H, s), 4.39 (1H, dd, J = 6.6 & 9.3 Hz), 7.80 (1H, t, J = 7.2 Hz), 8.29 (1H, d, J = 7.6 Hz), 8.63 (1H, d, J = 5.3 Hz).

### Example 29

- UVₘₐₓ nm (H₂O):: 300, 264, 257, 228;
- IRₘₐₓ cm⁻¹ (KBr):: 3400 (br), 1746, 1640, 1582, 1381, 1266;
- NMR δ (D₂O):: 1.21 (3H, d, J = 7.3 Hz), 1.30 (3H, d, J = 6.3 Hz), 1.56 (1H, m), 2.73 (1H, m), 2.97 (1H, dd, J = 4.6 & 12.2 Hz), 3.20 (2H, m), 3.40 (3H, m), 3.75 (1H, m), 4.00 (1H, dd, J = 6.0 & 9.6 Hz), 4.24 (3H, m), 4.34 (3H, s), 7.95 (2H, d, J = 6.6 Hz), 8.69 (2H, d, J = 6.6 Hz).

### Example 30

- UVₘₐₓ nm (H₂O):: 294;
- IRₘₐₓ cm⁻¹ (KBr):: 3400, 1749, 1640, 1588, 1382, 1252;
- NMR δ (D₂O):: 1.22 (3H, d, J = 7.3 Hz), 1.31 (3H, d, J = 6.6 Hz), 1.76 (1H, m), 2.10 (2H, m), 2.81 (1H, m), 3.20 (2H, m), 3.24 (3H, s), 3.43 (1H, m), 3.61 (7H, m), 3.73 (2H, m), 3.92 (3H, m), 4.27 (5H, m).

### Example 31

- UVₘₐₓ nm (H₂O):: 292;
- IRₘₐₓ cm⁻¹ (KBr):: 3430, 1752, 1640, 1592, 1390, 1260;
- NMR δ (D₂O):: 1.24 (3H, d, J = 7.3 Hz), 1.32 (3H, d, J = 6.3 Hz), 1.69 (1H, m), 2.80 (1H, m), 3.12 (1H, dd, J = 3.3 & 12.9 Hz), 3.24 (1H, dd, J = 5.0 & 12.9 Hz), 3.31 (3H, s), 3.43 (3H, s), 3.44 (2H, m), 3.52 - 4.10 (13H, m), 4.29 (3H, m).

### Example 32

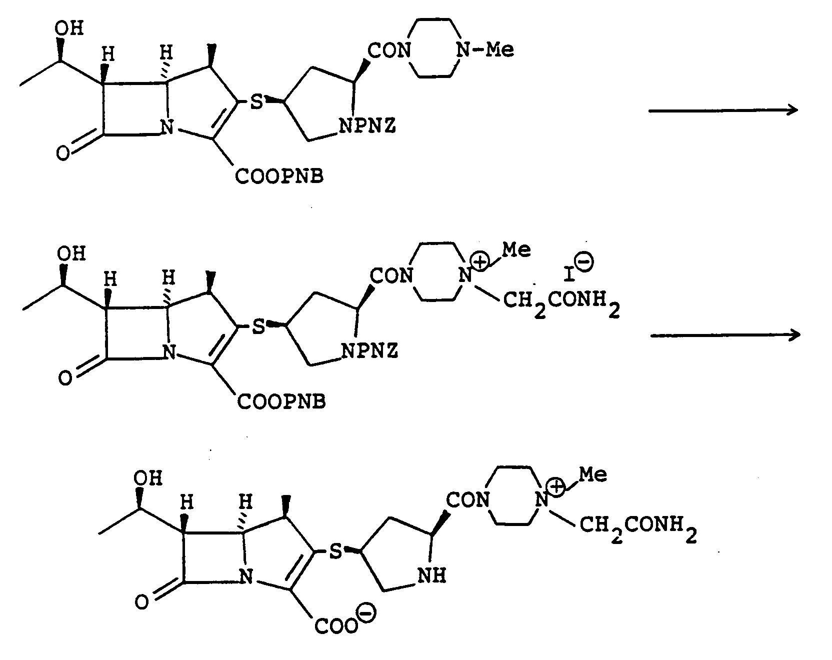

To a solution of (4R,5S,6S,8R,2'S,4'S)-p-nitrobenzyl-3-[1-p-nitrobenzyloxycarbonyl-2-(4-methylpiperazin-1-ylcarbonyl)pyrrolidin-4-ylthio]-4-methyl-6-(1-hydroxyethyl)-1-azabicyclo[3.2.0]hept-2-en-7-one-2-carboxylate (200 mg) in acetone (3.0 ml), iodoacetamide (200 mg) was added at room temperature, and the resultant mixture was stirred at the same temperature for 20 hours and concentrated under reduced pressure. The residue was combined with ethyl acetate (20 ml), stirred and allowed to stand. After removal of the supernatant by decantation, the insoluble material was dissolved in tetrahydrofuran (10 ml) and 0.1M phosphate buffer (pH, 7.0; 10 ml), followed by addition of 10 % palladium-carbon (430 mg). Catalytic reduction was performed at room temperature for 2 hours under ordinary or autogenic pressure. The reaction mixture was subjected to post-treatment in the same manner as in Example 1. The filtrate was purified by polymer chromatography (CHP-20P) using 1 % aqueous tetrahydrofuran as an eluent to give (4R,5S,6S,8R,2'S,4'S)-3-[2-(4-aminocarbonylmethyl -4-methylpiperazinium-1-ylcarbonyl)pyrrolidin-4-ylthio]-4-methyl-6-(1-hydroxyethyl)-1-azabicyclo[3.2.0]hept-2-en-7-one-2-carboxylate.
- UVₘₐₓ nm (H₂O):: 297;
- IRₘₐₓ cm⁻¹ (KBr):: 3400, 1740, 1692, 1652, 1441, 1400, 1253, 1177, 1136;
- NMR δ (D₂O):: 1.23 (3H, d, J = 7.3 Hz), 1.29 (3H, d, J = 6.3 Hz), 2.05 (1H, m), 3.10 (1H, m), 3.45 (3H, s), 3.48 (3H, m), 3.70 - 4.40 (13H, m).

### Examples 33 to 37

In the same manner as in Example 32, the compounds as shown in Table 4 were obtained. The physical properties of the compounds obtained follow the Table.

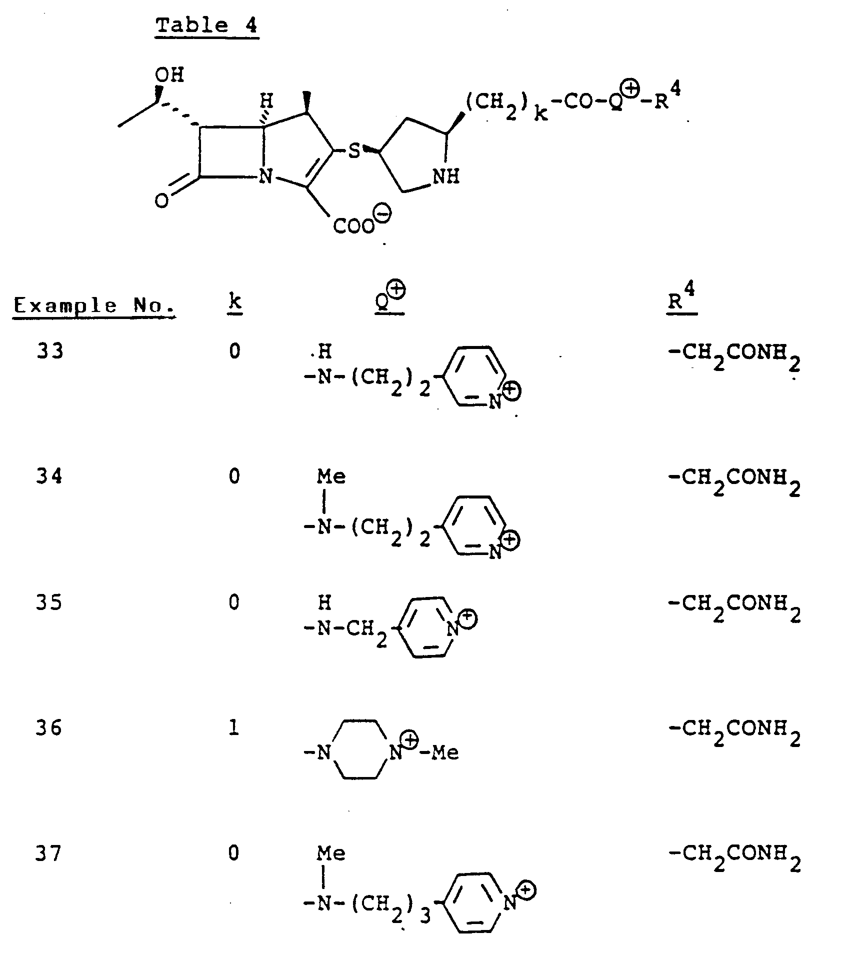

### Physical properties

### Example 33

- UVₘₐₓ nm (H₂O):: 273, 294;
- IRₘₐₓ cm⁻¹ (KBr):: 3380, 1742, 1677, 1580, 1434, 1380, 1275, 1242;
- NMR δ (D₂O):: 1.20 (3H, d, J = 7.3 Hz), 1.29 (3H, d, J = 6.3 Hz), 1.77 (1H, m), 2.80 (1H, m), 3.14 (2H, m), 3.33 (2H, m), 3.45 (1H, dd, J = 2.6 & 6.3 Hz), 3.50 - 3.75 (2H, m), 3.78 (1H, dd, J = 6.3 & 13.5 Hz), 4.20 - 4.40 (3H, m), 5.50 (2H, s), 8.09 (1H, dd, J = 6.3 & 8.3 Hz), 8.58 (1H, d, J = 8.3 Hz), 8.70 (1H, d, J = 6.3 Hz), 8.78 (1H, s).

### Example 34

- UVₘₐₓ nm (H₂O):: 271, 294;
- IRₘₐₓ cm⁻¹ (KBr):: 3390, 1751, 1693, 1638, 1592, 1378;
- NMR δ (D₂O):: 1.20 (3H, d, J = 7.3 Hz), 1.28 (3H, d, J = 6.3 Hz), 1.54 (1H, m), 3.02 (1H, m), 3.03 (3H, s), 3.22 (2H, m), 3.36 (2H, m), 3.46 (1H, dd, J= 2.6 & 5.9 Hz), 3.61 (2H, m), 3.97 (1H, m), 4.12 (1H, m), 4.24 (2H, m), 4.74 (1H, m), 5.50 (2H, s), 8.08 (1H, dd, J = 6.3 & 8.2 Hz),8.60 (1H, d, J = 8.2 Hz), 8.71 (1H, d, J = 6.3 Hz), 8.79 (1H, s).

### Example 35

- UVₘₐₓ nm (H₂O):: 259, 265 (sh), 297;
- IRₘₐₓ cm⁻¹ (KBr):: 3400, 1748, 1682, 1639, 1545, 1388, 1279;
- NMR δ (D₂O):: 1.23 (3H, d, J = 7.3 Hz), 1.29 (3H, d, J = 6.3 Hz), 2.28 (1H, m), 3.05 (1H, m), 3.35 - 3.60 (3H, m), 3.84 (1H, dd, J = 7.3 & 12.2 Hz), 4.10 - 4.45 (3H, m), 5.51 (2H, s), 8.02 (2H, d, J = 7.0 Hz), 8.75 (2H, d, J = 7.0 Hz).

### Example 36

- UVₘₐₓ nm (H₂O):: 297;
- IRₘₐₓ cm⁻¹ (KBr):: 3380, 1747, 1687, 1636, 1586, 1444, 1378, 1244, 1089;
- NMR δ (D₂O):: 1.17 (3H, d, J = 7.3 Hz), 1.24 (3H, d, J = 6.3 Hz), 1.54 (1H, m), 2.63 (1H, m), 2.93 (2H, br.d, J = 6.6 Hz), 3.12 (1H, br.d, J = 12.5 Hz), 3.37 (3H, s), 4.23 (4H, m).

### Example 37

- UVₘₐₓ nm (H₂O):: 299, 263 (sh), 256, 226 (sh);
- IRₘₐₓ cm⁻¹ (KBr):: 3400, 1746, 1691, 1637, 1584, 1387;
- NMR δ (D₂O):: 1.22 (3H, d, J = 7.3 Hz), 1.31 (3H, d, J = 6.3 Hz), 1.62 (1H, m), 2.13 (2H, m), 2.86 (1H, m), 3.03 (2H, m), 3.08 (3H, s), 3.22 (2H, m), 3.45 (3H, m), 3.66 (1H, m), 3.87 (1H, m), 4.28 (3H, m), 5.52 (2H, s), 8.00 (2H, d, J = 6.9 Hz), 8.68 (2H, d, J = 6.9 Hz).

### Examples 38 to 47

In the same manner as in Example 32 but using different alkylating agents (Y) in place of iodoacetamide, the compounds as shown in Table 5 were obtained. The physical properties of the compounds obtained follow the Table.

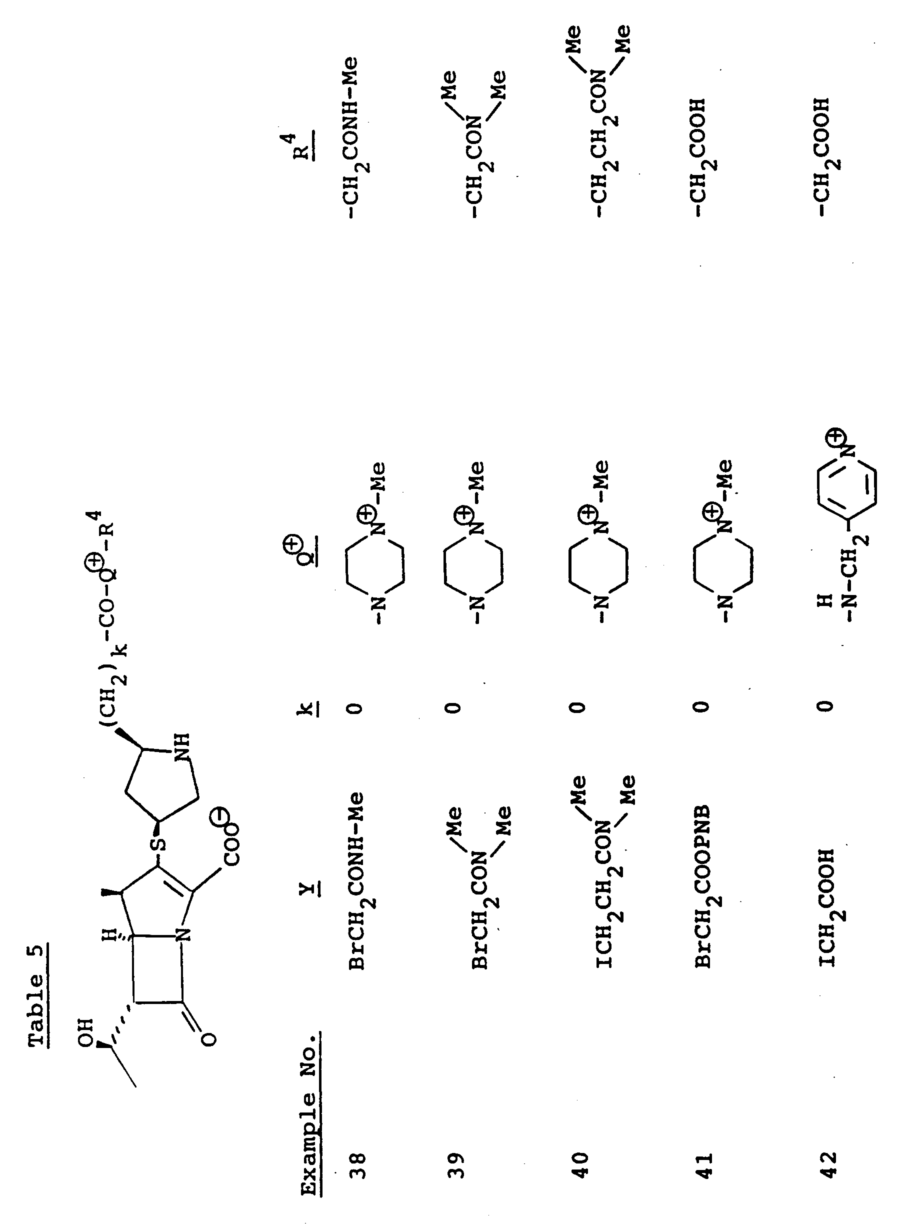

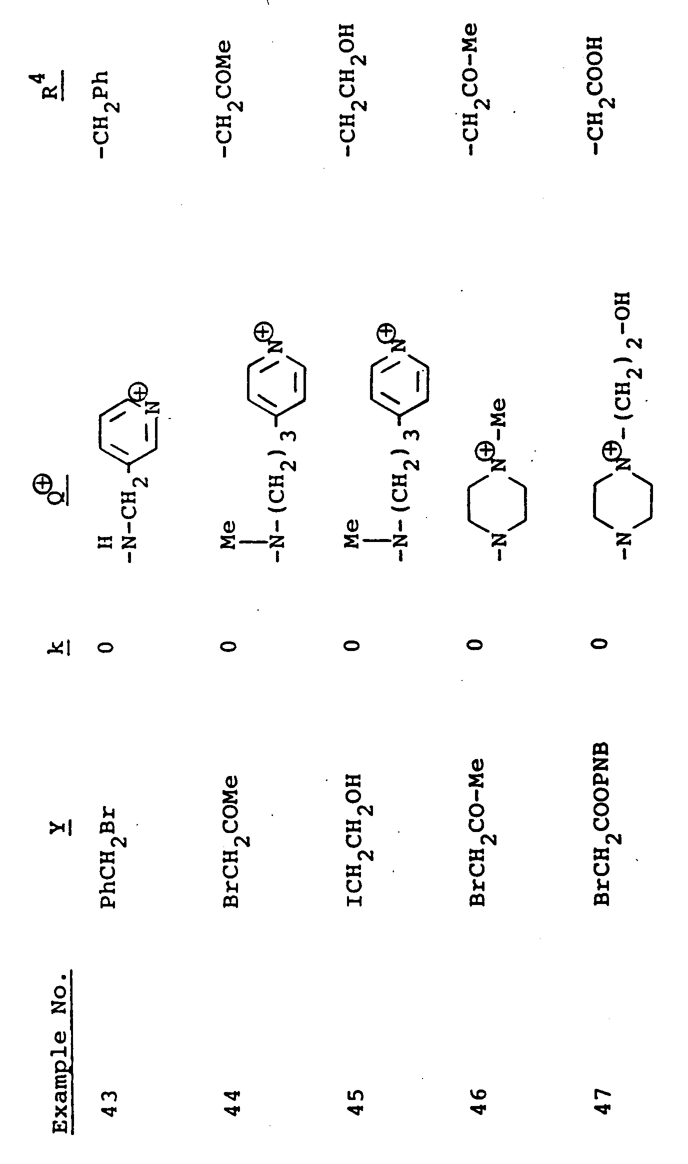

### Physical properties

### Example 38

- UVₘₐₓ nm (H₂O):: 299;
- IRₘₐₓ cm⁻¹ (KBr):: 3410, 1736, 1638, 1362;
- NMR δ (D₂O):: 1.19 (3H, d, J = 7.3 Hz), 1.26 (3H, d, J = 6.3 Hz), 2.77 (3H, s), 3.15 (1H, dd, J = 3.3 & 12.2 Hz), 3.28 (1H, dd, J = 4.4 & 12.2 Hz), 3.38 (3H, s).

### Example 39

- UVₘₐₓ nm (H₂O):: 296;
- IRₘₐₓ cm⁻¹ (KBr):: 3400, 1746, 1644, 1589, 1378, 1253;
- NMR δ (D₂O):: 1.22 (3H, d, J = 7.3 Hz), 1.30 (3H, d, J = 6.3 Hz), 1.71 (1H, m), 2.78 (1H, m), 2.98 (3H, s), 3.05 (3H, s), 3.13 (1H, dd, J = 3.6 & 12.2 Hz), 3.25 (1H, dd, J = 4.0 & 12.2 Hz), 3.46 (3H, s), 4.53 (2H, br. s).

### Example 40

- UVₘₐₓ nm (H₂O):: 297;
- IRₘₐₓ cm⁻¹ (KBr):: 3430, 1745, 1633, 1583, 1480, 1369, 1242, 1087;
- NMR δ (D₂O):: 1.22 (3H, d, J = 7.3 Hz), 1.31 (3H, d, J = 6.6 Hz), 1.93 (1H, m), 2.96 (3H, s), 3.11 (3H, s), 3.26 (3H, s).

### Example 41

- UVₘₐₓ nm (H₂O):: 298;
- IRₘₐₓ cm⁻¹ (KBr):: 3420, 1745, 1627, 1592, 1448, 1382, 1254;
- NMR δ (D₂O):: 1.23 (3H, d, J = 7.3 Hz), 1.30 (3H, d, J = 6.3 Hz), 1.82 (1H, m), 2.92 (1H, m), 3.42 (3H, s).

### Example 42

- UVₘₐₓ nm (H₂O):: 258, 266 (sh), 292;
- NMR δ (D₂O):: 1.22 (3H, d, J = 7.3 Hz), 1.29 (3H, d, J = 6.3 Hz), 2.21 (1H, m), 3.01 (1H, m), 3.38 (1H, m), 3.49 (3H, m) 3.79 (1H, dd, J = 6.6 & 12.2 Hz), 4.10 (1H, m), 4.26 (2H, m), 4.66 (2H, m), 5.21 (2H, s), 7.97 (2H, d, J = 6.9 Hz), 8.71 (2H, d, J = 6.9 Hz).

### Example 43

- UVₘₐₓ nm (H₂O):: 261, 266, 298;
- IRₘₐₓ cm⁻¹ (KBr):: 3400, 1753, 1672, 1596, 1367;
- NMR δ (D₂O):: 1.15 (3H, d, J = 7.3 Hz), 1.30 (3H, d, J = 6.6 Hz), 1.85 (1H, m), 2.72 (1H, m), 3.10 - 4.00 (5H, m), 4.25 (3H, m), 4.59 (1H, d, J = 15.9 Hz), 4.68 (1H, d, J = 15.9 Hz), 5.84 (2H, s), 7.60 (5H, m), 8.07 (1H, t, J = 7.9 Hz), 8.51 (1H, d, J = 8.5 Hz), 8.81 (1H, s), 8.87 (1H, d, J = 5.9 Hz).

### Example 44

- UVₘₐₓ nm (H₂O):: 299, 264 (sh), 257, 230;
- IRₘₐₓ cm⁻¹ (KBr):: 3410 (br), 1745, 1638, 1593, 1378;
- NMR δ (D₂O):: 1.22 (3H, d, J = 7.6 Hz), 1.31 (3H, d, J = 6.3 Hz), 1.66 (1H, m), 2.16 (2H, m), 2.44 (3H, s), 2.90 (1H, m), 3.07 (3H, s), 3.68 (1H, m), 3.87 (1H, m), 4.27 (4H, m), 8.00 (2H, d, J = 6.9 Hz), 8.53 (2H, d, J = 6.9 Hz).

### Example 45

- UVₘₐₓ nm (H₂O):: 296, 261, 255, 223;
- IRₘₐₓ cm⁻¹ (KBr):: 3425, 1751, 1639, 1592, 1304;
- NMR δ (D₂O):: 1.23 (3H, d, J = 7.3 Hz), 1.32 (3H, d, J = 6.3 Hz), 1.62 (1H, m), 2.12 (2H, m), 2.86 (1H, m), 2.97 (2H, m), 3.09 (3H, s), 3.20 (2H, m), 3.44 (4H, m), 3.62 (1H, m), 3.90 (1H, m), 4.08 (2H, m), 4.26 (4H, m), 7.97 (2H, d, J = 6.6 Hz), 8.72 (2H, d, J = 6.6 Hz).

### Example 46

- UVₘₐₓ nm (H₂O):: 296;
- IRₘₐₓ cm⁻¹ (KBr):: 3400(br), 1743, 1724, 1630, 1593, 1380, 1251;
- NMR δ (D₂O):: 1.20 (3H, d, J = 7.3 Hz), 1.28 (3H, d, J = 6.3 Hz), 1.70 (1H, m), 2.26 (3H, s), 2.77 (1H, m), 3.07 (1H, dd, J = 12.5 & 3.6 Hz), 3.19 (1H, dd, J = 12.5 & 6.6 Hz), 3.39 (3H, s), 3.40 (1H, m), 3.60 - 4.10 (11H, m), 4.23 (4H, m).

### Example 47

- UVₘₐₓ nm (H₂O):: 297;
- IRₘₐₓ cm⁻¹ (KBr):: 3400 (br), 1742, 1624, 1590, 1382;
- NMR δ (D₂O):: 1.24 (3H, d, J = 7.3 Hz), 1.31 (3H, d, J = 6.3 Hz), 1.85 (1H, m), 2.92 (1H, m), 3.28 (1H, m), 3.47 (5H, m), 3.80 - 4.37 (15H, m), 4.52 (1H, m).

### Reference Example 1

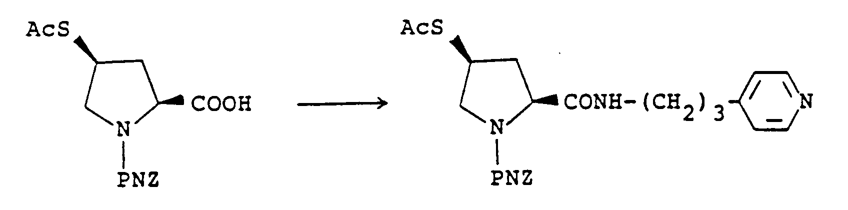

To a solution of cis-1-(p-nitrobenzyloxycarbonyl)-4-acetylthio-L-proline (552 mg; 1.5 mmol) and triethylamine (303 mg; 3.0 mmol) in dry tetrahydrofuran (6 ml), a solution of ethyl chloroformate (184 mg; 1.7 mmol) in dry tetrahydrofuran (1.5 ml) was dropwise added under ice-cooling, followed by stirring for 0.5 hour. To the reaction mixture, 4-(3-aminopropyl)pyridine (306 mg; 2.25 mmol) was added, and the resultant mixture was stirred for 1 hour. The reaction mixture was diluted with ethyl acetate, washed with aqueous sodium hydrogen carbonate solution and aqueous sodium chloride solution in order and dried over anhydrous magnesium sulfate-anhydrous sodium carbonate. After removal of the solvent, the residue was purified by silica gel chromatography to give (2S,4S)-1-(p-nitrobenzyloxycarbonyl)-2-[3-(4-pyridylpropyl)aminocarbonyl]-4-acetylthiopyrrolidine.
- IRₘₐₓ cm⁻¹ (neat):: 3300 (br), 1693, 1602, 1520, 1400, 1340, 1107;
- NMR δ (CDCl₃):: 2.32 (3H, s), 2.4 - 2.8 (4H, m), 3.2 - 3.5 (3H, m), 3.9 - 4.1 (1H, m), 4.1 - 4.2 (1H, m), 4.3 - 4.4 (1H, m), 5.25 (2H, s), 6.66 (1H, br.s), 7.10 (2H, d, J = 5.0 Hz), 7.49 (2H, d, J = 7.6 Hz), 8.20 (2H, d, J = 8.3 Hz), 8.49 (2H, m).

### Reference Examples 2 to 16

In the same manner as in Reference Example 1, the thioacetates as shown in Table 6 were obtained from the corresponding amines. The physical properties of the compounds obtained follow the Table.

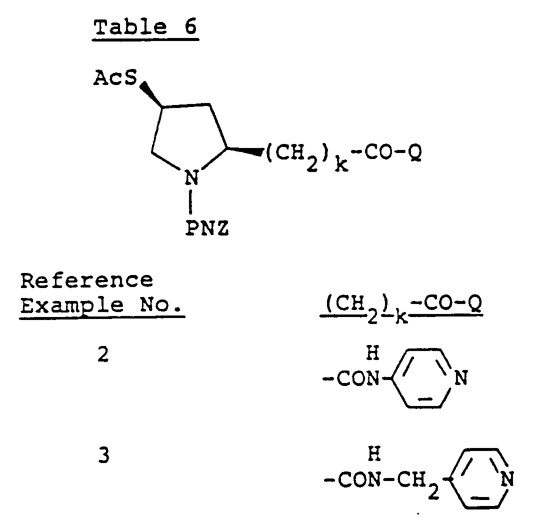

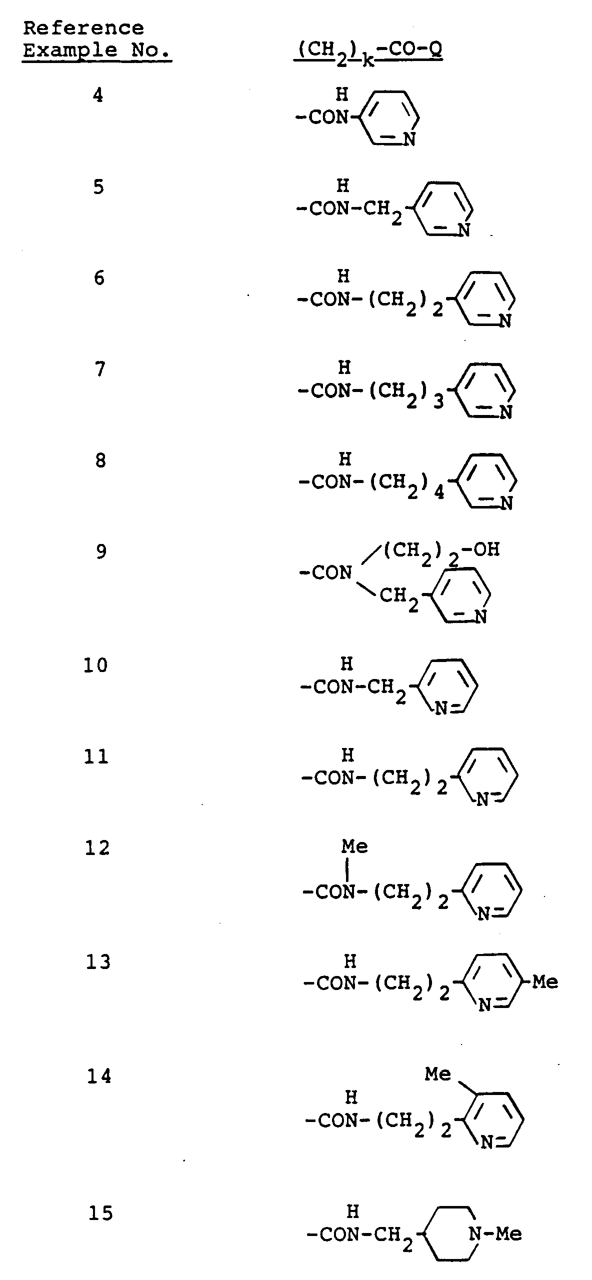

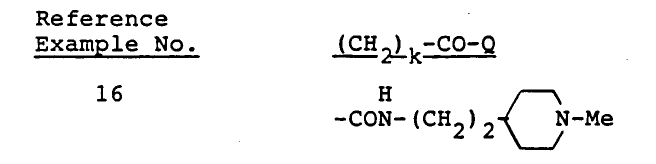

### Physical properties

### Reference Example 2

- IRₘₐₓ cm⁻¹ (neat):: 3310, 1700, 1592, 1523, 1402, 1340, 1290, 1211, 1182, 1162, 1118;
- NMR δ (CDCl₃):: 2.34 (3H, s), 2.54 (2H, m), 3.45 (1H, m), 4.03 (1H, m), 4.16 (1H, dd, J = 10.9 & 7.0 Hz), 4.54 (1H, m), 5.31 (2H, m), 7.39 (2H, d, J = 6.0 Hz), 7.52 (2H, m), 8.22 (2H, m), 8.41 (2H, m), 9.58 (1H, br. s).

### Reference Example 3

- IRₘₐₓ cm⁻¹ (KBr):: 1706, 1695, 1663, 1598, 1513, 1432, 1415, 1403, 1343, 1177, 1122;
- NMR δ (CDCl₃):: 2.32 (3H, s), 2.55 (2H, m), 3.42 (1H, dd, J = 11.6 & 6.0 Hz), 4.01 (1H, quint., J = 6.9 Hz), 4.12 (1H, dd, J = 11.2 & 6.6 Hz), 4.46 (3H, m), 5.23 (2H, s), 7.19 (2H, m), 7.49 (2H, m), 8.21 (2H, d, J = 8.6 Hz), 8.52 (2H, m).

### Reference Example 4

- NMR δ (CDCl₃):: 2.17 (3H, s), 2.5 - 2.9 (2H, m), 3.45 (1H, m), 4.03 (1H, m), 4.16 (1H, dd, J = 11.2 & 6.9 Hz), 4.56 (1H, m), 5.31 (2H, m), 7.22 (1H, m), 7.52 (2H, m), 8.05 (1H, m), 8.22 (2H, m), 8.32 (1H, m), 8.56 (1H, br. s).

### Reference Example 5

- NMR δ (CDCl₃):: 2.32 (3H, s), 2.4 - 2.7 (2H, m), 3.40 (1H, dd, J = 11.2 & 5.9 Hz), 3.99 (1H, m), 4.11 (1H, dd, J = 11.2 & 6.9 Hz), 4.40 (3H, m), 5.21 (2H, br. s), 7.25 (1H, m), 7.48 (2H, m), 7.61 (1H, m), 8.19 (2H, m), 8.51 (2H, m).

### Reference Example 6

- IRₘₐₓ cm⁻¹ (neat):: 3270, 1700 (sh), 1660, 1508, 1395, 1333, 1100;
- NMR δ (CDCl₃):: 2.33 (3H, s), 2.70 - 3.00 (2H, m), 3.20 - 3.70 (3H, m), 3.70 - 4.40 (3H, m), 5.21 (2H, m), 6.83 (1H, br. s), 7.10 - 7.40 (1H, m), 7.4 - 7.7 (3H, m), 8.22 (2H, d, J = 8.3 Hz), 8.46 (2H, m).

### Reference Example 7

- IRₘₐₓ cm⁻¹ (neat):: 3280, 2920, 1700 (sh), 1660 (br), 1510, 1390, 1330, 1100;
- NMR δ (CDCl₃):: 1.75 - 2.0 (2H, m), 2.32 (3H, s), 2.4 - 2.8 (3H, m), 3.15 - 3.6 (3H, m), 3.99 (1H, t, J = 6.6 Hz), 4.05 - 4.25 (1H, m), 4.3 - 4.5 (1H, m), 5.25 (1H, s), 6.66 (1H, br. s), 7.4 - 7.7 (3H, m), 8.20 (2H, d, J = 7.9 Hz), 8.45 (2H, d, J = 4.6 Hz).

### Reference Example 8

- IRₘₐₓ cm⁻¹ (neat):: 3300 (br), 1718, 1707 (sh), 1690, 1520, 1422, 1400, 1342, 1112;
- NMR δ (CDCl₃):: 1.35 - 1.8 (4H, m), 2.31 (3H, s), 2.4 - 2.8 (4H, m), 3.2 - 3.5 (3H, m), 3.90 - 4.05 (1H, m), 4.05 - 4.2 (1H, m), 4.34 (1H, dd, J = 5.6 & 8.3 Hz), 5.23 (2H, s), 6.57 (1H, br. s), 7.1 - 7.3 (1H, m), 7.3 - 7.7 (3H, m), 8.22 (2H, d, J = 8.3 Hz), 8.44 (2H, m).

### Reference Example 9

- IRₘₐₓ cm⁻¹ (CHCl₃):: 3400 (br), 1690, 1685 (sh), 1655, 1521, 1422, 1345, 1200, 1120;
- NMR δ (CDCl₃):: 2.35 (3H, s), 3.2 - 3.6 (3H, m), 3.6 - 4.8 (8H, m), 4.8 - 5.2 (2H, m), 5.24 (2H, s), 7.2 - 7.5 (1H, m), 7.51 (2H, d, J = 8.9 Hz), 7.6 - 7.8 (1H, m), 8.23 (2H, d, J = 8.9 Hz), 8.4 - 8.7 (2H, m).

### Reference Example 10

- IRₘₐₓ cm⁻¹ (KBr):: 3310, 1767, 1700, 1653, 1518, 1435, 1344, 1260, 1240, 1172, 1098;
- NMR δ (CDCl₃):: 2.28 (1H, m), 2.30 (3H, s), 2.75 (1H, m), 3.44 (1H, m), 3.99 (1H, m), 4.21 (1H, m), 4.53 (3H, m), 5.12 (1H, m), 5.24 (2H, br. s), 7.2 - 7.7 (5H, m), 8.0 (1H, m), 8.23 (1H, m), 8.51 (1H, m).

### Reference Example 11

- NMR δ (CDCl₃):: 2.20 (1H, m), 2.28 (3H, s), 2.98 (2H, m), 3.37 (1H, m), 3.64 (2H, m), 4.03 (1H, m), 5.23 (2H, m), 7.3 - 7.7 (4H, m), 8.03 (2H, m), 8.19 (1H, m), 8.42 (1H, m).

### Reference Example 12

- IRₘₐₓ cm⁻¹ (neat):: 3400, 1684, 1653, 1421, 1396, 1337, 1104;
- NMR δ (CDCl₃):: 1.82 (1H, m), 2.3 - 2.4 (3H, m), 2.5 - 3.25 (3H, m), 2.9 - 3.0 (3H, m), 3.45 (2H, m), 3.5 - 4.1 (5H, m), 4.13 (2H, m), 4.73 (1H, m), 5.23 (2H, m), 7.1 - 7.7 (4H, m), 8.20 (2H, m), 8.52 (1H, m).

### Reference Example 13

- IRₘₐₓ cm⁻¹ (neat):: 3300, 1675, 1595, 1510, 1415, 1392, 1333, 1284, 1247, 1198, 1160, 1103;
- NMR δ (CDCl₃):: 2.33 (3H, s), 2.35 (1H, m), 2.51 (3H, s), 2.78 (2H, m), 3.34 (1H, dd, J = 11.2 & 6.3 Hz), 3.50 (2H, m), 3.95 (1H, m), 4.10 (1H, dd, J = 8.6 & 6.9Hz), 4.31 (1H, dd, J = 8.6 & 5.6 Hz), 5.17 (1H, d, J = 13.5 Hz), 5.24 (1H, d, J = 13.5 Hz), 6.72 (1H, br. s), 7.09 (1H, d, J = 7.9 Hz), 7.3 - 7.7 (3H, m), 8.22 (2H, d, J = 8.2 Hz), 8.31 (1H, s).

### Reference Example 14

- IRₘₐₓ cm⁻¹ (KBr):: 3320, 1705, 1656, 1518, 1399, 1340, 1160, 1115;
- NMR δ (CDCl₃):: 1.65 (1H, m), 2.33 (3H, s),2.50 (1H, s), 2.57 (3H, s), 2.83 (2H, m), 3.34 (1H, dd, J = 10.8 & 5.9 Hz), 3.50 (2H, m), 3.97 (1H, m), 4.10 (1H, dd, J = 11.2 & 6.9 Hz), 4.34 (1H, dd, J = 7.9 & 6.3 Hz), 5.20 (2H, m), 7.06 (1H, dd, J = 3.9 & 7.6 Hz), 7.42 (1H, m), 7.50 (2H, m), 8.22 (2H, d, J = 8.6 Hz), 8.37 (1H, d, J = 3.9 Hz).

### Reference Example 15

- IRₘₐₓ cm⁻¹ (KBr):: 3320, 1700, 1665, 1605, 1550, 1518, 1428, 1402, 1342, 1178, 1118;
- NMR δ (CDCl₃):: 1.15 - 1.95 (7H, m), 2.24 (3H,s), 2.33 (3H, s), 2.4-2.7 (1H, m), 2.7 - 3.0 (2H, m), 3.0 - 3.3 (2H, m), 3.3 - 3.5 (1H, m), 3.9 - 4.5 (4H, m), 5.24 (2H, s), 6.65 (1H, br. s), 7.51 (2H, d, J = 8.4 Hz), 8.23 (2H, d, J = 8.4 Hz).

### Reference Example 16

- IRₘₐₓ cm⁻¹ (KBr):: 3290, 1707, 1687, 1645, 1522, 1421, 1340;
- NMR δ (CDCl₃):: 1.1 - 1.6 (5H, m), 1.6 - 2.0 (5H, m), 2.24 (3H, s), 2.33 (3H, s), 2.4 - 2.6 (1H, m), 2.82 (2H, d, J = 11.0 Hz), 3.2 - 3.6 (3H, m), 3.9 - 4.25 (2H, m), 4.3 - 4.5 (1H, m), 5.26 (2H, s), 6.51 (1H, br. s), 7.51 (2H, d, J = 8.3 Hz), 8.24 (2H, d, J = 8.3 Hz).

### Reference Example 17

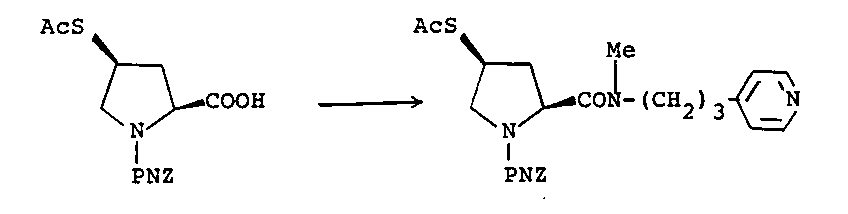

To a solution of cis-1-(p-nitrobenzyloxycarbonyl)-4-acetylthio-L-proline (736 mg; 2.0 mmol) in dry methylene chloride (6 ml), a catalytic amount of dimethylformamide was added, and a solution of oxalic chloride (305 mg; 2.4 mmol) in dry methylene chloride (2 ml) was added thereto. The resultant mixture was stirred at room temperature for 1 hour. Under ice-cooling, methyl [3-(4-pyridyl)propyl]amine (300 mg; 2.0 mmol) and a solution of triethylamine (485 mg; 4.8 mmol) in dry methylene chloride (2. ml) were added thereto, followed by stirring for 15 minutes. The reaction mixture was combined with aqueous sodium hydrogen carbonate solution, and the organic phase was separated from the aqueous phase, washed with aqueous sodium chloride solution and dried over anhydrous magnesium sulfate. After removal of the solvent, the residue was purified by silica gel column chromatography to give (2S,4s)-1-(p-nitrobenzyloxycarbonyl)-2-[3-(4-pyridyl)propyl]methylaminocarbonyl-4-acetylthiopyrrolidine.
- IRₘₐₓ cm⁻¹ (neat):: 1715 (sh), 1700, 1654, 1600, 1518, 1340, 1160, 1107;
- NMR δ (CDCl₃):: 1.7 - 2.2 (3H, m), 2.33 (3H, s), 2.4 - 2.9 (3H, m) , 2.9 - 3.1 (3H, m), 3.3 - 3.7 (3H, m), 3.9 - 4.2 (2H, m), 4.5 - 4.8 (1H, m), 5.21 (2H, s), 6.9 - 7.2 (2H, m), 7.3 - 7.6 (2H, m), 8.1 - 8.3 (2H, m), 8.4 - 8.6 (2H, m).

### Reference Examples 18 to 26

In the same manner as in Reference Example 17, the thioacetates as shown in Table 7 were obtained from the corresponding amines. The physical properties of the compounds obtained follow the Table.

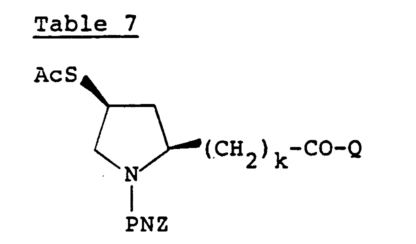

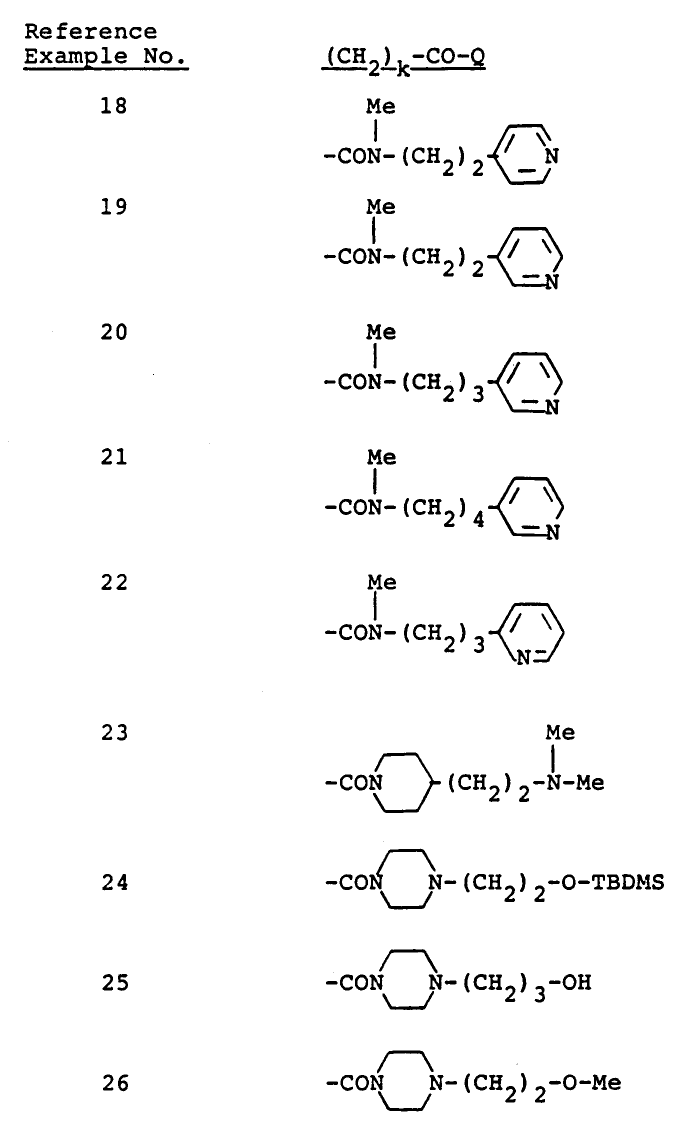

### Physical properties

### Reference Example 18

- IRₘₐₓ cm⁻¹ (neat):: 1715 (sh), 1700, 1687 (sh), 1602, 1520, 1340, 1162, 1107;
- NMR δ (CDCl₃):: 1.7 - 2.0 (1H, m), 2.34 (3H, s), 2.5 - 3.1 (6H, m), 3.3 - 3.85 (3H, m), 3.85 - 4.2 (2H, m), 4.5 - 4.8 (1H, m), 5.22 (2H, s), 7.0 - 7.25 (2H, m), 7.4 - 7.6 (2H, m), 8.1 - 8.3 (2H, m), 8.45 - 8.65 (2H, m).

### Reference Example 19

- IRₘₐₓ cm⁻¹ (neat):: 1730 (sh), 1692 (sh), 1660, 1507, 1390, 1335, 1150, 1110;
- NMR δ (CDCl₃):: 1.6 - 1.9 (1H, m), 2.34 (3H, s), 2.5 - 3.1 (6H, m), 3.3 - 4.3 (5H, m), 4.5 - 4.8 (1H, m), 5.22 (2H, s), 7.2 - 7.4 (1H, m), 7.4 - 7.7 (3H, m), 8.22 (2H, d, J = 8.9 Hz), 8.47 (2H, br. s).

### Reference Example 20

- IRₘₐₓ cm⁻¹ (neat):: 2930, 1715 (sh), 1704, 1696 (sh), 1650, 1518, 1420, 1400, 1340, 1105;
- NMR δ (CDCl₃):: 1.7 - 2.2 (3H, m), 2.33 (3H, s), 2.4 - 2.85 (3H, m), 2.85 - 3.15 (3H, m), 3.3 - 3.6 (2H, m), 3.85 - 4.2 (2H, m), 4.45 - 4.8 (1H, m), 5.22 (2H, s), 7.1 - 7.3 (1H, m), 7.3 - 7.6 (3H, m), 8.05 - 8.3 (2H, m), 8.3 - 8.6 (2H, m).

### Reference Example 21

- IRₘₐₓ cm⁻¹ (neat):: 2925, 1714 (sh), 1682, 1654, 1518, 1420, 1400, 1340, 1160, 1115;
- NMR δ (CDCl₃):: 1.8 - 2.15 (2H, m), 2.33 (3H, s), 2.4 - 2.8 (3H, m), 2.8 - 3.1 (3H, m), 3.2 - 3.6 (3H, m), 3.9 - 4.2 (3H, m), 4.69 (1H, m), 5.20 (2H, m), 7.1 - 7.6 (4H, m), 8.1 - 8.3 (2H, m), 8.3 - 8.6 (2H, m).

### Reference Example 22

- IRₘₐₓ cm⁻¹ (neat):: 1720 (sh), 1705, 1650, 1515, 1430, 1400, 1340, 1110;
- NMR δ (CDCl₃):: 1.7 - 2.3 (2H, m), 2.33 (3H, s), 2.5 - 3.2 (7H, m), 3.2 - 3.7 (3H, m), 3.8 - 4.3 (2H, m), 5.6 - 5.8 (1H, m), 5.21 (2H, s), 7.0 - 7.3 (2H, m), 7.4 - 7.7 (3H, m), 8.0 - 8.3 (2H, m), 8.5 - 8.7 (1H, m).

### Reference Example 23

- IRₘₐₓ cm⁻¹ (neat):: 2920, 1700 (sh), 1688, 1642, 1507, 1400, 1336, 1100;
- NMR δ (CDCl₃):: 0.8 - 2.0 (10H, m), 2.21 (6H, s), 2.33 (3H, s), 2.5 - 3.2 (3H, m), 3.3 - 5.0 (5H, m), 5.22 (2H, s), 7.51 (2H, d, J = 8.5 Hz), 8.22 (2H, d, J = 8.5 Hz).

### Reference Example 24

- IRₘₐₓ cm⁻¹ (neat):: 1700, 1660, 1523, 1438, 1402, 1342, 1253, 1103;
- NMR δ (CDCl₃):: 0.05 (6H, s), 0.89 (9H, s), 1.88 (1H, m), 2.33 (3H, s), 2.50 (6H, m), 3.40 (2H, m), 3.55 (2H, m), 3.72 (1H, m), 3.75 (2H, m), 4.00 (1H, m), 4.13 (2H, m), 4.72 (1H, m), 5.05 - 5.40 (2H, m), 7.50 (2H, m), 8.23 (2H, m).

### Reference Example 25

- IRₘₐₓ cm⁻¹ (neat):: 3450 (br), 1700, 1653, 1521, 1435, 1342, 1120;
- NMR δ (CDCl₃):: 1.80 - 2.00 (1H, m), 2.2 - 2.9 (8H, m), 2.34 (3H, s), 3.3 - 3.9 (8H, m), 3.9 - 4.2 (2H, m), 4.6 - 4.8 (1H, m), 5.0 - 5.4 (2H, m), 7.51 (2H, d, J = 8.9 Hz), 8.22 (2H, d, J = 8.9 Hz).

### Reference Example 26

- NMR δ (CDCl₃):: 1.90 (1H, m), 2.34 (3H, s), 2.30 - 2.85 (7H, m), 3.34 (3H x 0.3, s), 3.35 (3H x 0.7, s), 3.40 - 3.78 (7H, m), 4.01 (1H, m), 4.14 (1H, m), 4.75 (1H, m), 5.07 (0.3H, d, J = 13.9 Hz), 5.23 (2H x 0.7, s), 5.31 (0.3H, d, J = 13.9 Hz), 7.50 (2H,m), 8.23 (2H, m).

### Reference Example 27

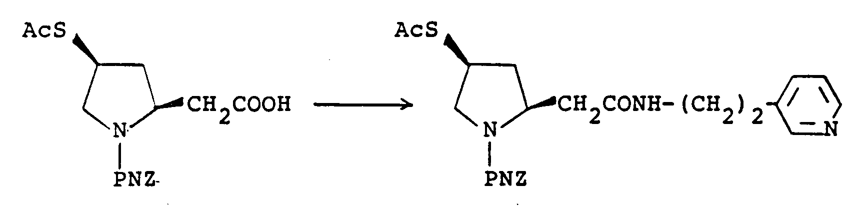

In the same manner as in Reference Example 1, there was obtained (2R,4S)-1-(p-nitrobenzyloxycarbonyl)-2-[2-(3-pyridylethyl)aminocarbonyl]methyl-4-acetylthiopyrrolidine from (2R,4S)-1-(p-nitrobenzyloxycarbonyl)-2-carboxymethyl-4-acetylthiopyrrolidine (382 mg; 1.0 mmol).
- IRₘₐₓ cm⁻¹ (neat):: 3295 (br), 1690 (sh), 1680, 1650 (sh), 1513, 1418, 1395, 1338, 1100;
- NMR δ (CDCl₃):: 2.2 - 2.7 (2H, m), 2.34 (3H, s), 2.7 - 3.0 (3H, m), 3.25 (1H, dd, J = 7.3 & 11.2 Hz), 3.4 - 3.7 (2H, m), 3.8 - 4.3 (3H, m), 5.19 (2H, s), 5.98 (1H, br.s), 7.15 - 7.35 (1H, m), 7.35 - 7.65 (3H, m), 8.22 (2H, d, J = 8.6 Hz), 8.4 - 8.6 (2H, m).

### Reference Examples 28 and 29

In the same manner as in Reference Example 27, the thioacetates as shown in Table 8 were obtained from the corresponding amines. The physical properties of the compounds obtained follow the Table.

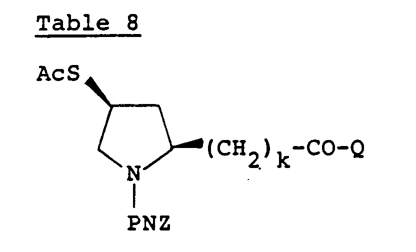

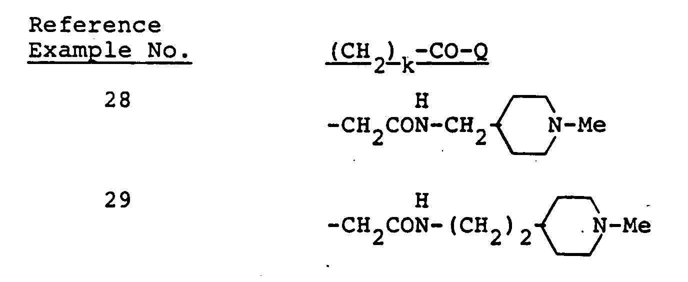

### Physical properties

### Reference Example 28

- IRₘₐₓ cm⁻¹ (KBr):: 3310, 1700, 1645, 1527, 1445, 1430, 1405, 1347, 1320, 1200, 1147, 1110;
- NMR δ (CDCl₃):: 1.15 - 1.55 (4H, m), 1.55 - 2.0 (5H, m), 2.25 (3H, s), 2.34 (3H, s), 2.4 - 2.7 (1H, m), 2.7 - 3.0 (3H, m), 3.0 - 3.6 (3H, m), 3.8 - 4.5 (3H, m), 5.21 (2H, s), 5.86 (1H, br. s), 7.52 (2H, d, J = 8.8 Hz), 8.23 (2H, d, J = 8.8 Hz).

### Reference Example 29

- IRₘₐₓ cm⁻¹ (KBr):: 3290, 1690 (sh), 1687, 1630, 1520, 1424, 1342;
- NMR δ (CDCl₃):: 1.1 - 1.5 (5H, m), 1.5 - 1.8 (3H, m), 1.87 (2H, t, J = 10.7 Hz), 2.25 (3H, s), 2.34 (3H, s), 2.4 - 2.7 (2H, m), 2.7 - 3.0 (3H, m), 3.1 - 3.4 (3H, m), 3.8 - 4.3 (3H, m), 5.21 (2H, s), 5.71 (1H, br. s), 7.51 (2H, d, J = 8.6 Hz), 8.23 (2H, d, J = 8.6 Hz).

### Reference Example 30

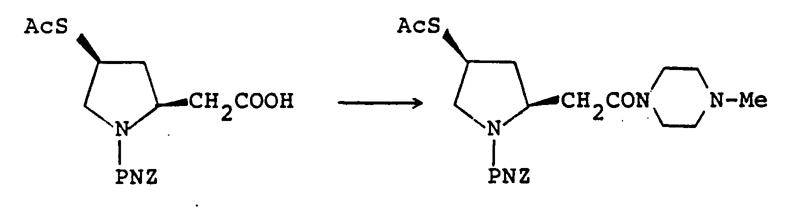

In the same manner as in Reference Example 2, there was obtained (2R,4S)-1-(p-nitrobenzyloxycarbonyl)-2-[(4-methyl)piperazin-1-yl]carbonylmethyl-4-acetylthiopyrro lidine from (2R,4S)-1-(p-nitrobenzyloxycarbonyl)-2-carboxymethyl-4-acetylthiopyrrolidine (382 mg; 1.0 mmol).
- IRₘₐₓ cm⁻¹ (neat):: 1687, 1634, 1515, 1420, 1398, 1340, 1285, 1100;
- NMR δ (CDCl₃):: 1.8 - 2.0 (1H, m), 2.2 - 2.6 (6H, m), 2.29 (3H, s), 2.34 (3H, s), 2.6 - 2.9 (1H, m), 3.2 - 3.8 (5H, m), 3.8 - 4.0 (1H, m), 4.0 - 4.5 (2H, m), 5.21 (2H, s), 7.51 (2H, d, J = 8.6 Hz), 8.23 (2H, d, J = 8.6 Hz).

### Reference Example 31

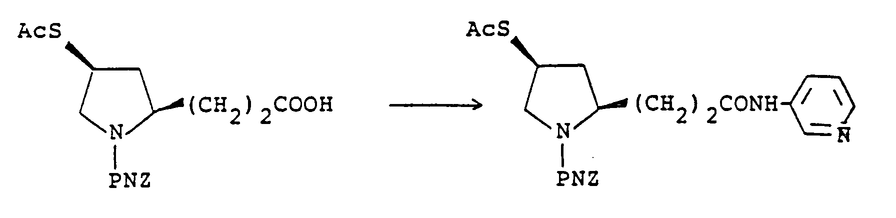

In the same manner as in Reference Example 2, there was obtained (2R,4S)-1-(p-nitrobenzyloxycarbonyl)-2-(3-pyridylamino)carbonylethyl-4-acetylthiopyrrolidine from (2R,4S)-1-(p-nitrobenzyloxycarbonyl)-2-(2-carboxy)ethyl-4-acetylthiopyrrolidine (198 mg; 0.50 mmol).
- IRₘₐₓ cm⁻¹ (neat):: 3280 (br), 1700 (sh), 1680, 1516, 1400, 1338;
- NMR δ (CDCl₃):: 1.6 - 2.8 (6H, m), 2.35 (3H, s), 3.28 (1H, dd, J = 6.8 & 11.7 Hz), 3.92 (1H, m), 4.0 - 4.3 (2H, m), 5.26 (2H, s), 7.2 - 7.4 (2H, m), 7.53 (2H, d, J = 8.7 Hz), 8.25 (2H, d, J = 8.7 Hz), 8.3 - 8.45 (1H, m), 8.67 (1H, d, J = 2.3 Hz), 9.23 (1H, br. s).

### Reference Example 32

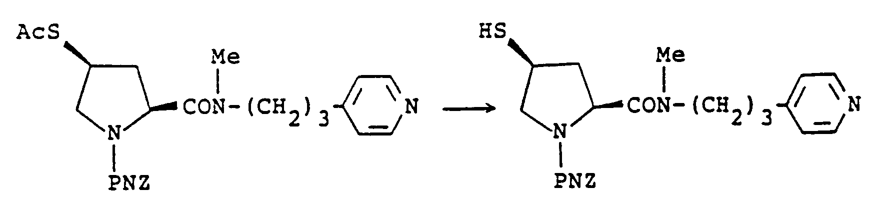

To a solution of (2S,4S)-1-(p-nitrobenzyloxycarbonyl)-2-((3-(4-pyridyl)propyl)methylaminocarbonyl)-4-acetylthiopyrrolidine (332 mg) in methanol (30 ml), 1N aqueous sodium hydroxide solution (0.70 ml) was added at room temperature, and the resultant mixture was stirred for 10 minutes. 1N Hydrochloric acid (0.70 ml) was added to the reaction mixture, and methanol was removed by distillation under reduced pressure. The residue was combined with dichloromethane, washed with water and dried over anhydrous magnesium sulfate, followed by removal of the solvent to give (2S,4S)-1-p-nitrobenzyloxycarbonyl-2-(3-(4-pyridyl)propyl)methylaminocarbonyl-4-mercaptopyrrolidine, which was subjected to the subsequent reaction without purification.

In the same manner as in Reference Example 32, the mercaptan compounds as shown in Table 9 were obtained from the corresponding thioacetates.

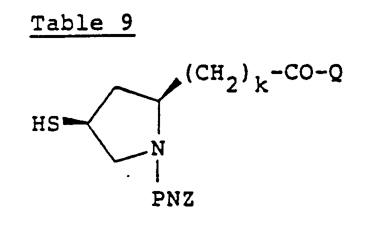

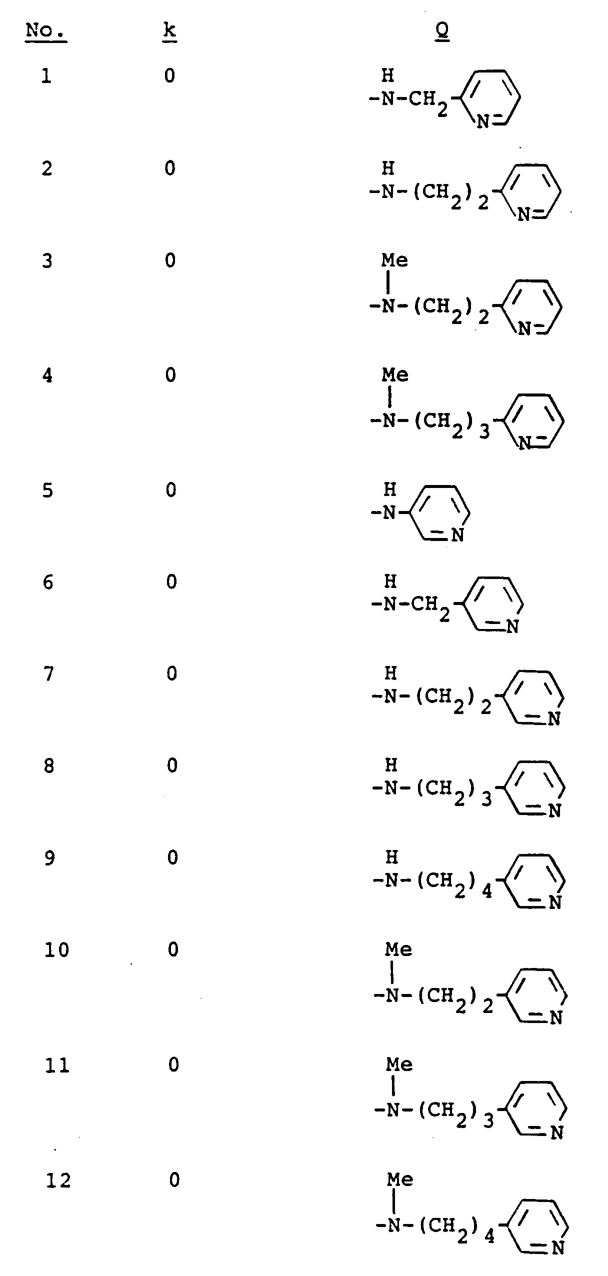

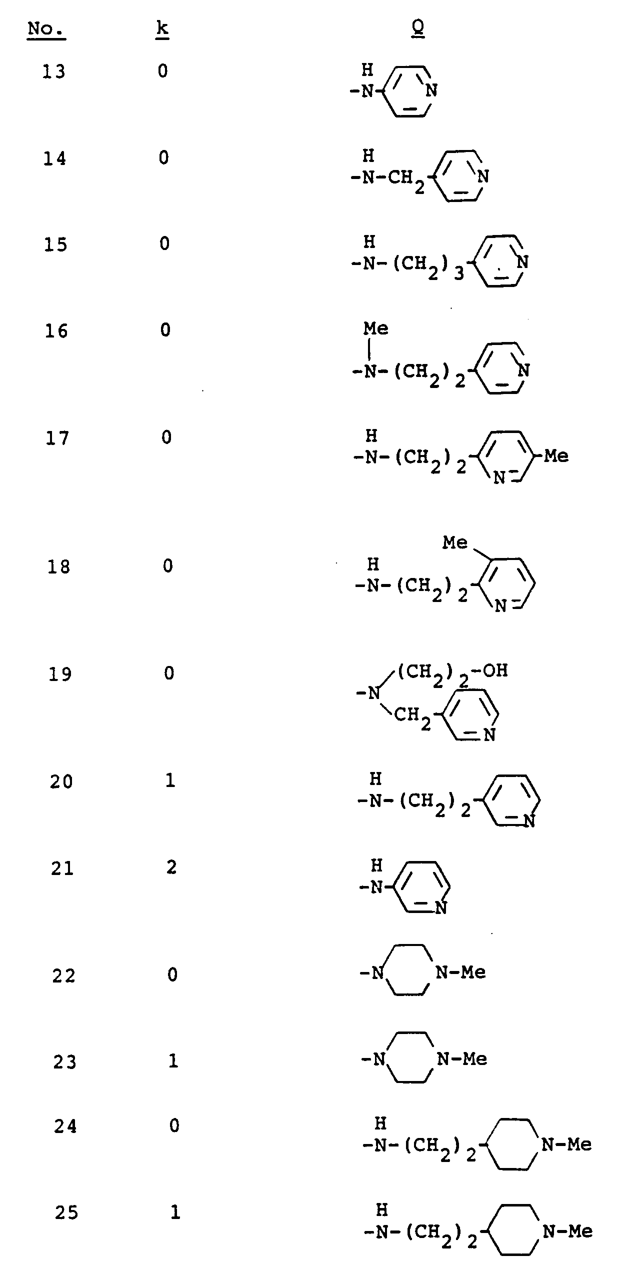

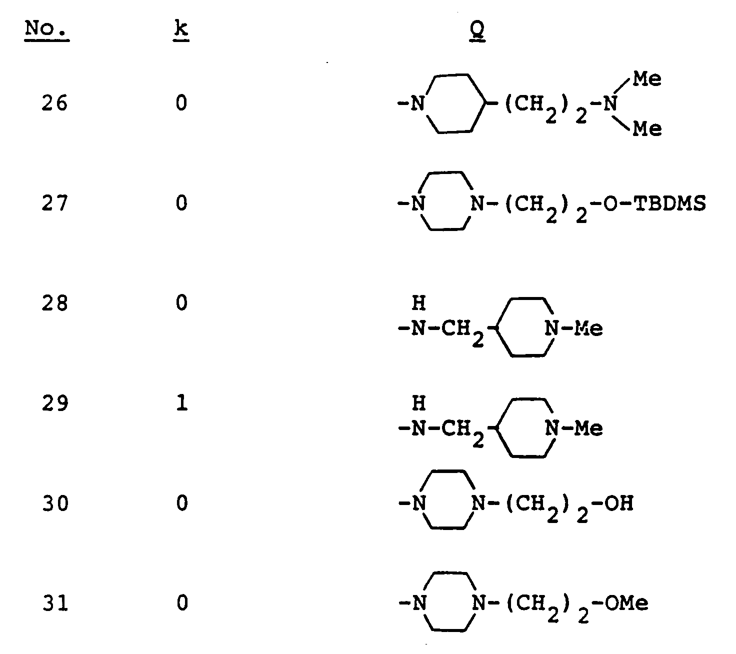

### Reference Example 33

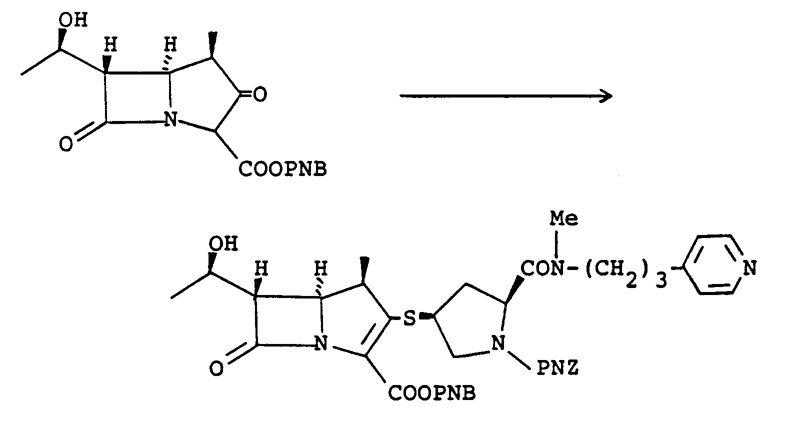

To a solution of (4R,5R,6S,8R)-p-nitrobenzyl-4-methyl-6-(1-hydroxyethyl)-1-azabicyclo[3.2.0]hept-3,7-dione-2-carboxylate (218 mg) in dry acetonitrile (2.0 ml), diisopropylethylamine (94 mg) and diphenyl chlorophosphate (178 mg) were added under ice-cooling, and the resultant mixture was stirred at the same temperature for 2 hours. A solution of (2S,4S)-p-nitrobenzyloxycarbonyl-2-(3-(4-pyridyl)propyl)methylaminocarbonyl-4-mercaptopyrrolidine (311 mg) and diisopropylethylamine (94 mg) in dry acetonitrile (3.0 ml) was added to the reaction mixture, followed by stirring for 2 hours. The reaction mixture was diluted with ethyl acetate, washed with aqueous potassium phosphate solution and a saturated aqueous sodium chloride solution in order and dried over anhydrous magnesium sulfate. After removal of the solvent, the residue was purified by silica gel column chromatography to give (4R,5S,6S,8R,2'S,4'S)-p-nitrobenzyl-3-[1-p-nitrobenzyloxycarbonyl-2-((3-(4-pyridyl)propyl)methylaminocarbonyl)pyrrolidin-4-ylthio]-4-methyl-6-(1-hydroxyethyl)-1-azabicyclo[3.2.0]hept-2-en-7-one-2-carboxylate.
- IRₘₐₓ cm⁻¹ (neat):: 3380, 1763, 1700, 1644, 1601, 1517, 1403, 1339;
- NMR δ (CDCl₃):: 1.28 (3H, d, J = 6.9 Hz), 1.37 (3H, d, J = 6.3 Hz), 3.08, 2.96 (3H as a whole, each s), 5.21 (2H, br.s), 5.24 (1H, d, J = 13.8 Hz), 5.51 (1H, d, J = 13.8 Hz), 6.97 - 7.20 (2H, m), 7.35 - 7.63 (2H, m), 7.65 (2H, d, J = 8.9 Hz), 8.10 - 8.30 (4H, m), 8.52 (2H, m).

### Reference Example 34

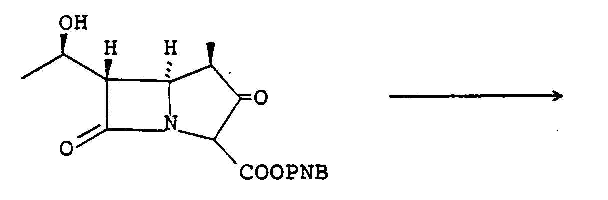

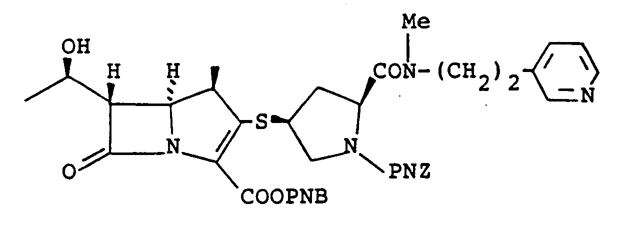

To a solution of (4R,5R,6S,8R)-p-nitrobenzyl-4-methyl-6-(1-hydroxyethyl)-1-azabicyclo[3.2.0]hept-3,7-dione-2-carboxylate (54 mg) in dry acetonitrile (1.0 ml), diisopropylethylamine (22 mg) and diphenyl chlorophosphate (45 mg) were added under ice-cooling, and the resultant mixture was stirred at the same temperature for 1 hour. A solution of (2S,4S)-1-p-nitrobenzyloxycarbonyl-2-(2-(3-pyridyl)ethyl)methylaminocarbonyl-4-mercaptopyrrolidine (95 mg) and diisopropylethylamine (22 mg) in dry acetonitrile (1.0 ml) was added to the reaction mixture, followed by stirring for 1.5 hours. The reaction mixture was diluted with dichloromethane, washed with water and dried over anhydrous magnesium sulfate. After removal of the solvent, the residue was purified by silica gel column chromatography to give (4R,5S,6S,8R,2'S,4'S)-p-nitrobenzyl-3-[1-p-nitrobenzyloxycarbonyl-2-((2-(3-pyridyl)ethyl)methylaminocarbonyl)pyrrolidin-4-ylthio]-4-methyl-6-(1-hydroxyethyl)-1-azabicyclo[3.2.0]hept-2-en-7-one-2-carboxylate.
- IRₘₐₓ cm⁻¹ (neat):: 3400, 1755, 1690, 1512, 1332;
- NMR δ (CDCl₃):: 1.27 (3H, d, J = 7.0 Hz), 1.37 (3H, d, J = 6.3 Hz), 2.88, 2.96, 3.00 (3H as a whole, each s), 3.27 (1H, m), 5.30 (3H, m), 5.50 (1H, d, J = 13.5 Hz), 7.26 (1H, m), 7.4 - 7.6 (3H, m), 7.65 (2H, d, J = 8.6 Hz), 8.22 (4H, d, J = 8.6 Hz), 8.4 - 8.6 (2H, m).

### Reference Example 35

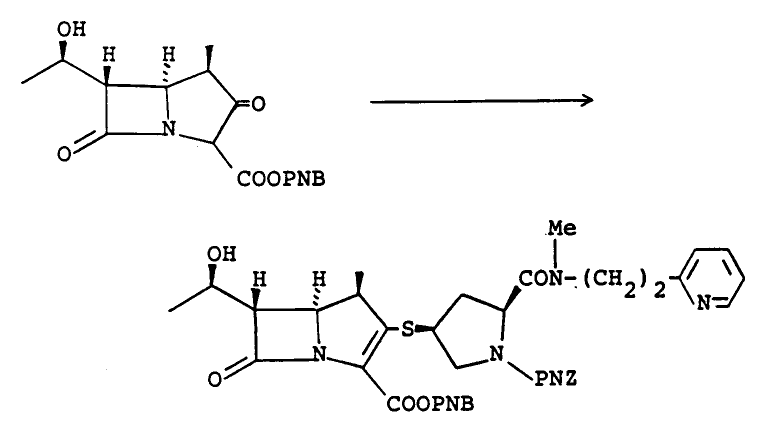

To a solution of (4R,5R,6S,8R)-p-nitrobenzyl-4-methyl-6-(1-hydroxyethyl)-1-azabicyclo[3.2.0]hept-3,7-dione-2-carboxylate (181 mg) in dry acetonitrile (2.0 ml), diisopropylethylamine (81 mg) and diphenyl chlorophosphate (175 mg) were added under ice-cooling, and the resultant mixture was stirred at the same temperature for 1 hour. A solution of (2S,4S)-p-nitrobenzyloxycarbonyl-2-(2-(2-pyridyl)ethyl)methylaminocarbonyl-4-mercaptopyrrolidine (303 mg) in dry acetonitrile (3.0 ml) and then diisopropylethylamine (81 mg) were added to the reaction mixture, followed by stirring for 2 hours. The reaction mixture was diluted with ethyl acetate, washed with aqueous potassium phosphate solution and a saturated aqueous sodium chloride solution in order and dried over magnesium sulfate. After removal of the solvent, the residue was purified by silica gel column chromatography to give (4R,5S,6S,8R,2'S,4'S)-p-nitrobenzyl-3-[1-p-nitrobenzyloxycarbonyl-2-((2-(2-pyridyl)ethyl)methyl aminocarbonyl)pyrrolidin-4-ylthio]-4-methyl-6-(1-hydroxyethyl)-1-azabicyclo[3.2.0]hept-2-en-7-one-2-carboxylate.
- NMR δ (CDCl₃):: 1.28 (3H, d, J = 7.3 Hz), 1.34 (3H, d, J = 6.3 Hz), 1.87 (1H, m), 2.73 (1H, m), 2.92, 2.93, 2.95, 3.01 (3H as a whole, each s), 4.80 (1H, m), 5.26 (3H, m), 5.49 (1H, d, J = 13.9 Hz), 7.00 - 7.75 (7H, m), 8.22 (4H, m), 8.50 (1H, m).

### Reference Example 36

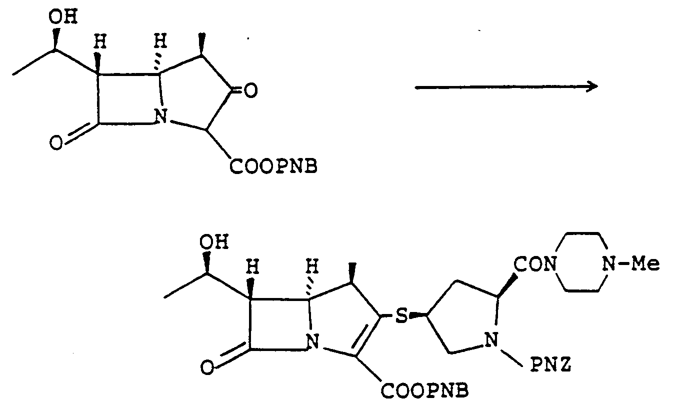

To a solution of (4R,5R,6S,8R)-p-nitrobenzyl-4-methyl-6-(1-hydroxyethyl)-1-azabicyclo[3.2.0]hept-3,7-dione-2-carboxylate (2.55 g) in dry acetonitrile (10.0 ml), diisopropylethylamine (1.09 g) and diphenyl chlorophosphate (2.06 g) were added under ice-cooling, and the resultant mixture was stirred at the same temperature for 2 hours. A solution of (2S,4S)-p-nitrobenzyloxycarbonyl-2-(4-methylpiperazin-1-ylcarbonyl)-4-mercaptopyrrolidine (3.08 g) and diisopropylethylamine (1.09 g) in dry acetonitrile (10.0 ml) was added to the reaction mixture, followed by stirring for 4 hours. The reaction mixture was diluted with ethyl acetate, washed with aqueous potassium phosphate solution and a saturated aqueous sodium chloride solution in order and dried over anhydrous magnesium sulfate. After removal of the solvent, the residue was purified by silica gel column chromatography to give (4R,5S,6S,8R,2'S,4'S)-p-nitrobenzyl-3-[1-p-nitrobenzyloxycarbonyl-2-(4-methylpiperazin-1-ylcarbonyl)pyrrolidin-4-ylthio]-4-methyl-6-(1-hydroxyethyl)-1-azabicyclo[3.2.0]hept-2-en-7-one-2-carboxylate.
- IRₘₐₓ cm⁻¹ (neat):: 3400, 1750, 1695, 1630, 1593, 1500, 1423, 1390, 1324, 1271, 1193, 1120;
- NMR δ (CDCl₃):: 1.26 (3H, d, J = 7.3 Hz), 1.34 (3H, d, J = 6.3 Hz), 1.91 (1H, m), 2.32 (3H, s), 2.73 (1H, s), 4.72 (1H, m), 5.22 (3H, m), 5.43 (1H, d, J = 13.9 Hz), 7.40 - 7.60 (2H, m), 7.64 (2H, d, J = 8.9 Hz), 8.20 (4H, d, J = 8.9 Hz).

### Reference Examples 37 to 50

In the same manner as in Reference Example 36, the compounds as shown in Table 10 were obtained. The physical properties of the compounds obtained follow the Table.

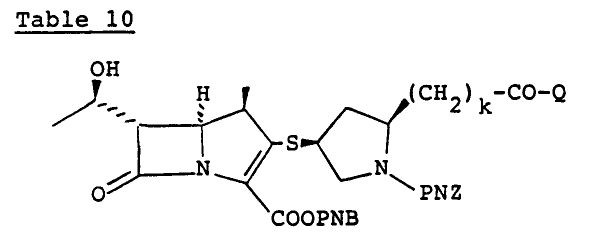

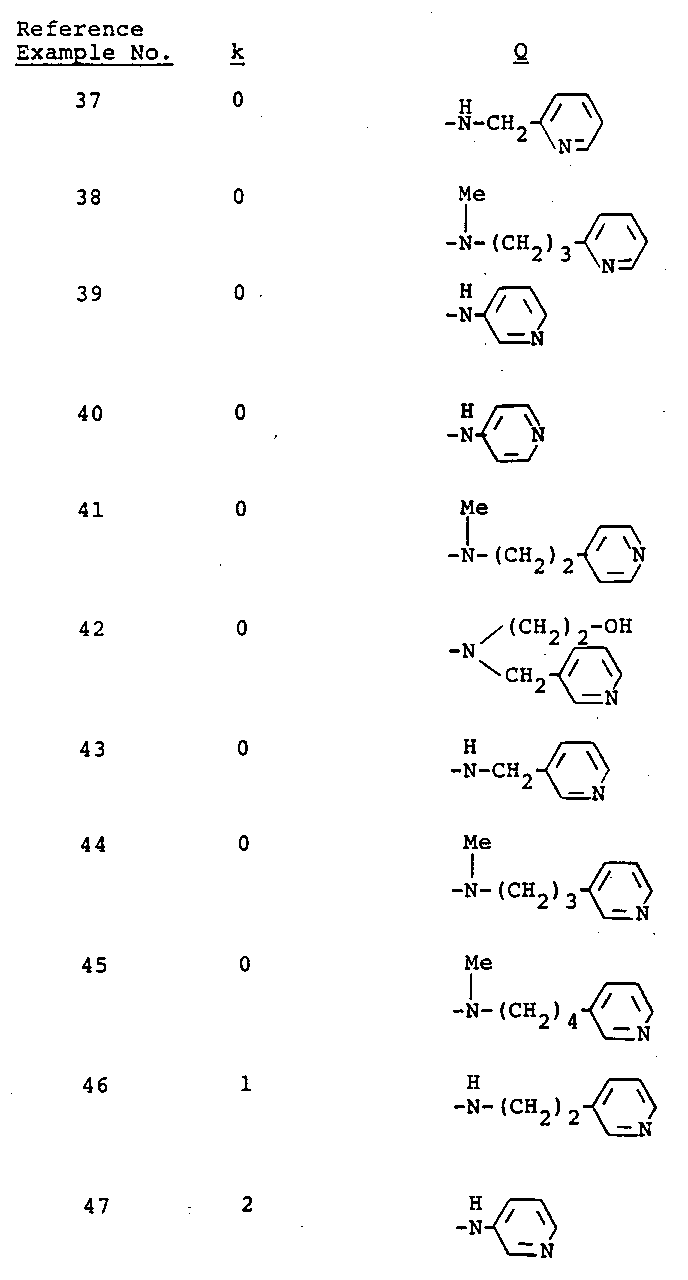

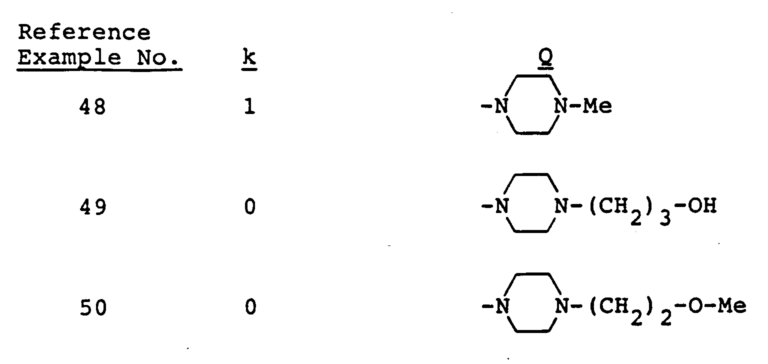

### Physical properties

### Reference Example 37

- IRₘₐₓ cm⁻¹ (neat):: 3370, 1763, 1700, 1602, 1517, 1430, 1398, 1341, 1203, 1130, 1106;
- NMR δ (CDCl₃):: 1.27 (3H, d, J = 7.3 Hz), 1.36 (3H, d, J = 6.3 Hz), 2.80 (1H, m), 3.28 (1H, dd, J = 3.0 & 6.9 Hz), 3.36 (1H, m), 3.50 (1H, dd, J = 8.0 & 10.9 Hz), 3.71 (1H, m), 4.30 (2H, m), 5.10 - 5.50 (4H, m), 7.10 - 7.70 (6H, m), 7.98 (1H, m), 8.21 (4H, d, J = 8.9 Hz), 8.39 (1H, m).

### Reference Example 38

- IRₘₐₓ cm⁻¹ (neat):: 3400, 1761, 1697, 1637, 1515, 1426, 1400, 1340, 1202, 1175, 1132, 1104;
- NMR δ (CDCl₃):: 1.28 (3H, d, J = 6.9 Hz), 1.34 (3H, d, J = 5.6 Hz), 1.88 (3H, m), 2.50 - 2.90 (4H, m), 2.92, 2.97, 3.00, 3.11 (3H as a whole, each s), 3.28 (1H, m), 3.48 (4H, m), 3.69 (1H, m), 3.87 (1H, m), 4.27 (3H, m), 4.75 (1H, m), 5.23 (3H, m), 5.48 (1H, d, J= 13.9 Hz), 7.39 (1H, dd, J = 4.9 & 8.9 Hz), 7.50 (1H, d, J = 8.9 Hz), 8.06 (1H, t, J = 8.9 Hz), 8.18 (4H, d, J = 8.9 Hz), 8.48 (1H, m).

### Reference Example 39

- IRₘₐₓ cm⁻¹ (neat):: 3330, 1761, 1710, 1603, 1519, 1420, 1340, 1205;
- NMR δ (CDCl₃):: 1.26 (3H, d, J = 7.3 Hz), 1.35 (3H, d, J = 6.3 Hz), 3.35 (2H, m), 3.84 (1H, m), 4.03 (1H, m), 4.28 (2H, m), 4.56 (1H, m), 5.17 (1H, d, J = 13.6 Hz), 5.30 (2H, s), 5.33 (1H, d, J = 13.6 Hz), 7.60 (2H, d, J = 8.9 Hz), 8.16 (4H, d, J = 8.9 Hz), 8.35 (1H, m), 8.58 (1H, s).

### Reference Example 40

- IRₘₐₓ cm⁻¹ (neat):: 3290, 1760, 1702, 1586, 1508, 1397, 1337, 1203,1183;
- NMR δ (CDCl₃):: 1.27 (3H, d, J = 7.2 Hz), 1.35 (3H, d, J = 6.3 Hz), 2.30 (1H, m), 2.64 (1H, m), 3.32 (2H, m), 3.53 (1H, m), 3.83 (1H, m), 4.00 (1H, m), 4.28 (2H, m), 4.55 (1H, m), 5.18 (1H, d, J = 13.8 Hz), 5.25 (2H, m), 5.38 (1H, d, J = 13.8 Hz), 7.47 (2H, m), 7.60 (2H, d, J = 8.6 Hz), 8.17 (2H, d, J = 8.6 Hz), 8.45 (2H, m).

### Reference Example 41

- IRₘₐₓ cm⁻¹ (neat):: 3400, 1762, 1698, 1643, 1600, 1517, 1339;
- NMR δ (CDCl₃):: 1.27 (3H, d, J = 6.9 Hz), 1.37 (3H, d, J = 6.3 Hz), 2.89, 2.96, 2.98 (3H as a whole, each s), 5.22 (2H, br. s), 5.25 (1H, d, J = 13.9 Hz), 5.49 (1H, d, J = 13.9 Hz), 7.00 - 7.23 (2H, m), 7.40 - 7.58 (2H, m), 7.65 (2H, d, J = 8.6 Hz), 8.21 (4H, m), 8.53 (2H, m).

### Reference Example 42

- IRₘₐₓ cm⁻¹ (neat):: 3380, 1755, 1693, 1643, 1508, 1337;
- NMR δ (CDCl₃):: 1.27 (3H, d, J = 7.3 Hz), 1.36 (3H, d, J = 6.3 Hz), 2.03 (1H, m), 2.67 (1H, m), 3.20 - 3.95 (9H, m), 4.05 (1H, m), 4.26 (2H, m), 4.96 (1H, d, J = 13.5 Hz), 5.23 (4H, m), 5.48 (1H, d, J = 13.5 Hz), 7.26 (1H, m), 7.51 (2H, d, J = 8.9 Hz), 7.65 (3H, d, J = 8.6 Hz), 8.22 (4H, m), 8.53 (2H, m).

### Reference Example 43

- IRₘₐₓ cm⁻¹ (KBr):: 3350, 1774, 1704, 1656, 1600, 1508, 1423, 1395, 1337, 1315;
- NMR δ (CDCl₃):: 1.24 (3H, m), 1.33 (3H, d, J = 6.3 Hz), 2.47 (1H, m), 2.91 (1H, m), 3.31 (2H, m), 3.54 (1H, dd, J = 5.3 & 11.2 Hz), 3.79 (1H, m), 4.02 (1H, dd, J = 6.0 & 11.2 Hz), 4.20 - 4.60 (5H, m), 5.12 (1H, d, J = 14.2 Hz), 5.20 (2H, br. s), 5.40 (1H, d, J = 14.2 Hz), 7.22 (1H, m), 7.50 (2H, m), 7.60 (2H, d, J = 8.9 Hz), 7.62(1H, m), 8.13 (4H, d, J = 8.9 Hz), 8.45 (1H, m), 8.50(1H, s).

### Reference Example 44

- IRₘₐₓ cm⁻¹ (KBr):: 3410, 1768, 1704, 1653, 1603, 1522, 1422, 1403, 1342, 1262;
- NMR δ (CDCl₃):: 1.28 (3H, m), 1.37 (3H,d, J = 6.3 Hz), 1.87 (2H, m), 2.70 (3H, m), 2.97, 2.98, 3.09 (3H as a whole, each s), 3.30 - 3.80 (7H, m), 4.78 (2H, m), 5.24 (1H, d, J = 13.8 Hz), 5.30 (2H, br. s), 5.46 (2H, d, J = 13.8 Hz), 7.22 (1H, m), 7.40 (1H, m), 7.50 (2H, d, J = 8.6 Hz), 7.65 (2H, d, J = 8.9 Hz), 8.2 (4H, m), 8.45 (2H, m).

### Reference Example 45

- IRₘₐₓ cm⁻¹ (KBr):: 3300, 1773, 1707, 1663, 1604, 1518, 1438, 1402, 1340, 1280, 1265, 1206, 1168, 1147, 1109;
- NMR δ (CDCl₃):: 1.27 (3H, m), 1.37 (3H, d, J = 6.3 Hz), 1.80 - 2.10 (4H, m), 2.67 (2H, m), 2.88, 2.93, 2.95, 3.04 (3H as a whole, each s), 5.20 (2H, br.s), 5.25 (1H, d, J = 13.5 Hz), 5.48 (1H, d, J = 13.5 Hz), 7.23 (1H, m), 7.40 (1H, m), 7.51 (2H, d, J = 8.9 Hz), 7.65 (2H, d, J = 8.3 Hz), 8.12 (4H, d, J = 8.9 Hz), 8.42 (2H, m).

### Reference Example 46

- IRₘₐₓ cm⁻¹ (neat):: 3350, 1760, 1696, 1652, 1517, 1419, 1400, 1338, 1196, 1130, 1102;
- NMR δ (CDCl₃):: 1.28 (3H, d, J = 7.3 Hz), 1.37 (3H, d, J = 6.0 Hz), 2.82 (2H, m), 5.20 (2H, br. s), 5.23 (1H, d, J = 14.0 Hz), 5.50 (1H, d, J = 14.0 Hz), 7.25 (1H, m), 7.55 (3H, m), 7.66 (2H, d, J = 8.9 Hz), 8.23 (4H, d, J = 8.9 Hz), 8.44 (1H, br.s), 8.48 (1H, d, J = 5.0 Hz).

### Reference Example 47

- IRₘₐₓ cm⁻¹ (neat):: 3350, 1760, 1700, 1684, 1598, 1518, 1400, 1336;
- NMR δ (CDCl₃):: 1.27 (3H, d, J = 6.9 Hz), 1.37 (3H, d, J = 6.3 Hz), 2.20 - 2.80 (5H, m), 3.20 - 3.50 (2H, m), 3.28 (1H, dd, J = 2.6 & 6.9 Hz), 3.50 - 3.80 (1H, m), 4.00 - 4.35 (5H, m), 5.37 (2H, ABq, J = 76.9 & 13.9 Hz), 7.53 (2H, d, J = 8.9 Hz), 7.65 (2H, d, J = 8.9 Hz), 8.10 - 8.50 (7H, m), 8.64 (1H, s), 9.09 (1H, br.s).

### Reference Example 48

- IRₘₐₓ cm⁻¹ (neat):: 3370, 1760, 1695, 1627, 1517, 1433, 1420, 1398, 1337, 1194, 1132, 1101;
- NMR δ (CDCl₃):: 1.28 (3H, d, J = 7.3 Hz), 1.37 (3H, d, J = 6.3 Hz), 2.30 (3H, s), 5.25 (3H,m), 5.50 (1H, d, J = 13.9 Hz), 7.51 (2H, d, J = 8.9 Hz), 7.65 (2H, d, J = 8.9Hz), 8.22 (4H, m).

### Reference Example 49

- IRₘₐₓ cm⁻¹ (neat):: 3200 (br), 1760, 1700, 1652, 1512, 1336;
- NMR δ (CDCl₃):: 1.28 (3H, m), 1.36 (3H, d, J = 6.3 Hz), 2.02 (3H, m), 2.73 (1H, m), 3.10 (2H, m), 3.24 - 4.80 (18H, m), 5.05 - 5.56 (4H, m), 7.43 (2H x 0.3, d, J = 7.9 Hz), 7.51 (2H x 0.7, d, J = 8.9 Hz), 7.64 (2H, d, J = 8.6 Hz), 8.20 (4H, m).

### Reference Example 50

- IRₘₐₓ cm⁻¹ (neat):: 3420 (br), 1763, 1700, 1648, 1602, 1520, 1438, 1243;
- NMR δ (CDCl₃):: 1.26 (3H, m), 1.35 (3H, d, J = 6.3 Hz), 2.92 (1H, m), 2.30 - 2.85 (7H, m), 3.34 (3H x 0.5, s), 3.56 (3H x 0.5, s), 3.20 - 3.83 (9H, m), 4.20 (2H, m), 4.76 (1H, m), 5.08 - 5.55 (4H, m), 7.44 (2H x 0.5, d, J = 8.9 Hz), 7.52 (2H x 0.5, d, J = 8.6 Hz), 7.65 (2H, d, J = 8.9 Hz), 8.20 (4H, m).

### Reference Example 51

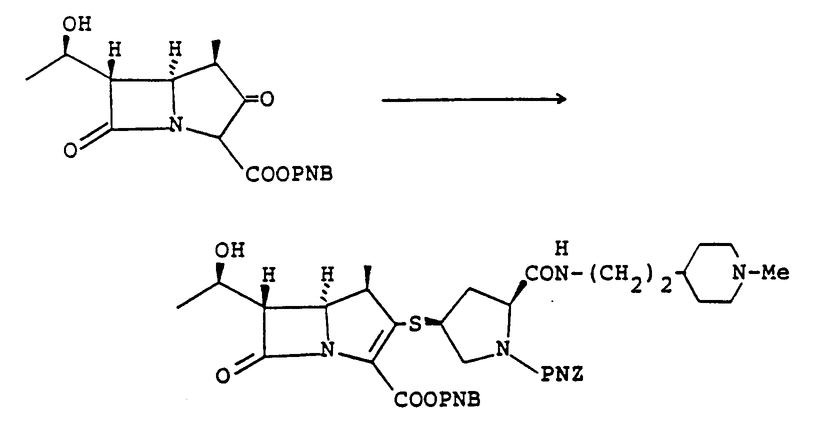

To a solution of (4R,5R,6S,8R)-p-nitrobenzyl-4-methyl-6-(1-hydroxyethyl)-1-azabicyclo[3.2.0]hept-3,7-dione-2-carboxylate (217 mg) in dry acetonitrile (2.0 ml), diisopropylethylamine (93 mg) and diphenyl chlorophosphate (178 mg) were added under ice-cooling, and the resultant mixture was stirred for 3 hours. A solution of (2S,4S)-p-nitrobenzyloxycarbonyl-2-(2-(1-methylpiperidin-4-yl)ethyl)aminocarbonyl-4-mercaptopyrrolidine (293 mg) and 1,8-diazabicyclo[5.4.0]-7-undecene (218 mg) in a mixture of dry acetonitrile (2.0 ml) and dry tetrahydrofuran (4.0 ml) was added to the reaction mixture, followed by stirring for 1 hour. The reaction mixture was combined with a phosphate buffer (pH, 7.0) and extracted with dichloromethane 3 times. The organic layer was dried over anhydrous magnesium sulfate. After removal of the solvent, the residue was purified by silica gel column chromatography to give (4R,5S,6S,8R,2'S,4'S)-p-nitrobenzyl-3-[1-p-nitrobenzyloxycarbonyl-2-((2-(1-methylpiperidin-4-yl)ethyl)aminocarbonyl) pyrrolidin-4-ylthio]-4-methyl-6-(1-hydroxyethyl)-1-azabicyclo[3.2.0]hept-2-en-7-one-2-carboxylate.
- IRₘₐₓ cm⁻¹ (neat):: 3300, 1762, 1703, 1519, 1487, 1342, 1204;
- NMR δ (CDCl₃):: 1.24 (3H, d, J = 7.3 Hz), 1.36 (3H, d, J = 6.3 Hz), 2.35 (3H, br.s).

### Reference Example 52

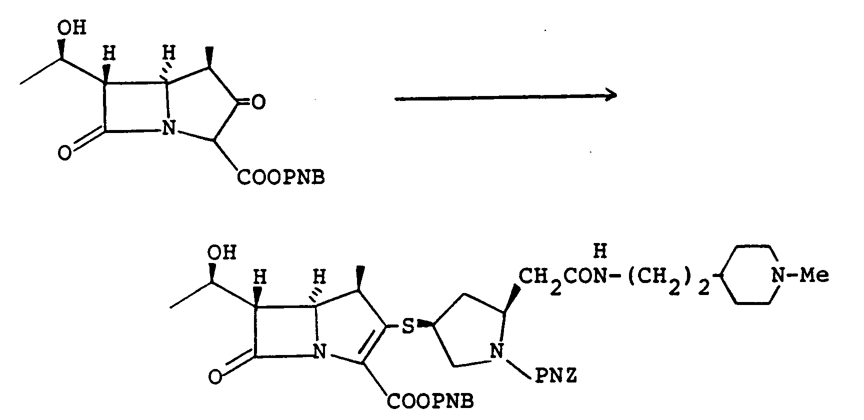

In the same manner as in Reference Example 51, there was obtained (4R,5S,6S,8R,2'S,4'S)-p-nitrobenzyl-[1-p-nitrobenzyloxycarbonyl-2-((2-(1-methylpiperidin-4-yl)ethyl)aminocarbonylmethyl)pyrrolidin-4-ylthio]-4-methyl-6-(1-hydroxyethyl)-1-azabicyclo[3.2.0]hept-2-en-7-one-2-carboxylate.
- IRₘₐₓ cm⁻¹ (neat):: 3350, 1758, 1693, 1518, 1339;
- NMR δ (CDCl₃):: 1.25 (3H, d, J = 7.0 Hz), 1.36 (3H, d, J = 6.3 Hz), 2.35 (3H, br.s).

### Reference Example 53

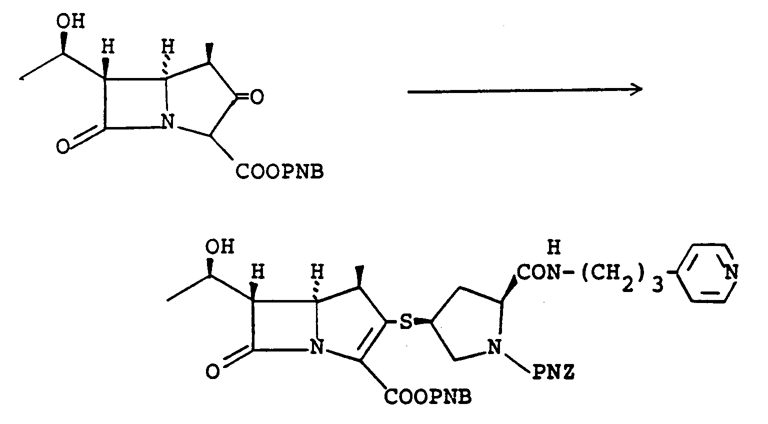

To a solution of (4R,5R,6S,8R)-p-nitrobenzyl-4-methyl-6-(1-hydroxyethyl)-1-azabicyclo[3.2.0]hept-3,7-dione-2-carboxylate (256 mg) in dry acetonitrile (1.5 ml), diisopropylethylamine (108 mg) and diphenyl chlorophosphate (206 mg) were added under ice-cooling, and the resultant mixture was stirred at the same temperature for 4 hours. To a suspension of (2S,4S)-1-p-nitrobenzyloxycarbonyl-2-(3-(4-pyridyl)propyl)aminocarbonyl-4-mercaptopyrrolidine (450 mg) in dry acetonitrile (3.0 ml), bis(trimethylsilyl)acetamide (165 mg) was added, and the mixture was heated to 60°C, followed by allowing to stand. The thus obtained solution was added to the above phosphate solution under cooling with ice, and diisopropylethylamine (108 mg) was added thereto. After 15 minutes, 1,8-diazabicyclo[5.4.0]-7-undecene (203 mg) was added, followed by stirring for 1 hour. The reaction mixture was diluted with ethyl acetate, washed with aqueous potassium phosphate solution and a saturated aqueous sodium chloride solution in order and dried over anhydrous magnesium sulfate. After removal of the solvent, the residue was dissolved in ethyl acetate (50 ml), 0.1 N hydrochloric acid (5.0 ml) was added while cooling with ice, and the resultant mixture was stirred vigorously. A phosphate buffer (pH, 7.0) was added to the reaction mixture, which was extracted with dichloromethane three times. The extracts were combined together, dried over anhydrous magnesium sulfate, concentrated and purified by silica gel column chromatography to give (4R,5S,6S,8R,2'S,4'S)-p-nitrobenzyl-3-(1-p-nitrobenzyloxycarbonyl-2-((3-(4-pyridyl)propyl)aminocarbonyl)pyrrolidin-4-ylthio]-4-methyl-6-(1-hydroxyethyl)-1-azabicyclo[3.2.0]hept-2-en-7-one-2-carboxylate.
- IRₘₐₓ cm⁻¹ (neat):: 3350, 1760, 1697, 1518, 1340;
- NMR δ (CDCl₃):: 1.26 (3H, d, J = 7.0 Hz), 1.36 (3H, d, J = 6.3 Hz), 1.84 (2H, m), 2.60 (2H, m), 7.09 (2H, m), 7.49 (2H, m), 7.62 (2H, m), 8.20 (4H, m), 8.48 (2H, d, J = 5.9 Hz).

### Reference Examples 54 to 60

In the same manner as in Reference Example 53, the compounds as shown in Table 11 were obtained. The physical properties of the compounds obtained follow the Table.

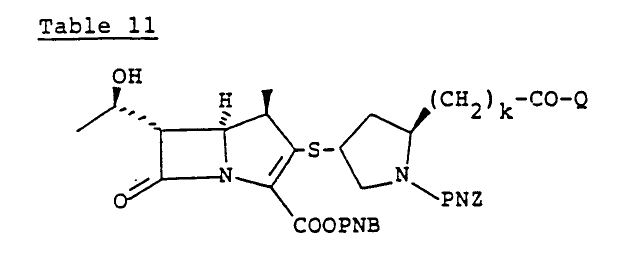

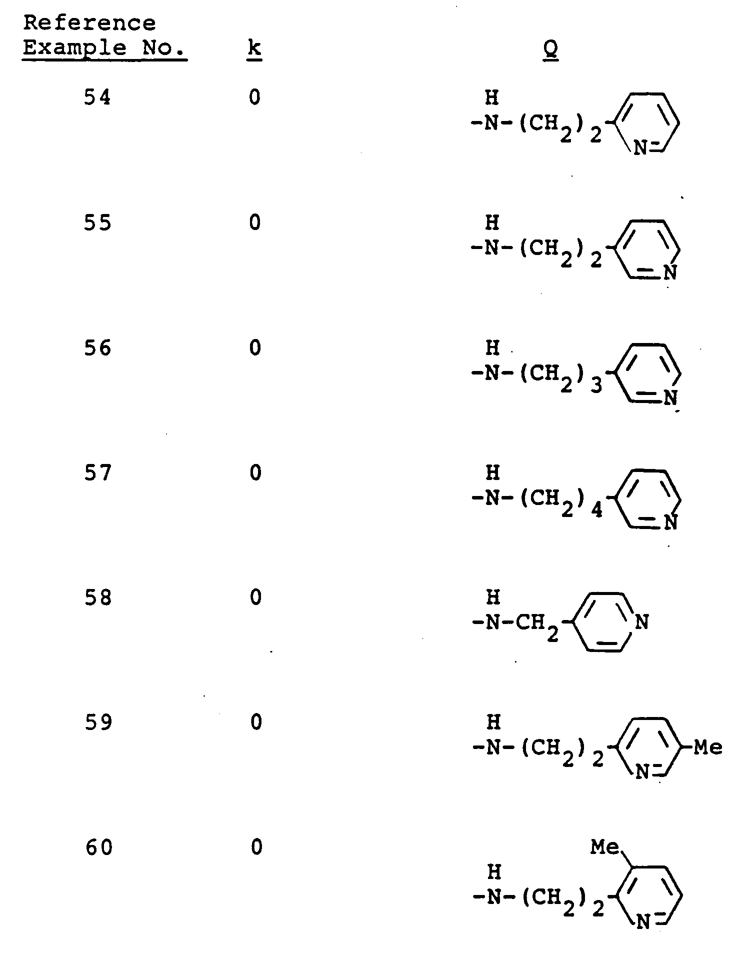

### Physical properties

### Reference Example 54

- NMR δ (CDCl₃):: 1.26 (3H, d, J = 7.3 Hz), 1.36 (3H, d, J = 6.3 Hz), 5.11 (1H, d, J = 13.5 Hz), 5.18 (2H, m), 5.42 (1H, d, J = 13.5 Hz), 7.00 - 7.80 (6H, m), 8.05 (1H, m), 8.19 (4H, d, J = 8.9 Hz), 8.39 (1H, m).

### Reference Example 55

- IRₘₐₓ cm⁻¹ (neat):: 3400, 1742, 1680, 1500, 1309, 1251;
- NMR δ (CDCl₃):: 1.27 (3H, d, J = 6.9 Hz), 1.36 (3H, d, J = 6.3 Hz), 1.95 (1H, m), 2.51 (1H, m), 2.84 (2H, m), 3.32 (2H, m), 3.49 (3H, m), 3.73 (1H, m), 3.97 (1H, m), 5.20 (3H, m), 5.42 (1H, d, J = 13.5 Hz), 7.23 (1H, m), 7.51 (3H, m), 7.62 (2H, d, J = 8.6 Hz), 8.18 (4H, d, J = 8.6 Hz), 8.43 (2H, m).

### Reference Example 56

- IRₘₐₓ cm⁻¹ (neat):: 3225, 1770, 1703, 1655, 1518, 1422, 1399, 1342, 1318, 1273, 1203, 1166, 1137, 1105;
- NMR δ (CDCl₃):: 1.24 (3H, d, J = 7.3 Hz), 1.35 (3H, d, J = 6.3 Hz), 1.82 (3H, m), 2.61 (2H, m), 3.36 (4H, m), 3.50 (1H, m), 3.76 (1H, m), 4.08 (1H, m), 4.28 (2H, m), 4.38 (1H, m), 5.14 (1H, d, J = 13.9 Hz), 5.24 (2H, br. s), 5.40 (1H, d, J = 13.9 Hz), 7.20 (1H, t, J = 6.0 Hz), 7.48 (3H, br.s), 7.62 (2H, d, J = 8.6 Hz), 8.18 (4H, d, J = 8.3 Hz), 8.42 (2H, br. s).

### Reference Example 57

- IRₘₐₓ cm⁻¹ (neat):: 3400, 1767, 1703, 1647, 1520, 1422, 1403, 1343, 1262;
- NMR δ (CDCl₃):: 1.26 (3H, d, J = 7.3 Hz), 1.37 (3H, d, J = 6.0 Hz), 2.16 (1H, m), 2.60 (3H, m), 3.27 (5H, m), 3.47 (1H, m), 3.73 (1H, m), 4.02 (1H, m), 4.32 (3H, m), 5.23 (3H, m), 5.46 (1H, d, J = 13.9 Hz), 7.22 (1H, m), 7.46 (3H, m), 7.64 (2H, d, J = 8.9 Hz), 8.21 (4H, d, J = 8.9 Hz), 8.42 (2H, m).

### Reference Example 58

- NMR δ (CDCl₃):: 1.27 (3H, d, J = 7.3 Hz), 1.36 (3H, d, J = 6.3 Hz), 3.29 (1H, dd, J = 3.0 & 5.9Hz), 5.17 (1H, d, J = 12.9 Hz), 5.23 (2H, br.s), 5.42 (1H, d, J = 12.9 Hz), 7.18 (2H, m), 7.48 (2H, m), 7.63 (2H, d, J = 8.9 Hz), 8.22 (4H, m), 8.51 (2H, m).

### Reference Example 59

- IRₘₐₓ cm⁻¹ (neat):: 3370, 1756, 1682, 1597, 1510, 1420, 1392, 1335, 1196, 1103;
- NMR δ (CDCl₃):: 1.27 (3H, d, J = 7.3 Hz), 1.34 (3H, d, J = 6.3 Hz), 2.49 (3H, s), 2.78 (2H, m), 3.29 (1H, dd, J = 2.3 & 6.6 Hz), 3.34 (1H, m), 3.49 (2H, m), 3.74 (1H, m), 4.00 (1H, m), 4.27 (2H, m), 4.36 (1H, m), 5.17 (1H, d, J = 13.9 Hz), 5.19 (2H, s), 5.42 (1H, d, J = 13.9 Hz), 7.07 (1H, d, J = 7.9 Hz), 7.44 (3H, m), 7.60 (2H, d, J = 8.9 Hz), 8.16 (4H, d, J = 8.9 Hz), 8.28 (1H, s).

### Reference Example 60

- IRₘₐₓ cm⁻¹ (neat):: 3400, 1767, 1703, 1521, 1441, 1399, 1343, 1262, 1203;
- NMR δ (CDCl₃):: 1.24 (3H, d, J = 7.3 Hz), 1.37 (3H, d, J = 6.3 Hz), 2.56 (3H, s), 2.84 (2H, m), 3.20 - 3.65 (6H, m), 3.73 (1H, m), 4.08 (1H, m), 4.20 - 4.45 (4H, m), 5.20 (1H, d, J = 13.5 Hz), 5.19 (2H, br. s), 5.45 (1H, d, J = 13.5 Hz), 7.04 (1H, dd, J = 4.9 & 7.6 Hz), 7.43 (1H, d, J = 7.6 Hz), 7.50 (2H, m), 7.63 (2H, d, J = 8.6 Hz), 8.21 (4H, d, J = 8.6 Hz), 8.35 (1H, d, J = 4.9 Hz).

### Reference Example 61

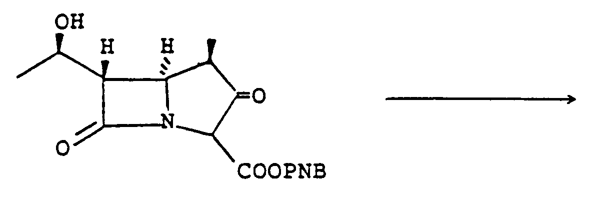

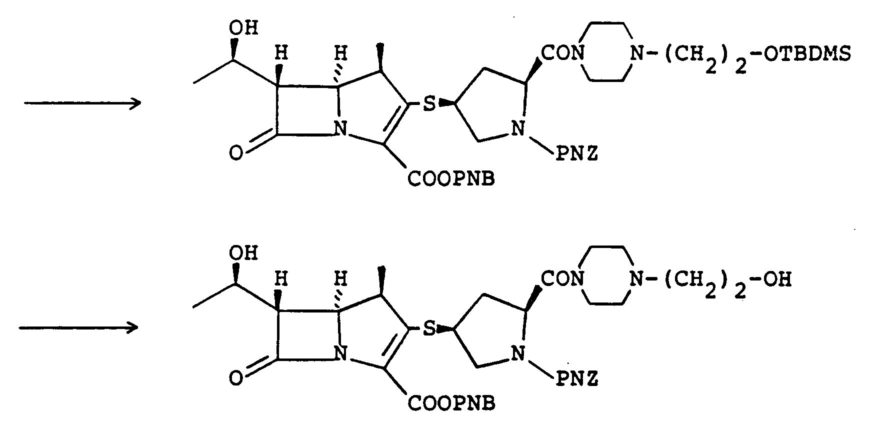

a) To a solution of (4R,5R,6S,8R)-p-nitrobenzyl-4-methyl-6-(1-hydroxyethyl)-1-azabicyclo[3.2.0]hept-3,7-dione-2-carboxylate (256 mg) in dry acetonitrile (2.0 ml), diisopropylethylamine (108 mg) and diphenyl chlorophosphate (200 mg) were added under ice-cooling, and the resultant mixture was stirred at the same temperature for 2 hours. A solution of 1-p-nitrobenzyloxycarbonyl-2-(4-(2-(t-butyldimethylsilyloxy)ethyl)piperazin-2-ylcarbonyl)-4-mercaptopyrrolidine (491 mg) and diisopropylethylamine (108 mg) in dry acetonitrile (2.0 ml) was added thereto, followed by stirring for 2 hours. The reaction mixture was diluted with ethyl acetate, washed with aqueous potassium phosphate solution and a saturated aqueous sodium chloride solution in order and dried over anhydrous magnesium sulfate. After removal of the solvent, the residue was purified by silica gel column chromatography to give (4R,5S,6S,8R,2'S,4'S)-p-nitrobenzyl-3-[1-p-nitrobenzyloxycarbonyl-2-(4-(2-(t-butyldimethylsilyloxy)ethyl)piperazin-1-ylcarbonyl)pyrrolidin-4-ylthio]-4-methyl-6-(1-hydroxyethyl)-1-azabicyclo[3.2.0] hept-2-en-7-one-2-carboxylate.
   - IRₘₐₓ cm⁻¹ (neat):: 3250, 1763, 1703, 1664, 1657, 1521, 1342;
   - NMR δ (CDCl₃):: 0.06 (6H, s), 0.89 (9H, s), 1.29 (3H, d, J = 7.3 Hz), 1.37 (3H, d, J = 6.3 Hz), 2.50 (6H, m), 3.38 (2H, m), 3.56 (2H, m), 3.76 (2H, m), 5.10 - 5.55 (4H, m), 7.40 - 7.60 (2H, m), 7.65 (2H, d, J = 8.3 Hz), 8.24 (4H, m).
b) The thus obtained (4R,5S,6S,8R,2'S,4'S)-p-nitrobenzyl-3-[1-p-nitrobenzyloxycarbonyl-2-(4-(2-(t-butyldimethylsilyloxy)ethyl)piperazin-1-ylcarbonyl)pyrrolidin-4-ylthio]-4-methyl-6-(1-hydroxyethyl)-1-azabicyclo[3.2.0]hept-2-en-7-one-2-carboxylate (476 mg) was dissolved in dry tetrahydrofuran (4.0 ml) and stirred at room temperature. Acetic acid (657 mg) and a 1 N tetrahydrofuran solution of tetrabutylammonium fluoride (2.16 ml) were added, and the resultant mixture was stirred at the same temperature for 9 hours. A phosphate buffer (pH, 7.0) was added to the reaction mixture, which was extracted with dichloromethane three times. The organic layer was dried over anhydrous magnesium sulfate, followed by removal of the solvent. The residue was purified by silica gel column chromatography to give (4R,5S,6S,8R,2'S,4'S)-p-nitrobenzyl-3-[1-p-nitrobenzyloxycarbonyl-2-(4-(2-hydroxyethyl)piperazin-1-ylcarbonyl)pyrrolidin-4-ylthio]-4-methyl-6-(1-hydroxyethyl)-1-azabicyclo[3.2.0]hept-2-en-7-one-2-carboxylate.
   - IRₘₐₓ cm⁻¹ (neat):: 3250, 1762, 1703, 1658, 1521, 1342;
   - NMR δ (CDCl₃):: 1.28 (3H, d, J = 7.3 Hz), 1.36 (3H, d, J = 6.0 Hz), 1.92 (1H, m), 2.80 (6H, m), 2.93 (1H, m), 3.20 - 3.80 (9H, m), 4.08 (1H, m), 4.26 (3H, m), 4.73 (1H, m), 5.25 (3H, m), 5.49 (1H, d, J = 13.8 Hz), 7.35 - 7.60 (2H, m), 7.64 (2H, d, J = 8.9 Hz), 8.22 (4H, m).

### Reference Example 62

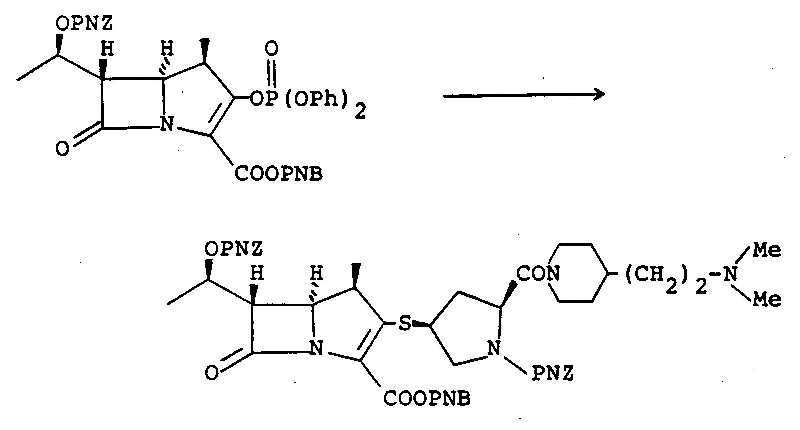

To a solution of (4R,5R,6S,8R)-p-nitrobenzyl-3-(diphenylphosphoryloxy)-4-methyl-6-(1-(p-nitrobenzyloxycarbonyloxy)ethyl)-1-azabicyclo[3.2.0]hept-2-en-7-one-2-carboxylate (714 mg) in dry acetonitrile (3.0 ml), a solution of (2S,4S)-1-p-nitrobenzyloxycarbonyl-2-(4-(2-dimethylaminoethyl)piperidin-1-ylcarbonyl)-4-mercaptopyrrolidine (505 mg) in dry acetonitrile (3.0 ml) was added under ice-cooling, and 1,8-diazabicyclo[5.4.0]-7-undecene (182 mg) was added thereto, followed by stirring at the same temperature for 2 hours. The reaction mixture was diluted with dichloromethane, washed with a saturated aqueous sodium chloride solution and dried over anhydrous magnesium sulfate. After removal of the solvent, the residue was purified by silica gel column chromatography to give (4R,5S,6S,8R,2'S,4'S)-p-nitrobenzyloxycarbonyl-3-[1-p-nitrobenzyl-2-(4-(2-dimethylaminoethyl)piperidin-1-ylcarbonyl)pyrrolidin-4-ylthio]-4-methyl-6-(1-(p-nitrobenzyloxycarbonyloxy)ethyl)-1-azabicyclo[3.2.0]hept-2-en-7-one-2-carboxylate.
- NMR δ (CDCl₃):: 1.22 (3H, d, J = 7.3 Hz), 1.29 (3H, d, J = 6.3 Hz), 2.38 (6H, s), 5.00 - 5.50 (6H, m), 7.00 - 7.70 (6H, m), 8.18 (6H, m).

## Claims

1. A compound of the formula: 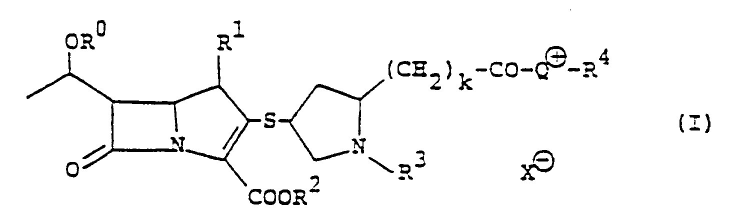 wherein R⁰ is a hydrogen atom or a protective group for hydroxyl, R¹ is a C₁₋₅ alkyl group, R² is a protective group for carboxyl or a negative charge, R³ is a hydrogen atom or a protective group for amino, R⁴ is a C₁₋₅ alkyl group or a C₁-₇ alkyl group substituted with carboxyl, C₂₋₅ alkanoyl, carbamoyl, C₁₋₅ alkylaminocarbonyl, di(C₁₋₅ alkyl)aminocarbonyl, cyano, C₁₋₅ alkoxy, hydroxy or phenyl, k is an integer of 0 to 4, X is an inorganic acid residue, benzenesulfonyloxy, p-toluenesulfonyloxy, methanesulfonyloxy or trifluoromethanesulfonyloxy or is nothing when R² is the negative charge and Q^{⊕} is a quaternary nitrogen atom-containing group represented by either one of the formulas (1) to (4): 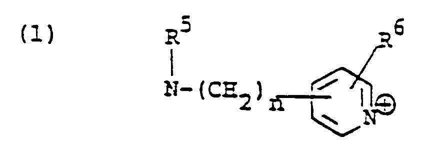 wherein R⁵ is a hydrogen atom, a C₁₋₅ alkyl group or a 2-hydroxyethyl group, R⁶ is a hydrogen atom or a C₁₋₅ alkyl group and n is an integer of 0 to 4; 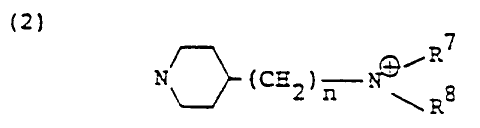 wherein R⁷ and R⁸ are each a C₁₋₅ alkyl group or may be combined together to form a lower alkylene group, or R⁸ represents a C₁-₇ alkyl group substituted with carboxyl, C₂₋₅ alkanoyl, carbamoyl, C₁₋₅ alkylaminocarbonyl, di(C₁₋₅ alkyl)aminocarbonyl, cyano, C₁₋₅ alkoxy, hydroxy or phenyl and n is as defined above; 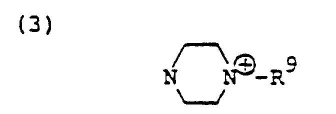 wherein R⁹ is a C₁₋₅ alkyl group or a C₁-₇ alkyl group substituted with carboxyl, C₂₋₅ alkanoyl, carbamoyl, C₁₋₅ alkylaminocarbonyl, di(C₁₋₅ alkyl)aminocarbonyl, cyano, C₁₋₅ alkoxy, hydroxy or phenyl; or 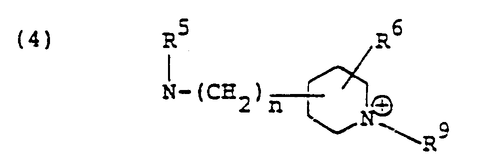 wherein R⁵, R⁶, R⁹ and n are each as defined above, or its acid or base addition salt.

2. The compound according to claim 1, wherein R⁰ and R³ are each a hydrogen atom, R² is a negative charge and X is nothing, or its acid or base addition salt.

3. The compound according to claim 2, wherein Q^{⊕} is a quaternary nitrogen atom-containing group represented by either one of the formulas (1) and (3).

4. The compound according to claim 3, wherein Q^{⊕} is a quaternary nitrogen atom-containing group represented by the formula (1) wherein R⁵ is a hydrogen atom or a methyl group, R⁶ is a hydrogen atom and n is an integer of 0 to 4.

5. The compound according to claim 3, wherein Q^{⊕} is a quaternary nitrogen atom-containing group represented by the formula (3) wherein R⁹ is a methyl group.

6. The compound according to claim 1, wherein R⁴ is a C₁-C₅ alkyl group, a C₂-C₅ alkanoyl(C₁-C₅)alkyl group, a carbamoyl(C₁-C₅)alkyl group, a C₁-C₅ alkylaminocarbonyl(C₁-C₅)alkyl group, a di(C₁-C₅)alkylaminocarbonyl(C₁-C₅)alkyl group or a hydroxy(C₂-C₅)alkyl group.

7. The compound according to claim 1, wherein R¹ is a methyl group.

8. The compound according to claim 1, wherein k is zero.

9. The compound according to claim 1, which has a (5S)-configuration.

10. The compound according to claim 1, which has a (4R,5S,6S,8R)-configuration.

11. A process for producing a compound of the formula I as disclosed in any of claims 1 to 10, which comprises reacting a compound of the formula: 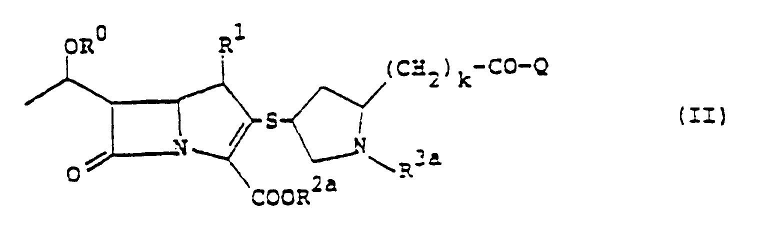 wherein R⁰, R¹ and k are each as defined in any of claims 1 to 10, R^{2a} is a protective group for carboxyl, R^{3a} is a protective group for amino and Q is a tertiary nitrogen atom-containing group resulting from elimination of a positive charge from either one of the groups (1) to (4) represented by Q^{⊕} with a compound of the formula:
R⁴-X^{a} (III)
wherein R⁴ is as defined in any of claims 1 to 10 and X^{a} is an inorganic acid residue, benzenesulfonyloxy, p-toluenesulfonyloxy, methanesulfonyloxy or trifluoromethanesulfonyloxy to give a compound of the formula: 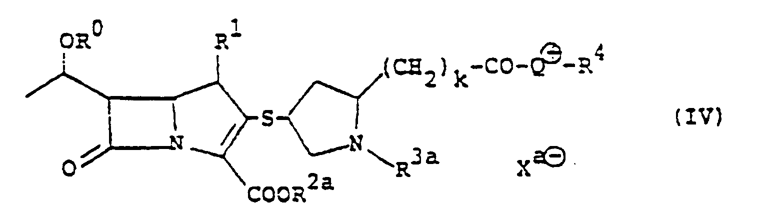 wherein R⁰, R¹, R^{2a}, R^{3a}, R⁴, k, Q^{⊕} and X^{a} are each as defined in any of claims 1 to 10, optionally followed by subjecting the latter to reaction for elimination of the hydroxyl-protecting group represented by R⁰, the carboxyl-protecting group represented by R^{2a} and/or the amino-protecting group represented by R^{3a}, thereby giving the compound (I) wherein R⁰ and R³ are each a hydrogen atom and R² is a negative charge.

12. A pharmaceutical composition which comprises as an active ingredient a pharmaceutically effective amount of at least one of the compounds as claimed in any preceding claim, and at least one pharmaceutically acceptable inert carrier or diluent.

13. Use of a compound according to any one of claims 1 to 10 for the preparation of a pharmaceutical composition for the treatment of microbial infections.

## Patentansprüche

1. Verbindung der Formel: 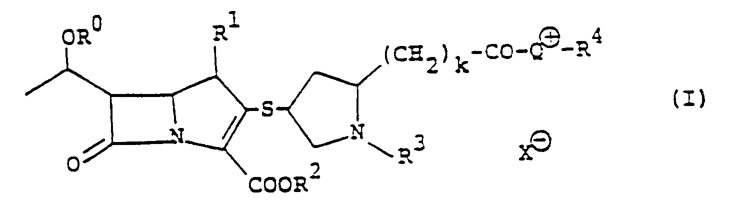 wobei R⁰ ein Wasserstoffatom oder eine Schutzgruppe für eine Hydroxylgruppe bedeutet, R¹ einen C₁₋₅-Alkylrest darstellt, R² eine Schutzgruppe für eine Carboxylgruppe oder eine negative Ladung bedeutet, R³ ein Wasserstoffatom oder eine Schutzgruppe für eine Aminogruppe darstellt, R⁴ einen C₁₋₅-Alkylrest oder einen mit einer Carboxylgruppe, einem C₂₋₅-Alkanoylrest, einer Carbamoylgruppe, einem C₁₋₅-Alkylaminocarbonylrest, einem Di(C₁₋₅-alkyl)aminocarbonylrest, einer Cyanogruppe, einem C₁₋₅-Alkoxyrest, einer Hydroxy- oder Phenylgruppe substituierten C₁₋₇-Alkylrest bedeutet, k eine ganze Zahl von 0 bis 4 darstellt, X einen anorganischen Säurerest, eine Benzolsulfonyloxy-, p-Toluolsulfonyloxy-, Methansulfonyloxy- oder Trifluormethansulfonyloxygruppe bedeutet oder nichts darstellt, wenn R² die negative Ladung bedeutet und Q^{⊕} einen ein quartäres Stickstoffatom enthaltenden Rest darstellt, der durch eine der Formeln (1) bis (4) wiedergegeben wird: 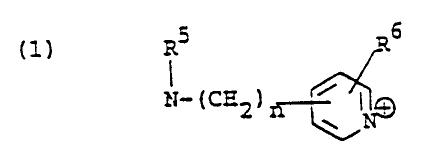 wobei R⁵ ein Wasserstoffatom, einen C₁₋₅-Alkylrest oder eine 2-Hydroxyethylgruppe bedeutet, R⁶ ein Wasserstoffatom oder einen C₁₋₅-Alkylrest darstellt und n eine ganze Zahl von 0 bis 4 bedeutet; 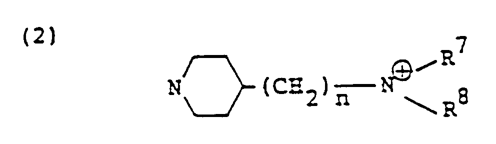 wobei R⁷ und R⁸ jeweils einen C₁₋₅-Alkylrest darstellen oder miteinander unter Bildung eines Niederalkylenrestes verbunden sein können oder R⁸ einen mit einer Carboxylgruppe, einem C₂₋₅-Alkanoylrest, einer Carbamoylgruppe, einem C₁₋₅-Alkylaminocarbonylrest, einem Di(C₁₋₅-alkyl)aminocarbonylrest, einer Cyanogruppe, einem C₁₋₅-Alkoxyrest, einer Hydroxy- oder Phenylgruppe substituierten C₁₋₇-Alkylrest wiedergibt und n wie vorstehend definiert ist; 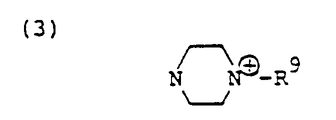 wobei R⁹ einen C₁₋₅-Alkylrest oder einen mit einer Carboxylgruppe, einem C₂₋₅-Alkanoylrest, einer Carbamoylgruppe, einem C₁₋₅-Alkylaminocarbonylrest, einem Di(C₁₋₅-alkyl)aminocarbonylrest, einer Cyanogruppe, einem C₁₋₅-Alkoxyrest, einer Hydroxy- oder Phenylgruppe substituierten C₁₋₇-Alkylrest bedeutet, oder 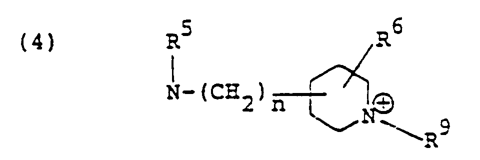 wobei R⁵, R⁶, R⁹ und n jeweils wie vorstehend definiert sind, oder ihr Säure- oder Baseadditionssalz.

2. Verbindung nach Anspruch 1, wobei R⁰ und R³ jeweils ein Wasserstoffatom darstellen, R² eine negative Ladung bedeutet und X nichts darstellt, oder ihr Säure- oder Baseadditionssalz.

3. Verbindung nach Anspruch 2, wobei Q^{⊕} einen ein quartäres Stickstoffatom enthaltenden Rest darstellt, der durch eine der Formeln (1) und (3) wiedergegeben wird.

4. Verbindung nach Anspruch 3, wobei Q^{⊕} einen ein quartäres Stickstoffatom enthaltenden Rest darstellt, der durch die Formel (1) wiedergegeben wird, wobei R⁵ ein Wasserstoffatom oder eine Methylgruppe bedeutet, R⁶ ein Wasserstoffatom darstellt und n eine ganze Zahl von 0 bis 4 bedeutet.

5. Verbindung nach Anspruch 3, wobei Q^{⊕} einen ein quartäres Stickstoffatom enthaltenden Rest darstellt, der durch die Formel (3) wiedergegeben wird, wobei R⁹ eine Methylgruppe bedeutet.

6. Verbindung nach Anspruch 1, wobei R⁴ einen C₁-C₅-Alkylrest, einen C₂-C₅-Alkanoyl(C₁-C₅)alkylrest, einen Carbamoyl(C₁-C₅)alkylrest, einen C₁-C₅-Alkylaminocarbonyl(C₁-C₅)alkylrest, einen Di(C₁-C₅)alkylaminocarbonyl(C₁-C₅)alkylrest oder einen Hydroxy(C₂-C₅)alkylrest darstellt.

7. Verbindung nach Anspruch 1, wobei R¹ eine Methylgruppe bedeutet.

8. Verbindung nach Anspruch 1, wobei k Null ist.

9. Verbindung nach Anspruch 1, die eine (5S)-Konfiguration aufweist.

10. Verbindung nach Anspruch 1, die eine (4R,5S,6S,8R)-Konfiguration aufweist.

11. Verfahren zur Herstellung einer Verbindung der Formel (I) nach einem der Ansprüche 1 bis 10, umfassend das Umsetzen einer Verbindung der Formel: 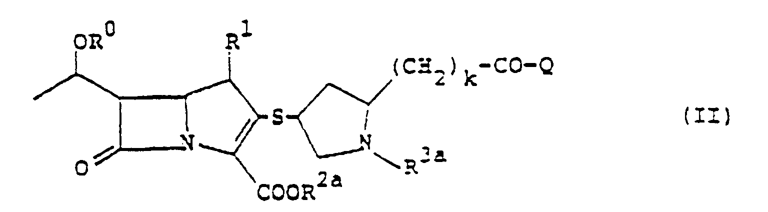 wobei R⁰, R¹ und k jeweils wie in einem der Ansprüche 1 bis 10 definiert sind, R^{2a} eine Schutzgruppe für eine Carboxylgruppe ist, R^{3a} eine Schutzgruppe für eine Aminogruppe ist und Q einen ein tertiäres Stickstoffatom enthaltenden Rest darstellt, der durch Entfernen einer positiven Ladung aus einem der durch Q^{⊕} wiedergegebenen Reste (1) bis (4) entsteht, mit einer Verbindung der Formel:
R⁴-X^{a} (III)
wobei R⁴ wie in einem der Ansprüche 1 bis 10 definiert ist und X^{a} einen anorganischen Säurerest, eine Benzolsulfonyloxy-, p-Toluolsulfonyloxy-, Methansulfonyloxy- oder Trifluormethansulfonyloxygruppe bedeutet, wobei eine Verbindung der Formel: 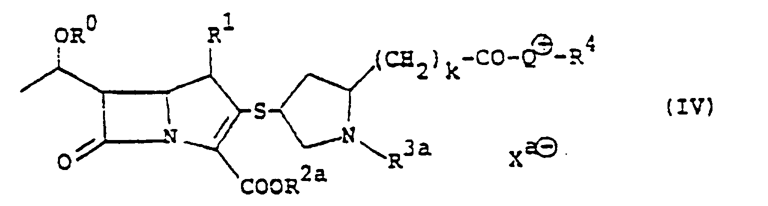 erhalten wird, wobei R⁰, R¹, R^{2a}, R^{3a}, R⁴, k, Q^{⊕} und X^{a} jeweils wie in einem der Ansprüche 1 bis 10 definiert sind, die gegebenenfalls einer Umsetzung zum Entfernen der durch R⁰ wiedergegebenen Hydroxylschutzgruppe, der durch R^{2a} wiedergegebenen Carboxylschutzgruppe und/oder der durch R^{3a} wiedergegebenen Aminoschutzgruppe unterworfen wird, wodurch die Verbindung (I) erhalten wird, wobei R⁰ und R³ jeweils ein Wasserstoffatom bedeuten und R² eine negative Ladung darstellt.

12. Arzneimittel, das als Wirkstoff eine pharmazeutisch wirksame Menge von mindestens einer der Verbindungen nach einem der vorstehenden Ansprüche und mindestens ein(en) pharmazeutisch verträgliches(n) inertes(n) Verdünnungsmittel oder Träger umfaßt.

13. Verwendung einer Verbindung nach einem der Ansprüche 1 bis 10 zur Herstellung eines Arzneimittels zur Behandlung von mikrobiellen Infektionen.

## Revendications

1. Composé de la formule : 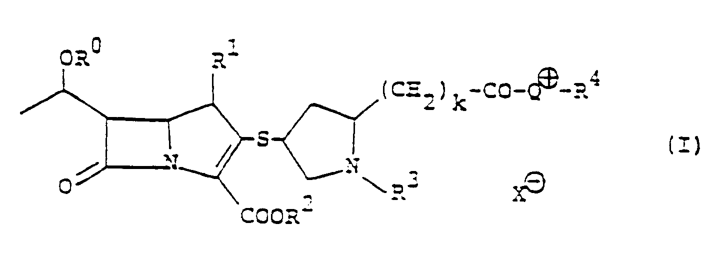 dans laquelle R⁰ est un atome d'hydrogène ou un groupe protégeant la fonction hydroxyle, R¹ représente un radical alkyle en C₁ à C₅, R² représente un groupe protégeant la fonction carboxyle, ou une charge négative, R³ représente un atome d'hydrogène ou un groupe protégeant la fonction amino, R⁴ représente un radical alkyle en C₁ à C₅, ou un radical alkyle en C₁ à C₇ substitué par des radicaux carboxyle, alcanoyle en C₂ à C₅, carbamoyle, alkyl(C₁-C₅)aminocarbonyle, di(alkyl en C₁-C₅)aminocarbonyle, cyano, alcoxy en C₁ à C₅, hydroxyle, ou phényle, k est un nombre entier dont la valeur varie de 0 à 4, X représente un reste d'acide inorganique, un radical benzènesulfonyloxy, p-toluènesulfonyloxy, méthanesulfonyloxy, ou trifluorométhanesulfonyloxy ou bien il ne représente rien lorsque R² est la charge négative et Q^{⊕} représente un groupe contenant un atome d'azote quaternaire, représenté par l'une des formules (1) à (4) : 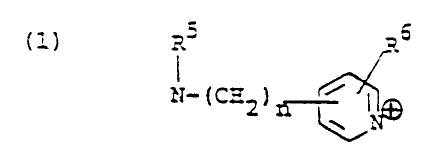 dans laquelle R⁵ représente un atome d'hydrogène, un radical alkyle en C₁ à C₅, ou un radical 2-hydroxyéthyle, R⁶ représente un atome d'hydrogène, ou un radical alkyle en C₁ à C₅ et n est un nombre entier dont la valeur varie de 0 à 4, 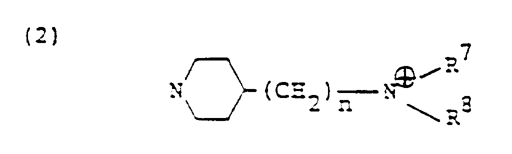 dans laquelle R⁷ et R⁸ représentent chacun un groupe alkyle en C₁ à C₅, ou peuvent être mutuellement combinés pour former un radical alkylène inférieur, ou bien R⁸ représente un radical alkyle en C₁ à C₇ substitué par des radicaux carboxyle, alcanoyle en C₂ à C₅, carbamoyle, alkyl(C₁-C₅)aminocarbonyle, di(alkyl en C₁-C₅)aminocarbonyle, cyano, alcoxy en C₁ à C₅, hydroxyle, ou phényle et n possède les significations qui lui ont été attribuées ci-dessus, 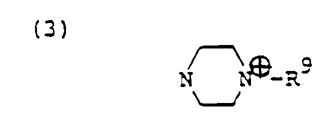 dans laquelle R⁹ représente un radical alkyle en C₁ à C₅, ou un radical alkyle en C₁ à C₇ substitué par des radicaux carboxyle, alcanoyle en C₂ à C₅, carbamoyle, alkyl(C₁-C₅)aminocarbonyle, di(alkyl en C₁-C₅)aminocarbonyle, cyano, alcoxy en C₁ à C₅, hydroxyle, ou phényle ou 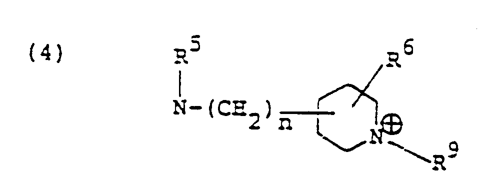 dans laquelle R⁵, R⁶, R⁹ et n possèdent chacun des significations qui leur ont été attribuées ci-dessus, ou son sel d'addition avec un acide ou une base.

2. Composé suivant la revendication 1, caractérise en ce que R⁰ et R³ représentent chacun un atome d'hydrogène, R² représente une charge négative et X ne représente rien, ou son sel d'addition avec un acide ou une base.

3. Composé suivant la revendication 2, caractérisé en Q^{⊕} représente un groupe contenant un atome d'azote quaternaire représenté par l'une des formules (1) et (3).

4. Composé suivant la revendication 3, caractérisé en ce que Q^{⊕} représente un groupe contenant un atome d'azote quaternaire représenté par la formule (1) dans laquelle R⁵ représente un atome d'hydrogène ou le radical méthyle, R⁶ représente un atome d'hydrogène et n est un nombre enter dont la valeur varie de 0 à 4.

5. Composé suivant la revendication 3, caractérisé en ce que Q^{⊕} représente un groupe contenant un atome d'azote quaternaire représenté par la formule (3) dans laquelle R⁹ représente le radical méthyle.

6. Composé suivant la revendication 1, caractérisé en ce que R⁴ représente un radical alkyle en C₁ à C₅, alcanoyl(C₂-C₅)alkyle(C₁-C₅), un radical carbamoylalkyle(C₁-C₅), un radical alkyl(C₁-C₅)aminocarbonylalkyle(C₁-C₅), un radical di(alkyl(C₁-C₅)aminocarbonylalkyle(C₁-C₅), ou un radical hydroxyalkyle en C₂ à C₅.

7. Composé suivant la revendication 1, caractérisé en ce que R¹ représente le radical méthyle.

8. Composé suivant la revendication 1, caractérisé en ce que k est égal à zéro.

9. Composé suivant la revendication 1, caractérisé en ce qu'il possède la configuration (5S).

10. Composé suivant la revendication 1, caractérisé en ce qu'il possède la configuration (4R,5S,6S,8R).

11. Procédé de production d'un composé de la formule I tel que défini dans l'une quelconque des revendications 1 à 10, caractérisé en ce que l'on fait réagir un composé de la formule : 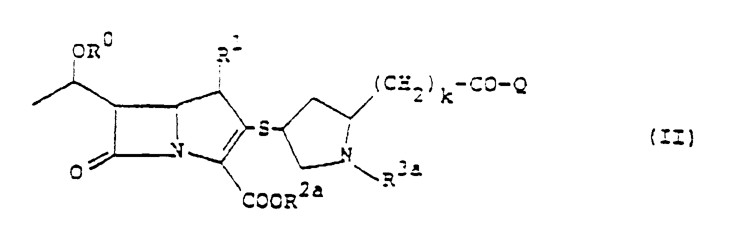 dans laquelle R⁰, R¹ et k possèdent chacun les significations qui leur ont été attribuées dans l'une quelconque des revendications 1 à 10, R^{2a} représente un groupe protégeant la fonction carboxyle, R^{3a} représente un groupe protégeant la fonction amino et Q représente un groupe contenant un atome d'azote tertiaire résultant de l'élimination d'une charge positive de l'un des groupes (1) à (4) représentés par Q^{⊖}, avec un composé de la formule :
R⁴-X^{a} (III)
dans laquelle R⁴ possède les significations qui lui ont été attribuées dans l'une quelconque des revendications 1 à 10 et X^{a} représente un reste d'acide inorganique, un radical benzènesulfonyloxy, p-toluènesulfonyloxy, méthanesulfonyloxy, ou trifluorométhanesulfonyloxy, de manière à obtenir un composé de la formule : 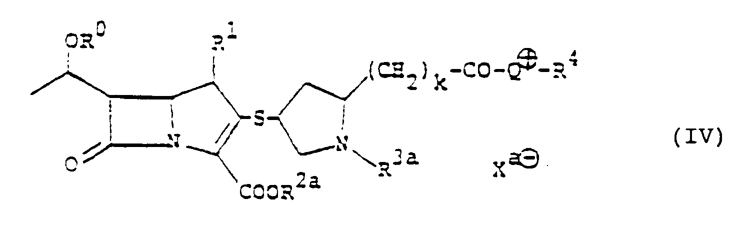 dans laquelle R⁰, R¹, R^{2a}, R^{3a}, R⁴, k, Q^{⊕} et X^{a} possèdent chacun les significations qui leur ont été attribuées dans l'une quelconque des revendications 1 à 10, puis on soumet éventuellement ce dernier à une réaction d'élimination du groupe protégeant la fonction hydroxyle représenté par R⁰, du groupe protégeant la fonction carboxyle représente par R^{2a} et/ou du groupe protégeant la fonction amino représenté par R^{3a}, de façon à obtenir le composé (I) dans lequel R⁰ et R³ représente chacun un atome d'hydrogène et R² est une charge négative.

12. Composition pharmaceutique, qui comprend, à titre d'ingrédient actif, une quantité pharmaceutiquement efficace d'au moins l'un des composés suivant l'une quelconque des revendications précédentes et au moins un diluant ou véhicule inerte, pharmaceutiquement acceptable.

13. Utilisation d'un composé suivant l'une quelconque des revendications 1 à 10 pour la préparation d'une composition pharmaceutique destinée au traitement d'infections microbiennes.
